(19) Europäisches Patentamt
European Patent Office
Office européen des brevets

(11) EP 0 896 282 A1

# (12) EUROPEAN PATENT APPLICATION

(43) Date of publication:
10.02.1999 Bulletin 1999/06

(51) Int. Cl.⁶: G06F 17/00

(21) Application number: 97810562.5

(22) Date of filing: 08.08.1997

(84) Designated Contracting States:
AT BE CH DE DK ES FI FR GB GR IE IT LI LU MC NL PT SE

(71) Applicant: R & S Incorporated
Westerbrook, Ontario K7P 2Y7 (CA)

(72) Inventor: Seegobin, Ronald
Westbrook, Ontario K0H 2XO (CA)

(74) Representative:
Laight, Martin Harvey et al
W.H. Beck, Greener & Co.
7 Stone Buildings
Lincoln's Inn
London WC2A 3SZ (GB)

# (54) A noninvasive method for identifying coronary disfunction utilizing electrocardiography derived data

(57) A method of analyzing experimentally derived electrocardiograph (ECG) data, and system for practicing said method, which allow tracking of subject cardiac status change and which allow surprisingly accurate catagorization of subjects into various abnormal and normal classifications is disclosed. The presently preferred embodiment of the present invention applies an algorithm which compares representative parameter, (eg. root-mean-square (RMS) mean), values derived from analysis of a representative composite of selected portions of a number of ECG PQRST waveforms obtained from (ECG) investigation of a subject, to similarly derived representative parameter, (eg. RMS mean and RMS standard deviation), values present in a compiled data bank derived from (ECG) investigation of numerous normals, in each of a plurality of frequency range bands. A highly diagnostic numerical "Score" is calculated by addition of "Score" components found to be acceptable under certain mathematical criteria, and provided by the algorithm. Visually interpretable time domain and power spectral density plots enhance the method. In addition, comparison of the calculated "Score" to subject cardiac ejection fraction provides indication of risk for sudden death as does the presence of "rhomboids" following a QRS complex in frequency domain plots. The present invention is directly adapted to tracking subject cardiac status change by substituting a baseline subject data set for the normal population data set.

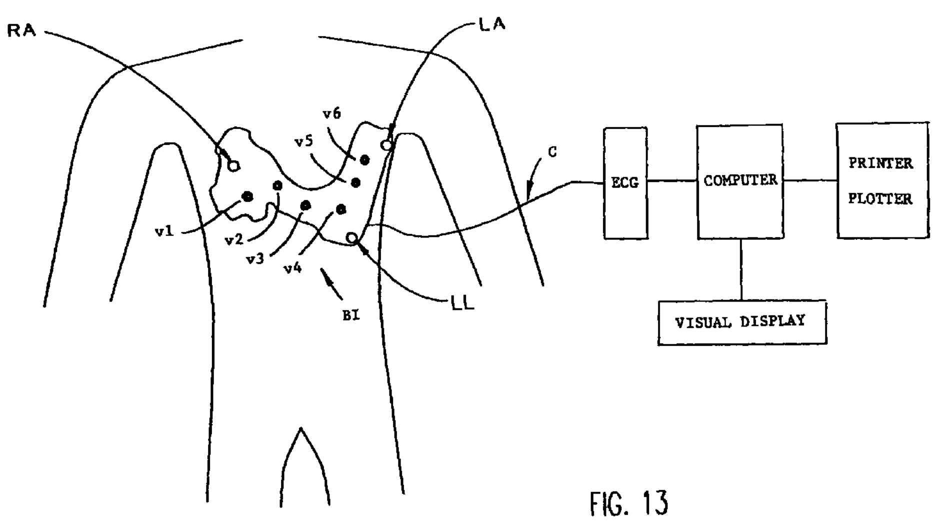


FIG. 13



Printed by Xerox (UK) Business Services
2.16.7/3.6

## Description

TECHNICAL FIELD

The present invention relates to safe noninvasive systems and methods of use thereof for application in identifying coronary disfunction, which methods and systems are suitable for application in investigation of human subjects. More particularly the present invention is primarily a method of processing data derived from application of an electrocardiograph (ECG) system which involves mathematical and statistical techniques, data filtering and windowing, and application of a unique algorithm to the end that highly predictive, easily interpreted numerically precise SEECADtm "Scores" and data patterns are determined.

BACKGROUND

It is generally accepted that approximately one-quarter of North Americans have some degree of Coronary Artery Disease (CAD). It is also generally accepted that approximately half thereof are not reliably detectable by conventionally applied diagnostic techniques, such as noting contour changes in the S-T segments of electrocardiograms (ECG's). The problematic nature the situation poses is perhaps most critically apparent when one considers that perioperative complications can be much more prevalent and serious in a patient with (CAD) than in a normal patient who does not have (CAD). That is, knowledge that a patient has (CAD), or any coronary dysfunction, can be critical in fostering morbidity and mortality reduction procedure planning and scheduling on the part of medical professionals. As well, detection of (CAD) is, of course, important in everyday matters such as the planning and execution of a simple exercise routine.

It is noted that conventional (ECG) analysis provides time domain graphical results based primarily upon the low frequency (eg. 0 to 40 Hz), content of a subject's cardiac signals monitored by on (ECG) system. This is the case whether a Frank orthogonal X-Y-Z; a standard 12 Lead or a multi-Lead monitoring etc. (ECG) system is utilized. For instance, a Patent to Brown et al., No. 5,077,667 describes a method for measuring a clinically useful characteristic of a fibrillating heart related to the elapsed time since the onset of ventricular fibrillation. Their significant variable is the power in the frequency range of 7 to 8 Hz. The Brown et al. Patent describes the use of a transformation of sampled analog time domain signals into the frequency domain and subsequent analysis thereof as a step intermediate to applying corrective treatment to a fibrillating heart.

A Patent, No. 4,680,708 to Ambos et al., describes the use of a Fast Fourier Transform (FFT) applied to a portion of an (ECG) cycle. Mathematical analysis of the last forty (40) milliseconds of a waveform derived from the time domain (QRS) complex allows for calculation of a Figure Of Merit, (FOM), based upon the frequency content thereof. Said (FOM) is correlated to the likelihood of a patient experiencing ventricular tachycardia. While the Ambos et al. Patent mentions the presence of high frequency components in a signal derived from the (ECG), said Patent primarily focuses upon the analysis of frequencies between 20 and 50 Hertz in arriving at the (FOM). The Ambos et al. Patent further states that "Recent studies...have used a variety of low (25 to 100 Hz) and high (250 to 300 Hz) band pass filters. A major limitation...is a lack of a-priori knowledge of the frequency distribution of signals of interest and the inherent risk that filtering will exclude signals of particular interest."

Other recent investigation has focused upon the diagnostic capability inherent in the presence of particular high frequency components present in an (ECG) signal. For instance, a very recent paper by Aversano et al., titled "High Frequency QRS Electrocardiography In The Detection Of Reperfusion Following Thrombolytic Therapy", (see Clinical Cardiology, (17, 175-182, April 1994)), states that the amplitude of the high frequency components, (eg. 150-250 Hz), of the (QRS) complexes decreases during cardiac ischemia, and returns to normal with resolution thereof. It is also stated that high frequency electrocardiography is a rapid and reliable bedside technique for discriminating between successful and failed reperfusion in patients treated with thrombolytic agents for myocardial infarction. The Aversano et al. paper also states that "Studies involving high-frequency QRS electrocardiography are few and modest."

A paper by Moss and Benhorin titled "Prognosis and Management After a First Myocardial Infarction", New England J. Medicine, Vol. 322, No. 11, 1990 points out the importance of being able to identify and distinguish patients with various types of (CAD) so that appropriate treatment can be prescribed. This paper, in conclusion acknowledges that noninvasive techniques currently available for detecting jeopardized ischemic myocardium are imperfect.

The above sampling of relevant prior reference materials shows that techniques such as direct morphologic analysis of conventional time domain signals, application of (FFT) to (ECG) time domain derived signals to provide frequency domain spectra for analysis, analysis of high frequency components of (ECG) signals and the focusing on specific portions of a QRS complex etc. are known. There remains, however, need for additional and more probative noninvasive methods of analyzing (ECG) derived data which allow incipient (CAD) in patients to be identified with improved certainty. In particular, there is a need for a method of accurately identifying subjects with (CAD), the validity of which has been shown to provide utility by actual clinical testing.

The present invention provides an improved method of analyzing (ECG) derived data which have as shown by actual test, (in view of an extensive data bank accumulated by the inventor containing both normal and abnormal (ECG) data), to enable greatly improved ability to accurately and noninvasively separate abnormal from normal cardiac subjects. The method of the present invention, for instance, routinely allows identification of subjects with truely silent (CAD), and subjects who do not present with the tell-tale classic QRST and T wave changes. The method of the present invention also routinely allows identification of subjects with nonspecific S-T and T wave changes, and allows identification and separate classification of subjects with prior myocardial infarction, abnormal patients who present with normal (ECG), and simultaneously distinguishes the population of abnormal subjects who present with normal (ECG). The present invention also allows identification of subjects who are at risk for sudden death.

DISCLOSURE OF THE INVENTION

The present invention, in its presently preferred embodiment, utilizes a Frank orthogonal X-Y-Z Lead electocardiograph (ECG) system, but is primarily a method of analyzing and categorizing individual subject electrocardiogram (ECG) data. Said method has been shown to be capable of identifying and classifying subjects into cardiac categories such as:

1. Normal, and

2. Abnormal:

   a. Presents with prior myocardial infarction,

   b. Presents with nonspecific S-T and T wave changes,

   c. Presents with normal (ECG) but known otherwise to be abnormal.

(Note, continued efforts are serving to greatly increase the specific classification capabilities of the present invention, and are even extending it to identification of specific anatomic abnornormality location(s), (eg. myocardium, cardiac artery etc), which cause said specific abnormality).

As a starting point the present invention requires a substantial data base from which (ECG) data attributable to a "normal" population can be derived and used to form a "template" against which unknown subject (ECG's) can be compared. The present invention provides that by application of a discriminant Algorithm, (see supra), these unknown subjects may be appropriately classified. In the presently preferred embodiment of the present invention such a data base was developed by selection and testing of fit and healthy, relatively young subjects from families with a low prevalence of, and low risk factors for, coronary artery disease (CAD). Suitable subjects were required to fill out a questionnaire, analysis of which aided in determination of subject suitability as a "normal". A total of two-hundred-fifty (250) normal subjects were identified and (ECG) data obtained from each thereof. A random sampling of data from one-hundred-forty (146) subjects from said group of two-hundred-fifty (250) normals was assembled and analyzed to provide relevant, (see supra), composite root-mean-square, (RMS), mean and standard deviation values.

To arrive at said relevant normal RMS mean and RMS standard deviation values, (ECG) signals for each normal were derived by acquiring a number of, (typically one-hundred (100)), full (ECG) cycles, (ie. full PQRST (ECG) cycles), for each normal subject, sampling each full cycle to provide six-hundred (600) data points over the extent thereof, and then selecting out the corresponding data points in each QRS complex in each of said full PQRST cardiac cycles. A single averaged (ECG) cardiac cycle was mathematically constructed from said number of QRS complexes and RMS mean and RMS standard deviation values calculated therefore.

In addition, similar RMS means and RMS standard deviation values were obtained from the same data, but which data had been subjected to digital filtering employing a Blackman-Harris window. The results are identified in the following table for each of the three Frank orthogonal (ECG) X-Y-Z system lead signals:

FOR FRANK (ECG) SYSTEM LEAD X, (HORIZONTAL AXIS):

| FREQUENCY RANGE | DATA PROVIDED | |
|---|---|---|
| (FULL-ALL FREQUENCIES) | RMS MEAN | RMS SD |
| ((0) TO (10) HZ) | RMS MEAN | RMS SD |
| ((10) TO (60) HZ) | RMS MEAN | RMS SD |
| ((60) TO (150) HZ) | RMS MEAN | RMS SD |
| ((150) TO (250) HZ) | RMS MEAN | RMS SD |

FOR FRANK (ECG) SYSTEM LEAD Y, (VERTICAL AXIS):

| FREQUENCY RANGE | DATA PROVIDED | |
|---|---|---|
| (FULL-ALL FREQUENCIES) | RMS MEAN | RMS SD |
| ((0) TO (10) HZ) | RMS MEAN | RMS SD |
| ((10) TO (60) HZ) | RMS MEAN | RMS SD |
| ((60) TO (150) HZ) | RMS MEAN | RMS SD |
| ((150) TO (250) HZ) | RMS MEAN | RMS SD |

FOR FRANK (ECG) SYSTEM LEAD Z, FRONT TO BACK AXIS):

| FREQUENCY RANGE | DATA PROVIDED | |
|---|---|---|
| (FULL-ALL FREQUENCIES) | RMS MEAN | RMS SD |
| ((0) TO (10) HZ) | RMS MEAN | RMS SD |
| ((10) TO (60) HZ) | RMS MEAN | RMS SD |
| ((60) TO (150) HZ) | RMS MEAN | RMS SD |
| ((150) TO (250) HZ) | RMS MEAN | RMS SD |

where SD stands for Standard Deviation.

| FREQUENCY (HZ) | LEAD X | LEAD Y | LEAD Z |
|---|---|---|---|
| TOTAL SIGNAL 0-INFINITE Hz | | | |
| 0-10 Hz | | | |
| 10-60 Hz | | | |
| 60-150 Hz | | | |
| 150-250 Hz | | | |

TABLE D-1, EACH TABULAR CATEGORY IS PROVIDED AN RMS MEAN AND STANDARD DEVIATION

Said resulting normal RMS mean and RMS standard deviation values for each of the frequency ranges and utilized Frank (ECG) system X-Y-Z lead serve to define assumed Gaussian "templates" for normals, against which similarly derived RMS means, acquired from individual subjects, are compared under the guidelines of the Algorithmic Method of the present invention, (see supra).

To date data from more than one-thousand (1000) abnormal subjects has been assembled by the inventor, and subject RMS mean values derived therefrom. It is noted that abnormal subjects tested provide representatives from each the three abnormal groups identified infra.

Continuing, to apply the Algorithm Method of the present invention many, (typically one-hundred (100)), of human subject derived full PQRST (ECG) cardiac cycles are obtained. Said full (ECG) cardiac cycles are each then sampled to provide six-hundred data points over the extent thereof and the sampled data points corresponding to the QRS complex in each full cycle are mathematically averaged to provide a composite QRS complex. As well, the digital filtering and application of the Blackman-Harris Window to allow calculation of the RMS means which correspond to each frequency range and Frank (ECG) system X-Y-Z lead utilized, are performed. The end result can be expressed as a table of data, (not shown), similar to the table presented above for the normal data but which contains only composite RMS mean values.

The Algorithm employed in the method of the present invention embodiment then provides for a maximum of thirty (30) calculations to be performed as follows:

a. Up to Fifteen of said calculations involve calculating the difference between the subject composite RMS mean and the corresponding normal composite RMS mean, and dividing the result by the corresponding normal RMS standard deviation, for each Frank X-Y-Z (ECG) lead and each frequency range band, (ie. the fifteen calculations break down as five (5) frequency range bands per each of the Frank X-Y-Z (ECG) leads.)

b. Twelve of said calculations involve finding the RMS ratio for each frequency range band, (eg. 0-10 Hz, 10-60 Hz, 60-150 Hz and 150-250 Hz) to the total sum of all said frequency band RMS contributions for each appropriate said Frank (ECG) X-Y-Z lead, for a subject, and subtracting therefrom equivalent RMS ratio mean results derived based upon the same calculations as applied to normal data, and dividing the result by the RMS standard deviation for the corresponding frequency range, of said normal data.

c. Three of said calculations involve calculating the difference between ratios of the subject composite RMS means of Frank lead X/Y, Y/Z and X/Z ratios and the corresponding RMS means of normal X/Y, Y/Z and X/Z ratios and dividing by the RMS value of the normal RMS standard deviations of said ratios.

Each of the above identified thirty (30) calculations will result in a number (Pi). A "Score" component number (Si) is then derived based upon where a (Pi) number lies in an assumed Gaussian Distribution. This is calculated based upon normal data RMS Means and RMS Standard Deviations (X) as follows:

$$\text{If } -1X < Pi < 1X \text{ then } Si = 0,$$

$$\text{If } -2X < Pi < -1X \text{ or } 1X < Pi < 2X \text{ then } Si = 1,$$

$$\text{If } -3X < Pi < -2X \text{ or } 2X < Pi < 3X \text{ then } Si = 2,$$

$$\text{If } -4X < Pi < -3X \text{ or } 3X < Pi < 4X \text{ then } Si = 3 \text{ etc.}$$

Each of the resulting subject RMS mean values associated with a calculated Si value is then analyzed to determine if it is greater than or equal to ninety-five (95%) percent of the data points from which the normal RMS mean was calculated. If this is the case the associated Si is accepted. Otherwise it is rejected. That is, a ninety-five (95%) confidence interval, based upon normal data spread, is imposed, in determining whether to accept a calculated (SI) value.

Accepted values are then selected and added together to provide a final numerical "Score". (Note, two Scores in the catagory a. above are commonly not selected as being redundant to other Scores. Said commonly unselected Scores are better identified in the Detailed Description Section).

(Note that the truncation involved in obtaining an Si value can be eliminated and the Pi Score utilized in determining said final numerical "Score").

In either approach to "Score" calculation it has been found that if said final numerical "Score" is "low" (eg. approximately 0 to 7) then the subject is more likely to be normal. If the final numerical "Score" is high (eg. greater than about 8), then the subject is more likely to be abnormal. For instance, a "Score" of 7 provides a ninety (90%) percent confidence of normality, and a "Score" of 8.4 provides a ninety-five (95%) confidence of normality, (See Fig. 9).

As will be better presented in the Detailed Description Section of this Disclosure, the results of the application of the method of the present invention as described above can be presented in numerous ways. A particularly relevant approach is to present the results on a graph of ("Sensitivity" vs. "100 - Specificity"). (The present invention provides that the "Score" value be plotted against the abscissa (100 - Specificity) and that percentage of a group having said "Score" be plotted on the (Sensitivity) ordinate). Said approach to presentation is generally known as an ROC curve, (ROC stands for Receiver Operation Characteristic as the technique was originally derived for use in testing radio receiver quality). Said approach to presentation serves to visually demonstrate the success of the present invention method of analyzing (ECG) derived data. In particular, abnormal subjects which can not be identified by conventional (ECG) analysis techniques, are seen to be easily identified by application of the present invention algorithm.

In addition, the present invention provides that time domain data obtained from Frank X-Y-Z (ECG) leads should be subjected to a Fourier Transform and manipulated to provide Power Spectral Density (PDS) vs. Frequency plots. As will also be better presented in the Detailed Description Section of this Disclosure, said (PDS) plots are typically easier than associated (ECG) data vs. time plots to visually interpret. Said plots complement the above described "Scoring" system approach to identifying coronary disfunction.

It should also be appreciated that if an initial patient specific data base is accumulated, it can serve as a baseline data base and be utilized as a replacement for the normal subject population data base. At later times, additional patient specific data can then be obtained, and compared to the patient baseline data base, in a tracking scenario.

It has further been found that, if dividing a "Score" for a patient as provided by practice of the present invention, by the ejection fraction of the patient, (as obtained by radionuclide imaging or other accurate technique), provides a result greater than one (1.0) then the subject patient involved is at high risk of sudden death. In addition, it has been found that if the S-T segement following a QRS complex has "Rhomboids" present therein, (eg. electrical signal activity on the order of three (3) standard deviations from a baseline signal, particulary in Time Domain plots in 60-150 and/or 150-250 Hz band(s)), then the patient involved is at high risk of sudden death. The present invention methodology can include as steps inclusion of said criteria.

A system for practicing the present invention method comprises (ECG) signal monitoring electrode means, (perhaps preferably such as described in an Allowed U.S. Patent to Stratbucker, Serial No. 434,658 which Claims a Bioelectric Interface with all necessary (ECG) Chest Mounted Electrodes present in a common electrode separation maintaining support material), and any necessary interface such that data monitored by said (ECG) electrodes is fed to a memory device, and possibly means for determining ejection fraction. In addition, computational means for performing necessary calculations and displaying results, and necessary interconnection and interfacing means are required. It should be appreciated that a system for practicing the present invention method can be fashioned from essentially any computer system with sufficient memory means and computational capability means, and it is the configuration thereof to carry out the method of the present invention which distinguishes said system over computer systems in general, rather than any specific system elements.

BRIEF DESCRIPTION OF THE DRAWINGS

Fig. 1a shows a representation of a human torso with Frank X and Y (ECG) system leads attached thereto.

Fig. 1b shows a cross section taken at a--a in Fig. 1a, with Frank Z (ECG) system leads attached thereto.

Fig. 2 shows a demonstrative "PQRST" (ECG) waveform.

Fig. 3 demonstrates a table for recording data necessary for practice of the present invention.

Fig. 4 shows an assumed gaussian distribution for use in assigning "Score" component numbers during practice of the present algorithm.

Fig. 5 shows sample results as provided by practice of the present invention plotted on an ROC curve in which a (100 - Specificity), and Sensitivity, appear on the abscissa and ordinate respectively.

Fig. (6X1, 6X2, 6X3, 6X4), (6Y1, 6Y2, 6Y3, 6Y4), (6Z1, 6Z2, 6Z3, 6Z4), show twelve related plots of subject time domain sample data recorded from Frank X-Y and Z (ECG) system leads for a plurality of frequency range bands. Higher frequency frequency bands are presented as one progresses from Fig. 6X1 to 6X4, and from 6Y1 to 6Y4 and from 6Z1 to 6Z4.

Figs. (7aX1, 7aX2, 7aX3, 7aX4, 7aX5), (7aY1, 7aY2, 7aY3, 7aY4, 7aY5), (7aZ1, 7aZ2, 7aZ3, 7aZ4, 7aZ5), show fifteen related plots of subject frequency domain power spectral density. Shown in (7aX1, 7aY1 and 7aZ1) are transforms of full frequency band requisite data. Transforms of data from progressively higher frequency frequency bands are presented as one progresses from Fig. 7aX1 to 7aX5, from 7aY1 to 7aY5 and from 7aZ1 to 7aZ5.

Figs. (7bX1, 7bX2, 7bX3, 7bX4, 7bX5), (7bY1, 7bY2, 7bY3, 7bY4, 7bY5), (7bZ1, 7bZ2, 7bZ3, 7bZ4, 7bZ5), show fifteen plots of related subject time domain data. Shown in (7bX1, 7bY1 and 7bZ1) are full frequency band requisite data. Data from higher frequency frequency bands are presented as one progresses progresses from Fig. 7BX1 to 7aY5, from 7aY1 to 7aY5 and from 7aZ1 to 7aZ5.

Fig. 8 shows actual data obtained from various subject groups by practice of the present invention, on a ROC curve

Fig. 9 shows actual data obtained from various subject groups by practice of the present invention, on a graph in which the abcsissa is scaled linearly with the present invention "Score".

Fig. 10 shows a flow chart of the method of the present invention.

Figs. 11a and 11b show respectively, presentation of data for subjects at risk of sudden death and subjects not at risk of sudden death produced by present invention methodology.

Fig. 12 shows a presentation of data developed by present invention methodology and indicates that patterns of subject abnormality can be observed in such a presentation.

Fig. 13 shows a system for practicing the present invention method.

DETAILED DESCRIPTION

In the following a specific embodiment of the present invention is presented. Said specific embodiment assumes the use of an (ECG) system which utilizes Frank (ECG) orthogonal X-Y-Z leads. It is to be understood, however, that the present invention is not limited to such and can be practiced with (ECG) systems in which any number of leads, (eg. standard twelve (12), sixteen (16), or mapping arrays of twenty-four (24) or more etc.), are present, and in which only some of the present leads are utilized. The following specific embodiment is presented as it is well documented and is presently the preferred embodiment.

Turning now to the drawings, there is shown in Fig. 1(a) a frontal view of a torso of a human, with (ECG) Frank X and Y leads properly affixed thereto. Fig. 1(b) shows a cross section taken at a--a in Fig. 1(a) with (ECG) Frank Z leads properly attached thereto. In use said (ECG) Frank X-Y-Z leads are attached to an (ECG) system and serve to effect orthogonal monitoring of (ECG) full cardiac cycle PQRST signals which are essentially shaped as shown in Fig. 2.

The present invention requires as a starting point that a significant data base be available, which significant data base contains representative composite (ECG) data for all, or some portion of full (ECG) PQRST cycles for each (ECG) lead, for a normal population. (Note, a normal population is defined as one in which the subjects have no detectable coronary artery disease (CAD) by history and multiple conventional diagnostic tents, and are not at risk therefore based upon age, family history etc.) Such a significant data base for normals was acquired by obtaining a number of full (ECG) PQRST cycles from each of the Frank X-Y-Z (ECG) leads present in the presently discussed embodiment of the invention, from each of two-hundred-fifty (250) normals. (It is noted that the present invention is not limited to cases in which all leads present in an (ECG) system are monitored but that a preferred embodiment does utilize all available information). Next, a random sample of one-hundred-forty-six (146) of said two-hundred-fifty (250) normals was selected and a representative number of the full (ECG) PQRST cycles from each, (typically one-hundred (100)), for each Frank X-Y-Z (ECG) lead, were then selected and each subjected to a sampling procedure which provided some number of data points for each, (six-hundred (600) was chosen in the presently discussed embodiment). Next, the sampled data points corresponding to the QRS depolarization complexes in each selected full (ECG) PQRST cycle were selected and a representative composite QRS complex for each Frank (ECG) X-Y and Z leads formed therefrom by mathematical averaging thereof. Said representative composite was then subjected to filtering and windowing techniques to provide a number of data sets for each of the Frank (ECG) X-Y-Z leads. Said data sets in the presently preferred embodiment of the present invention provide information present in said representative composite in the frequency bands:

a. All frequencies;
b. Between zero (0) and ten (10) Hz;
c. Between ten (10) and sixty (60) Hz;
d. Between sixty (60) and
e. Between one-hundred-fifty (150) and two-hundred-fifty (250) Hz.

For each of the Frank (ECG) X-Y-Z leads then, five (5) sets of data were derived as described, and from each of said sets of data a Root-Mean-Square (RMS) mean and (RMS) standard deviation were calculated. This, it will be appreciated, resulted in fifteen (15) RMS representative composite means and standard deviations being available, (five for each Frank (ECG) X-Y-Z lead).

In view of the described RMS mean and RMS standard deviations (SD's) available clinical application of the present invention can be practiced.

To practice the present invention, data are obtained from a subject in a manner essentially the same as described infra for normals. That is, a number of full (ECG) PQRST full cardiac cycles from each Frank (ECG) X-Y-Z lead are obtained and a representative number thereof are selected and subjected to a sampling procedure. Some portion of each full PQRST waveform is selected, (eg. the QRS depolarization complex is utilized in the presently preferred embodiment of the present invention), and a representative composite thereof formed therefrom for each Frank (ECG) system X-Y-Z lead. For each representative composite a RMS mean is then calculated so that a table equivalent to that shown in Fig. 3, but containing subject RMS mean data, is formed.

With the described normal RMS mean and RMS standard deviation data, and subject RMS mean data then available, the algorithm of the method of the present invention can be applied to arrive at a diagnostic mathematical "Score".

The algorithm of the present invention involves mathematical comparison of:

a. Normal and subject RMS means in view of normal RMS standard deviation;

b. Ratios of normal and subject RMS frequency range and means to the summation of RMS means for all Frequency range bands for each Frank (ECG) X-Y-Z lead in view of normal standard deviation for the numerator frequency band.

c. Ratios of normal and subject Frank (ECG) X-Y-Z Lead RMS means in view of normal standard deviations of said ratios.

Briefly, application of each of the identified steps provides a numerical result (Pi), which in general is typically not a whole integer. The next step is to process said numerical result (Pi) by comparison to an assumed Gaussian Distribution derived from the normal population data to arrive at a whole number integer which represents how many RMS standard deviations the subject RMS mean is away from the normal RMS mean, and assign a whole integer "Score" component number (Si) based thereupon. The algorithm then requires that a ninety-five (95%) confidence interval, based upon normal RMS standard deviation data be applied to determine if a "Score" component should be accepted and included in calculation of a final "Score", said final "Score" being arrived at by an addition of accepted "Score" components. Said algorithm will now be described in detail.

The first step in applying the algorithm of the presently described presently preferred embodiment of the present invention is perform up to fifteen (15) calculations comprising subtracting the subject RMS mean from a corresponding Normal RMS mean and dividing the result by a corresponding normal RMS standard deviation for each Frank (ECG) X-Y-Z lead in each frequency range identified infra, to provide numbers (Pi).
(Note, the accompany computer printout page labeled "avgasc" provides the various RMS Means and Standard Deviations referred to in the following, corresponding to the P1 - P30 number.)

<u>For the Frank (ECG) X lead, (IE. HORIZONTAL AXIS):</u>

For all frequencies:

$$\frac{\text{(Subject RMS mean-Normal RMS mean)}}{\text{Normal RMS Standard Deviation}} = PX1 = P1$$

For the frequency range band zero (0) to ten (10 Hz):

$$\frac{\text{(Subject RMS mean-Normal RMS mean)}}{\text{Normal RMS Standard Deviation}} = PX2 = P2$$

For the frequency range band ten (10) to sixty (60 Hz):

$$\frac{\text{(Subject RMS mean-Normal RMS mean)}}{\text{Normal RMS Standard Deviation}} = PX3 = P3$$

For the frequency range band sixty (60) to one-hundred-fifty (150 Hz):

$$\frac{\text{(Subject RMS mean-Normal RMS mean)}}{\text{Normal RMS Standard Deviation}} = PX4 = P4$$

For the frequency range band one-hundred-fifty (150 Hz) to two-hundred-fifty (250 Hz):

$$\frac{\text{(Subject RMS mean-Normal RMS mean)}}{\text{Normal RMS Standard Deviation}} = PX5 = P5$$

<u>For the Frank (ECG) Y lead, (IE. VERTICAL AXIS):</u>

For all frequencies:

$$\frac{\text{(Subject RMS mean-Normal RMS mean)}}{\text{Normal RMS Standard Deviation}} = PY1 = P6$$

For the frequency range band zero (0) to ten (10 Hz):

$$\frac{\text{(Subject RMS mean-Normal RMS mean)}}{\text{Normal RMS Standard Deviation}} = PY2 = P7$$

For the frequency range band ten (10) to sixty (60 Hz):

$$\frac{\text{(Subject RMS mean-Normal RMS mean)}}{\text{Normal RMS Standard Deviation}} = PY3 = P8$$

For the frequency range band sixty (60) to one-hundred-fifty (150 Hz):

$$\frac{\text{(Subject RMS mean-Normal RMS mean)}}{\text{Normal RMS Standard Deviation}} = PY4 = P9$$

For the frequency range band one-hundred-fifty (150 Hz) to two-hundred-fifty (250 Hz):

$$\frac{\text{(Subject RMS mean-Normal RMS mean)}}{\text{Normal RMS Standard Deviation}} = PY5 = P10$$

<u>For the Frank (ECG) Z lead, (IE. FRONT TO BACK AXIS):</u>

For all frequencies:

$$\frac{\text{(Subject RMS mean-Normal RMS mean)}}{\text{Normal RMS Standard Deviation}} = PZ1 = P11$$

For the frequency range band zero (0) to ten (10 Hz):

$$\frac{\text{(Subject RMS mean-Normal RMS mean)}}{\text{Normal RMS Standard Deviation}} = PZ2 = P12$$

For the frequency range band ten (10) to sixty (60 Hz):

$$\frac{\text{(Subject RMS mean-Normal RMS mean)}}{\text{Normal RMS Standard Deviation}} = PZ3 = P13$$

For the frequency range band sixty (60) to one-hundred-fifty (150 Hz):

$$\frac{\text{(Subject RMS mean-Normal RMS mean)}}{\text{Normal RMS Standard Deviation}} = PZ4 = P14$$

For the frequency range band one-hundred-fifty (150 Hz) to two-hundred-fifty (250 Hz):

$$\frac{\text{(Subject RMS mean-Normal RMS mean)}}{\text{Normal RMS Standard Deviation}} = PZ5 = P15$$

Twelve (12) additional groups of calculations are then performed in which the relative RMS mean content of each frequency range band identified infra is determined as a percentage of the RMS means of the sum of the filter derived frequency range bands for each Frank (ECG) system X-Y-Z system lead, for both Subject and Normal data, the differences therebetween being divided by the corresponding normal RMS Standard Deviation to provide additional numbers (Pi):

For Frank (ECG) X lead, (IE. HORIZONTAL AXIS:

Define:

$$\frac{\text{( 100 X Subject RMS (0-10 Hz) mean )}}{\text{(Subject RMS mean (0-10 Hz)+(10-60 Hz)+ (60-150Hz)+(150-250 Hz))}} = SX0$$

$$\frac{(100 \times \text{Normal RMS (0-10 Hz) mean})}{(\text{Normal RMS mean (0-10 Hz)+(10-60 Hz)+(60-150 Hz)+(150-250 Hz)})} = NX0$$

Normal RMS Standard Deviation (0-10 Hz) = NXSD0
Then:

$$\frac{SX0-NX0}{NXSD0} = PX6 = P16$$

Define:

$$\frac{(100 \times \text{Subject RMS (10-60 Hz) mean})}{(\text{Subject RMS mean (0-10 Hz)+(10-60 Hz)+(60-150 Hz)+(150-250 Hz)})} = SX1$$

$$\frac{(100 \times \text{Normal RMS (10-60 Hz) mean})}{(\text{Normal RMS mean (0-10 Hz)+(10-60 Hz)+(60-150 Hz)+(150-250 Hz)})} = NX1$$

Normal RMS Standard Deviation (10-60 Hz) = NXSD1
Then:

$$\frac{SX1-NX1}{NXSD1} = PX7 = P17$$

Define:

$$\frac{(100 \times \text{Subject RMS (60-150 Hz) mean})}{(\text{Subject RMS mean (0-10 Hz)+(10-60 Hz)+(60-150 Hz)+(150-250 Hz)})} = SX2$$

$$\frac{(100 \times \text{Normal RMS (60-150 Hz) mean})}{(\text{Normal RMS mean (0-10 Hz)+(10-60 Hz)+(60-150 Hz)+(150-250 Hz)})} = NX2$$

Normal RMS Standard Deviation (60-150 Hz) = NXSD2
Then:

$$\frac{SX2-NX2}{NXSD2} = PX8 = P18$$

Define:

$$\frac{(100 \times \text{Subject RMS (150-250 Hz) mean})}{(\text{Subject RMS mean (0-10 Hz)+(10-60 Hz)+(60-150 Hz)+(150-250 Hz)})} = SX3$$

$$\frac{(100 \times \text{Normal RMS (150-250 Hz) mean})}{(\text{Normal RMS mean (0-10 Hz)+(10-60 Hz)+(60-150 Hz)+(150-250 Hz)})} = NX3$$

Normal RMS Standard Deviation (150-250 Hz) = NXSD3
Then:

$$\frac{SX3-NX3}{NXSD3} = PX9 = P19$$

For Frank (ECG) Y lead, (IE. VERTICAL AXIS):

Define:

$$\frac{(100 \text{ X Subject RMS (0-10 Hz) mean})}{(\text{Subject RMS mean (0-10 Hz)+(10-60 Hz)+(60-150 Hz)+(150-250 Hz)}) = SY0} = SY0$$

$$\frac{(100 \text{ x Normal RMS (0-10 Hz) mean})}{(\text{Normal RMS mean (0-10 Hz)+(10-60 Hz)+(60-150 Hz)+(150-250 Hz)})} = NY0$$

Normal RMS Standard Deviation (0-10 Hz) = NYSD0 Then:

$$\frac{SY0-NY0}{NYSD0} = PY6 = P20$$

Define:

$$\frac{(100 \text{ x Subject RMS (10-60 Hz) mean})}{(\text{Subject RMS mean (0-10 Hz)+(10-60 Hz)+(60-150 Hz)+(150-250 Hz)})} = SY1$$

$$\frac{(100 \text{ x Normal RMS (10-60 Hz) mean})}{(\text{Normal RMS mean (0-10 Hz)+(10-60 Hz)+(60-150 Hz)+(150-250 Hz)})} = NY1$$

Normal RMS Standard Deviation (10-60 Hz) = NYSD1
Then:

$$\frac{SY1-NY1}{NYSD1} = PY7 = P21$$

Define:

$$\frac{(100 \times \text{Subject RMS (60-150 Hz) mean})}{(\text{Subject RMS mean (0-10 Hz)+(10-60 Hz)+(60-150 Hz)+(150-250 Hz)})} = SY2$$

$$\frac{(100 \times \text{Normal RMS (60-150 Hz) mean})}{(\text{Normal RMS mean (0-10 Hz)+(10-60 Hz)+(60-150 Hz)+(150-250 Hz)})} = NY2$$

Normal RMS Standard Deviation (60-150 Hz) = NYSD2 Then:

$$\frac{SY2-NY2}{NYSD2} = PY8 = P22$$

Define:

$$\frac{(100 \times \text{Subject RMS (150-250 Hz) mean})}{(\text{Subject RMS mean (0-10 Hz)+(10-60 Hz)+(60-150 Hz)+(150-250 Hz)})} = SY3$$

$$\frac{(100 \times \text{Normal RMS (150-250 Hz) mean})}{(\text{Normal RMS mean (0-10 Hz)+(10-60 Hz)+(60-150 Hz)+(150-250 Hz)})} = NY3$$

Normal RMS Standard Deviation (150-250 Hz) = NYSD3
Then:

$$\frac{SY3-NY3}{NYSD3} = PY9 = P23$$

For Frank (ECG) Z lead, (IE. FRONT TO BACK AXIS):

Define:

$$\frac{(100 \times \text{Subject RMS (0-10 Hz) mean})}{(\text{Subject RMS mean (0-10 Hz)+(10-60 Hz)+(60-150 Hz)+(150-250 Hz)})} = SZ0$$

$$\frac{(100 \times \text{Normal RMS (0-10 Hz) mean})}{(\text{Normal RMS mean (0-10 Hz)+(10-60 Hz)+(60-150 Hz)+(150-250 Hz)})} = NZ0$$

Normal RMS Standard Deviation (0-10 Hz) = NZSD0

Then:

$$\frac{SZ0-NZ0}{NZSD0} = PZ6 = P24$$

Define:

$$\frac{(100 \times \text{Subject RMS (10-60 Hz) mean})}{(\text{Subject RMS mean (0-10 Hz)+(10-60 Hz)+(60-150 Hz)+(150-250 Hz)})} = SZ1$$

$$\frac{(100 \times \text{Normal RMS (10-60 Hz) mean})}{(\text{Normal RMS mean (0-10 Hz)+(10-60 Hz)+(60-150 Hz)+(150-250 Hz)})} = NZ1$$

Normal RMS Standard Deviation (10-60 Hz) = NZSD1

Then:

$$\frac{SZ1-NZ1}{NZSD1} = PZ7 = P25$$

Define:

$$\frac{(100 \times \text{Subject RMS (60-150 Hz) mean})}{(\text{Subject RMS mean (0-10 Hz)+(10-60 Hz)+(60-150 Hz)+(150-250 Hz)})} = SZ2$$

$$\frac{(100 \times \text{Normal RMS (60-150 Hz) mean})}{(\text{Normal RMS mean (0-10 Hz)+(10-60 Hz)+(60-150 Hz)+(150-250 Hz)})} = NZ2$$

Normal RMS Standard Deviation (60-150 Hz) = NZSD2
Then:

$$\frac{SZ2-NZ2}{NZSD2} = PZ8 = P26$$

Define:

$$\frac{(100 \times \text{Subject RMS (150-250 Hz) mean})}{(\text{Subject RMS mean (0-10 Hz)+(10-60 Hz)+(60-150 Hz)+(150-250 Hz)})} = SZ3$$

$$\frac{(100 \times \text{Normal RMS (150-250 Hz) mean})}{(\text{Normal RMS mean (0-10 Hz)+(10-60 Hz)+(60-150 Hz)+(150-250 Hz)})} = NZ3$$

Normal RMS Standard Deviation (150-250 Hz) = NZSD3
Then:

$$\frac{SZ3-NZ3}{NZSD3} = PZ9 = P27$$

Three (3) additional calculations are then performed in which Subject RMS means of ratios of RMS means obtained from the Frank (ECG) X-Y-Z leads, are subtracted from corresponding RMS means of ratios obtained similarly from normals, the results of which subtraction are then divided by RMS Standard Deviations of normal corresponding RMS ratios to provide additional numbers (Pi):

For the Frank (ECG) X-Y-Z leads:

$$\frac{\text{Subject RMS (X/Y) mean} - \text{Normal RMS (X/Y) mean}}{\text{Normal Standard Deviation (X/Y)}} = P(X/Y) = P28$$

$$\frac{\text{Subject RMS (Y/Z) mean} - \text{Normal RMS (Z/Y) mean}}{\text{Normal Standard Deviation (Y/Z)}} = P(Y/Z) = P29$$

$$\frac{\text{Subject RMS (X/Z) mean} - \text{Normal RMS (X/Z) mean}}{\text{Normal Standard Deviation (X/Z)}} = P(X/Z) = P30$$

Continuing, each of the above up to thirty (30) calculated numbers:

(PX1-P1, PX2-P2, PX3-P3, PX4-P4, PX5-P5, PX6-P6 PX7-P7, PX8-P8, PX9-P9), (PY1-P10, PY2-P11, PY3-P12, PY4-P14, PY6-P15, PY7-P16, PY8-P17, PY9-P18), (PZ1-P19, PZ2-P20, PZ3-P21, PZ4-P22, PZ5-P23, PZ6-P24, PZ7-P25, PZ8-P26, PZ9-P27), P(X/Y-P28), P(Y/Z)-P29 and P(X/Z)-P30), (generally identified as (Pi)),

can optionally be compared to a corresponding assumed distribution of normal data to arrive at a "Score" component number. If a number (Pi) is within some +/-"X" RMS Standard Deviation range of the RMS mean as shown below, a "Score" component number (Si) is taken to be:

If -1X < (Pi) < 1X then Si = 0;

If -2X < (Pi) < -1X or

If 1X < (Pi) < 2X then Si = 1;

If -3X < (Pi) < -2X or

If 2X < (Pi) < 3X then Si = 2;

If -4X < (Pi) < -3X or

If 3X < (Pi) < 4X then Si = 3 etc.

Fig. 4 demonstrates this graphically.

Continuing, each resulting "Score" component (PI) or (Si) calculated as just described is then subjected to a final test to determine if it should be accepted or rejected. Said final test involves comparing the Subject RMS mean to the data from which the Normal RMS mean was calculated. If less than or equal to ninety-five (95%) percent of the data points from which the Normal RMS mean was calculated are more than the subject's RMS mean the associated "Score" component is accepted, otherwise it is rejected. Accepted "Score" component numbers are then added to provide a final numerical "Score".

It has been found that if said final numerical "Score" is "low", (eg. approximately 0 to 7), then the Subject involved is more likely to be normal. If the final numerical "Score" is "high", (eg. greater than about 8), then the Subject is more likely to be abnormal.

A particularly relevant approach to presenting the results of applying the disclosed method of the present invention is demonstrated by Fig. 5. Fig. 5 shows a plot in which the abscissa is (100-specificity) and the ordinate is (sensitivity). These terms are well known and mean:

$$\text{Specificity} = \frac{\text{Normals with Negative Test}}{\text{All Normals Tested}}$$

$$\text{Sensitivity} = \frac{\text{Abnormal with Positive Test}}{\text{All Abnormals Tested}}.$$

The curve in Fig. 5 is demonstrative of these which populations of subjects provide in an (ROC) format. The present invention method provides that (ROC) curves be prepared by associating a "Score" value with the abscissa, in a nonlinear manner, and the percentage of a group having said "Score" value with the ordinate of such a plot. The success of the present invention in identifying and distinguishing abnormal subjects has been demonstrated to be quite striking. Figs. 8 and 9, discussed supra, better serve to demonstrate this with actual empirically derived data.
Figs. 6X1 through 6Z4 show twelve (12) diagrams, 6X1, 6X2, 6X3, 6X4, 6Y1, 6Y2, 6Y3, 6Y4, 6Z1, 6Z2, 6Z3 and 6Z4, of subject time domain sample date recorded from Frank X, Y and Z leads. Higher frequency band data are presented as one progresses from Fig. 6X1 to 6X4, and from Fig. 6Y1 to 6Y4, and from Fig. 6Z1 to 6Z4.

More particularly, Figs. 6X1, 6Y1 and 6Z1 show filtered composite subject (ECG) data set time domain waveforms obtained from X, Y and Z leads, respectively, of a Frank ECG system in frequency band range of 0.0 - 10 HZ. Figs. 6X2, 6Y2 and 6Z2 show filtered composite subject (ECG) data set time domain waveforms obtained from X, Y and Z leads, respectively, of a Frank ECG system for the frequency band of 10 - 60 HZ. Figs. 6X3, 6Y3 and 6Z3 show filtered composite subject (ECG) data set time domain waveforms obtained from X, Y and Z leads, respectively, of a Frank ECG system for the frequency band of 60 - 150 HZ. Figs. 6X4, 6Y4 and 6Z4 show filtered composite subject (ECG) data set time domain waveforms obtained from X, Y and Z leads, respectively, of a Frank ECG system for the frequency band of 150 - 250 HZ. It is noted that the plots for the 60 - 150 and 150 - 250 HZ bands present as "envelopes" as signals go positive to negative and vice versa in very short time periods, (ie. over a few "Sample Numbers"). All plots in Figs. 6X1 - 6Z4 have the ordinate marked in micro-volts, and the abscissa is marked in digital filter data points 0 to 600; taken at progressive times during an (ECG) cycle.

It is noted that Figs. 6Y3 and 6Y4 show "Rhomboids" present in the segment past the QRS complex region, (ie. in channels 375-600). The Rhomboids are shown as dashed-line to indicate that they were added to the actual patient data graph. This was done in preference to cluttering the Disclosure with an additional page of Drawings, however, the point to be made is that the presence of said "Rhomboids" in at least one time domain, frequency band plot, and especially said presence in more than one such frequency band plot, is very indicative of a patient in danger of Sudden Death.

Figs. 7aX1 - 7aZ5 show fifteen (15) diagrams of typical subject data in frequency domain Power Spectral Density form, (with Magnitude on ordinate), plotted as a function of Frequency, (on abscissa). Figs. 7bX1 - 7bZ5 show fifteen (15) diagrams of typical subject data in Time Domain form, (Magnitude on ordinate), plotted as a function of Time, (on abscissa). All said identified plots provide Magnitude, on the ordinate, in microvolts.

More particularly, it is noted that Figs. 7aX1, 7aY1 and 7aZ1 show subject composite (ECG) data set frequency domain power spectral density plots derived from X, Y and Z leads, respectively, of a Frank ECG system, over a frequency band of 0.0 to 100 HZ. Figs. 7aX2, 7aY2 and 7aZ2 show subject composite (ECG) data set frequency domain power spectral density plots derived from X, Y and Z leads, respectively, of a Frank ECG system, over a frequency band of 0.0 to 15 HZ. Figs. 7aX3, 7aY3 and 7aZ3 show subject composite (ECG) data set frequency domain power spectral density plots derived from X, Y and Z leads, respectively, of a Frank ECG system, over a frequency band of 0.0 to 80 HZ. Figs. 7aX4, 7aY4 and 7aZ4 show subject composite (ECG) data set frequency domain power spectral density plots derived from X, Y and Z leads, respectively, of a Frank ECG system, over a frequency band of 50 to 200 HZ. Figs. 7aX5, 7aY5 and 7aZ5 show subject composite (ECG) data set frequency domain power spectral density plots derived from X, Y and Z leads, respectively, of a Frank ECG system, over a frequency band of 100 to 300 HZ. All plots in Figs. 7aX1 - 7aZ4 have the ordinate marked in micro-volts, and the abscissa is marked in HZ, (ie. cycles per second).

As well, Figs. 7bX1, 7bY1 and 7bZ1 show subject composite (ECG) data set time domain waveforms obtained from X, Y and Z leads, respectively, of a Frank ECG system in an unfiltered full requisite frequency band. Figs. 7bX2, 7bY2 and 7bZ2 show subject composite (ECG) data set time domain waveforms obtained from X, Y and Z leads, respectively, of a Frank ECG system in a filtered frequency band range of 0.0 - 10 HZ. Figs. 7bX3, 7bY3 and 7bZ3 show subject composite (ECG) data set time domain waveforms obtained from X, Y and Z leads, respectively, of a Frank ECG system in a filtered frequency band range of 10 - 60 HZ. Figs. 7bX4, 7bY4 and 7bZ4 show subject composite (ECG) data set time domain waveforms obtained from X, Y and Z leads, respectively, of a Frank ECG system in a filtered frequency band range of 60 - 150 HZ. Figs. 7bX5, 7bY5 and 7bZ5 show subject composite (ECG) data set time domain waveforms obtained from X, Y and Z leads, respectively, of a Frank ECG system in a filtered frequency band range of 150 - 250 HZ. It is noted that the plots for the 60 - 150 and 150 - 250 HZ bands present as "envelopes" as signals go positive to negative and vice verse in very short time periods, (ie. over a few "Sample Numbers"). All plots in Figs. 7bX1 - 7bZ5 have the ordinate marked in micro-volts, and the abscissa is marked in digital filter Sample Number data points, taken at progressive times during a (ECG) cycle. The present invention then makes use of such visual aids as an added feature. The three curves in each plot represent normal mean and plus/minus one standard deviations, and subject data. It is also to be understood that the above described approach to diagnosis can be applied to tracking patients over time and can be applied before and after various stress tests which attempt to provoke otherwise indolent or silent coronary artery abnormalities. Stress tests can, for example, involve treadmill exertion or a cold pressor test in which a subject simply places an arm into cold water for a few minutes. Changes in "Score" results combined with changes in the appearance of Power Spectral Density (PSD) and Amplitude Plots over time or before and after stress tests can provide insight as to a subject's coronary health not made available by less vigerous testing. Multiple mean curves can be simultaneously presented on a single plot to allow easy visual comparison of changes in Power Spectral Density as a function of time or stress. Observation of changes in (PSD) plots in the various frequency bands is a correlated part of the method of the present invention. Of particular interest, the inventor has noted that plots of (PSD) in the frequency ranges of sixty (60) to one-hundred-fifty (150) HZ and one-hundred-fifty (150) HZ to two-hundred-fifty (250) HZ show the greatest change in visually observable shape when a cold pressor test is administered. This is considered a significant observation.

Note also that as shown in Fig. 7aX1, it is common to include numerical representation in frequency as well as the time domain plots. Four numbers can be present. Using the Power Spectral Density plot as an example, when present said numbers are representations of:

Upper left---the number of Standard Deviations a Subject Power Spectral Density Value is away from a corresponding Normal Power Spectral Density Value for the Frequency Band in the Plot.

Lower Left---the Percentage of Normals which are below the Subject Power Spectral Density Value for the Frequency Band in the Plot.

Upper Right---the number of Normalized, (ie. Subject Power Spectral Density Value in the Frequency Band of the Plot divided by the Sum of Power Spectral Density Values for all Frequency Bands), Standard Deviations a Subject Power Spectral Value is away from a corresponding Normalized Subject Power Spectral Density Value for Normals for the Frequency Band in the Plot.

Lower Right---the Percentage of Normals which are below the Normalized Subject Power Spectral Density Value for the Frequency band in the Plot.

(Note that (RMS) values can be substituted for Power Spectral Density). Said numbers and visual Plots aid in interpretation of a Subject's "Score".

Figs. 8 and 9 show (ROC) plots for actual data arrived at using the present invention method. Again, (ROC) curves typically plot Sensitivity vs. (100-Specificity) on ordinate and abscissa respectively, presented as percentages. Said Plots in Figs. 8 and 9 were generated by associating the present invention "Score" with the abscissa (100-Specificity), but with the zero (0) thereof being at the right side so that the "Score" increases to the left. As the "Score" increases the percentage of each group of subjects associated therewith is plotted on the ordinate. By observation of Figs. 8 and 9 it will be appreciated that as the "Score" increases the percentage of normals in a group of known normals having said "Score" value drops off rapidly, but the percentage of known abnormals in a group of known abnormals drops off such more slowly. For instance, at a "Score" of zero (0) all members of all groups are present. At a "Score" of five (5) approximately eighty (80%) percent of all members of an abnormal group will be present, but only approximately eleven (11%) percent of normals are present.

It is noted that a "Score" scale along the abscissa will be nonlinear, when compared to the (100 - Specificity) scale.

Fig. 8 shows data presented in (ROC) format for Abnormals in various categories:

For subjects known to have had a myocardial infarction (MI) shown by twelve (12) lead (ECG), identified as (BEMI);

For subjects with non-specific ST-T wave abnormality on twelve (12) lead (ECG), identified as (BEST);

For a subjects with normal resting twelve (12) lead (ECG) but awaiting surgery, identified as (BNOB).

For a test set of patients who have (CAD), identified as (BTEST) and (BGENSIA).

Fig. 9 shows data plotted in Fig. 8 plotted in a different format in which the abcissa is scaled in terms of the "Score" developed by the present invention method.

Present is also a curve for Normals data, identified as (NORM).

Also included are curves for two groups additional groups of volunteer subjects which contain patients who have known risk factors for (CAD) identified as (BMAQ) and (BTNR). These constitute a "real-world" population of what are considered normals, in that both normals and abnormals are present. As would be expected, the data for the (BMAQ) and (BTNR) groups is generally positioned between the data for the known abnormal (BTEST) and normal groups.

The important thing to note is that the method of the present invention very definitely separates the various groups whether resented in the format of Fig. 8 or Fig. 9.

Fig. 10 provides a Flow Chart representation of the primary focus of the preferred embodiment of the Method of the present invention, said method comprising a noninvasive approach to investigating cardiac status of a subject, and enabling classification of a subject into normal and abnormal cardiac categories utilizing electrocardiography (ECG) data obtained therefrom.

The first step (A) and (A') respectively, are shown to involve:

a. in step (A) obtaining data corresponding to (ECG) cycle(s) from at least one monitored lead(s) of an (ECG) system for a multiplicity of members of a population of subjects who have been documented as normal subjects, in that they do not show risk factors for, or demonstrate detectable cardiac abnormality;
and

b. in step (A') obtaining data corresponding to (ECG) cycle(s) from at least one monitored lead(s) of an (ECG) system for a subject, said monitored (ECG) system lead(s) utilized being the same as the monitored (ECG) system lead(s) utilized to obtain (ECG) cycle(s) for a multiplicity of normal subjects.

Next, in stops (B) and (B') respectively, a portion of an (ECG) cycle is selected, (eg. while the QRS complex is preferred, any portion, or the entire (ECG) cycle can be selected), for each of the normal subject population (B) and subject (B'). The same (ECG) cycle portion is typically selected for both the normal subject population and the subject.

Next, in steps (C) and (C'), in conjunction with application of filtering techniques, a plurality of data sets are arrived at for each monitored (ECG) system lead for both the normal subject population, (step (C)), and for the subject, (step (C')). Each of said data sets corresponds to a composite of said selected (ECG) cycle portion for said population of normal subjects in a selected frequency band range. It is disclosed that the preferred filtering technique is digital and has been utilized to provide data sets for:

a. data contained in all frequencies;
b. data contained in the frequency band of (0.0) to (10) HZ;
c. data contained in the frequency band of (10) to (60) HZ;
d. data contained in the frequency band of (60) to (150) HZ;
e. data contained in the frequency band of (150) to (250) HZ.

It is specifically disclosed, however, that data in only one frequency band range, (eg. all frequencies), can be provided in this step. It is also noted that other frequency bands can be selected, and that the recited bands are exemplary and nonlimiting.

(It is noted that steps (A), (B), (C) and (E) might be carried out only once for many runs of steps (A'), (B'), (C') and (E'). This is because steps (A), (B), (C) and (E) are applied to a normal subject population, which does not change, except perhaps when additional normal subject data are added to a normal subject populatinq data bank. Steps (A'), (B'), (C') and (E') must be run anew for each subject tested, however. Steps (D) and (F) will therefore access relatively standard values for the normal subject population, while accessing new values for each subject tested. However, steps (A), (B), (C) and (E) are inherently performed in the context of any testing of a subject.)

In step (D), normal subject population and subject (ECG) data set(s), (formed by user determined filtering techniques, (steps (C) and (C')), are plotted and displayed as a function of at least one parameter selected from the group consisting of time and frequency. It is noted that where data is plotted as a function of frequency a time to frequency domain conversion calculation must be performed to provide the frequency domain data. The results of this step are to provide as desired, visually interpretable plots of (ECG) magnitude and power spectral density. (It is noted that variations of the present invention procedure provide that, this step can omitted, or be performed after steps which directly follow, (eg. steps (E) and (E') or step (F)). Step (D) is presented at this point in the flow chart only because the data to be plotted and displayed is available at this point. It is further noted that step (D) can be performed utilizing a different selected (ECG) cycle portion, in steps (B) and (B'), than is selected and utilized in following steps (E), (E') and (F)), (eg. a full (ECG) cycle can be chosen for plotting and a QRS complex (ECG) cycle portion chosen for use in steps (E), (E') and (F)).

The next steps, (E) and (E') respectively, involve calculating corresponding representative parameter(s), and desired ratios thereof, from resulting data sets in said selected frequency band ranges for each monitored (ECG) system lead, for both the normal subject population (step (E)) and said subject, (step (E')). These steps are indicated as performed parallel to step (D) as data to allow both performance of step (D) and step (E) and (E') is available at this point. (As noted above, the selected (ECG) cycle portion chosen in steps (B) and (B') and utilized in steps (E), (E') and (F) can be different than that utilized in step (D). It is also to be understood that in one version of the present invention procedure, steps (E), (E') and (F) can be omitted and only step (D) performed).

In step (F) corresponding subject and normal subject population representative parameter(s) and/or corresponding ration of subject and ratios of normal subject population representative parameters are then compared and results of said comparison are combined to arrive at a "score", the magnitude of which "score" provides an indication of the cardiac status of said subject, and enables classification of a subject into normal and abnormal cardiac categories. A confidence level "acceptance test" can be optionally applied to qualify results of said comparisons for inclusion in arriving at said "score".

It is also noted that the step of calculating representative parameter(s) for normal subject population and subject data sets for monitored (ECG) system leads typically involves calculating at least one parameter selected from the group consisting of a root-mean-square mean and a root-mean-square standard deviation from said data set(s) from which composite(s) of a selected (ECG) cycle portion are calculated.

It is further noted that obtaining mean and standard deviation parameters, (typically, but not necessarily, based upon root-mean-square calculated parameters), enables practice of an "acceptance test" wherein a result of comparing subject to corresponding normal subject population parameters or ratios of a subject to corresponding ratios of the normal subject population parameters is accepted only if a subject acceptance parameter is offset by greater than, for instance, at least one normal subject population standard deviation from a mean of said normal subject population.

This step includes displaying of the "score" and when desired, components obtained from various composite data sets combined to arrive thereat.

It has further been found by investigation of CAMI/11 data base data for Subjects known to be at risk for Sudden Death, that if dividing a present invention "Score" for a patient, (as provided by the described practice of the present invention), by the ejection fraction, (in Percent), of the patient, provides a result greater than one (1.0), then the patient involved is at high risk of Sudden Death.

A visual presentation of the just described phenomona is quite striking, as is shown by in Figs. 11a and 11b which show scatter-graphs demonstrating the relationship between said present invention:

("Score"/Ejection Fraction)

plotted against the present invention "Score", (termed "SEECAD"tm Score. ("SEECAD" is a Trademark owned by R & S Incorporated, a Canadian Corporation). Note, as shown in Fig. 11b, that a Population of Subjects <u>not</u> at risk for Sudden Death present with results wherein Subject data "scatter" is closely confined about the line which begins at (0.0, 0.0) and ends at (50, 1.5); whereas a population which demonstrated Sudden Death presents with data wich demonstrate a much larger range of scatter. In addition, and most importantly on an individual patient basis, note that no Subject data in Fig. 11b exceed the value of 1.0 on the Abscissa, whereas a large number of Subjects shown in Fig. 11a provide data points above 1.0. The bottom line conclusion to be appreciated is that should a Subject present with a:

("Score"/Ejection Fraction)

value greater than 1.0, said Subject should be considered to definitely be a risk for Sudden Death. If said ratio is coupled with the presence of previously described "Rhomboids" present following a QRS complex in Time Domain Plots, (see for instance demonstration in Figs. 6Y3 and 6Y4), then the patient involved should be considered to be at very high risk for sudden death. This combination of present invention "SEECAD" Score with other typically obtained Cardiac Data provides insight to the potential scope of application of the present invention. The definition of and availability of the described "SEECAD" Score provided by practice of the present invention, has opened a whole new and very promising avenue In the area of Subject evaluation.

Fig. 12 shows a three-dimensional presentation of Data Components utilized in computing a "SEECAD"$_{tm}$ Score. Fig. 12 is included to show that such a presentation indicates that Patterns of:

Data components Standard Deviation from <u>Normality,</u>

which data components were derived utilizing present invention methodology, can identify specific Subject Abnormality Data Patterns. It is emphasized that known efforts of previous researchers have had as a focus the diagnosis of Subject abnormality by the comparison of:

Subject Data to <u>Abnormal</u> Subject Population Data, and looking for a match.

The present invention then has a new focus, emphasis added. Again, the present invention focus is on comparing Subject Data to Normal Subject Population Data, and Patterns of Data Components which naturally arise thereform are found to be indicative of Specific Catagories of Abnormality. The fact that the present invention approach, based in comparing Subject Data to Normal Subject Population Data, results in Data Patterns which serve to idicate a Specific Subject Abnormality is a distinguishing factor of the present invention, and provides an extremely exciting area of continued development. It is projected that further work utilizing present invention non-invasively obtained "SEECAD" Score data and methodology will provide the ability to not only separate Abnormal from Normal Subjects, (already possible), but to further identify the most likely anatomical location of the source of identified Abnormality, (eg. specific myocardium, specific coronary-arteries etc).

Fig. 13 shows a Diagram of the basic components of a system which can be utilized to practice the present invention method. A partial human torso is shown with a Chest mounted Bioelectric Interface (BI) thereon. (Note that equivalent limb electrodes (RA), (LA) (LL) are present therein). Conventional individual Limb and Precordial (ie. (v1), (v2), (v3), (v4), (v5) and (v6)) leads can, of course, be utilized as well. It has been found, however, that use of a chest mountable Bioelectric Interface (BI), as shown, provides better (ECG) signals by maintaining relatively better electrode contact to a subject and relatively constant electrode spacing, in use. A Cable (C) provides electrical signals from said electrodes (RA), (LA), (LL,) (v1), (v2), (v3), (v4), (v5) and (v6) to an (ECG) monitor (ECG), which feeds to a Computational Means (COMPUTER), which in turn provides $SEECAD_{tm}$ data to a (VISUAL DISPLAY) and to a (PRINTER/PLOTTER). Of course Fig. 13 is only demonstrative and the present invention system is not limited to the configuration shown.

It is to be understood that throughout this Disclosure the RMS Mean values are cited. It is possible to utilize other calculated values, such as Averages, in the method of the present invention. The term "Mean" should be interpreted broadly to include such alternatives.

The terms "Assumed Gaussian" have also been used throughtout this Disclosure when refering to Data Distribution RMS Means and RMS Standard Deviations. It is noted that in fact, analysis of empirically obtained data has proven the assumption to be valid.

It is also to be understood that the Term "Rhomboid" is used herein only to generally identify the presence of (ECG) activity beyond the QRS complex as shown by dashed lines in Figs. 6Y3 and 6Y4, and does not impose any plot locus shape limitations.

To provide full disclosure a print-out of major portions of the computer program utilized in the practice of the present invention is included herewith directly. Also included following the computer print-out is a table of data which documents the above discussed results.

| Dec 20 1994 19:31:35 | ecgzx.pro | Page 1 |
|---|---|---|

```
;************************************************************
; INITIALIZE ARRAYS
;************************************************************
INFILE_PATH='/usr2/multinorm/1_2/'
OUTFILE_PATH='/usr2/outplots/'
COMMON SP1, K3, K4, X3, X4, K3D, K4D
COMMON SP2, PXDATA, PYDATA, PZDATA, PXDATA1, PXDATA2, PXDATA3,PYDATA1
COMMON SP3, PYDATA2, PZDATA2, PZDATA1, PYDATA3, PZDATA3, XDATA, YDATA, ZDA
COMMON SP4, pidd0
COMMON SP5, XDATA1, YDATA1, ZDATA1, XDATA2, YDATA2, ZDATA2,XDATA3,YDATA3,Z
COMMON SP6, VMDATA2,pid,pidd1,pidd2,pidd3,pidd4,pidd5,pidd6,pidd7,pidd8,pi
COMMON SP7, VMDATA
COMMON SP8, XR
COMMON SP9, OXDATA, OYDATA, OZDATA, OXDATA1, OXDATA2,OXDATA3, OXDATAN,OVMD
COMMON SP10, OVMDATA, OYDATA1,OYDATA2,OYDATA3,OZDATA1,OZDATA2,OZDATA3,OYDA
COMMON SP11, OZDATAN, OPXDATA1,OPXDATA2,OPXDATA3,OPXDATA, OPYDATA,OPYDATA1
COMMON SP12, OPYDATA2,OPYDATA3, OPZDATA,OPZDATA1,OPZDATA2,OPZDATA3
COMMON SP13, PIDDA
COMMON SP14, YYDATA, XXDATA, ZZDATA
COMMON SP15, OX,OY,OZ,OX1,OX2,OX3,OY1,OY2,Oy3,OZ1,OZ2,OZ3,OPX,OPY,OPZ,OPX1
COMMON SP16, XDATA1A,YDATA1A,ZDATA1A,XXD,YYD,ZZD,XXD1,YYD1,ZZD1,XXD2,YYD2,
COMMON SP17, XXD3,YYD3,ZZD3,XXD4,YYD4,ZZD4,XXD5,YYD5,ZZD5,XXD6,YYD6,ZZD6,X
COMMON SP18, OX1A,OY1A,OZ1A,OPx1a,opy1a,opz1a
COMMON NOX9, PATNAME,NUMBER,IDATA,J,JK,filen,s55,pxx,ECG,s5
COMMON NOY4, CCA,TABLES,DDSET,TABNAM,tablab1,ref
cca=fltarr(10,7,50)
ddset=intarr(30)
tables=fltarr(50,120)
tabnam=strarr(5,30)
tablab1=strarr(30)
tablab2=strarr(5)
tablab3=strarr(20)
tablab4=strarr(20)
k3=FLTARR(600)
k4=FLTARR(600)
X3=FLTARR(600)
x4=FLTARR(600)
xr=fltarr(500)
rR=INTARR(256)
gG=INTARR(256)
bB=INTARR(256)
XINDEX=INTARR(600)
XINDEX2=INTARR(150)
XINDEX3=INTARR(256)
XINDEX4=INTARR(100)
IDATA=INTARR(2096)
XDATA=FLTARR(600)
YDATA=FLTARR(600)
ZDATA=FLTARR(600)
XXDATA=FLTARR(600)
YYDATA=FLTARR(600)
ZZDATA=FLTARR(600)
OXDATA=FLTARR(600)
OYDATA=FLTARR(600)
OZDATA=FLTARR(600)
OXDATAN=FLTARR(600)
OYDATAN=FLTARR(600)
OZDATAN=FLTARR(600)
```

```
OXXDATA=FLTARR(600)
OYYDATA=FLTARR(600)
OZZDATA=FLTARR(600)
XDATA1=FLTARR(600)
XDATA2=FLTARR(600)
XDATA3=FLTARR(600)
YDATA1=FLTARR(600)
YDATA2=FLTARR(600)
YDATA3=FLTARR(600)
ZDATA1=FLTARR(600)
ZDATA2=FLTARR(600)
ZDATA3=FLTARR(600)
XXD=fltarr(5,600)
YYD=fltarr(5,600)
ZZD=fltarr(5,600)
XXD1=fltarr(5,600)
YYD1=fltarr(5,600)
ZZD1=fltarr(5,600)
XXD2=fltarr(5,600)
YYD2=fltarr(5,600)
ZZD2=fltarr(5,600)
XXD3=fltarr(5,600)
YYD3=fltarr(5,600)
ZZD3=fltarr(5,600)
XXD4=fltarr(5,600)
YYD4=fltarr(5,600)
ZZD4=fltarr(5,600)
XXD5=fltarr(5,600)
YYD5=fltarr(5,600)
ZZD5=fltarr(5,600)
XXD6=fltarr(5,600)
YYD6=fltarr(5,600)
ZZD6=fltarr(5,600)
xume=fltarr(45,5,600)
PPXDATA=FLTARR(600)
PPYDATA=FLTARR(600)
PPZDATA=FLTARR(600)
PPXDATA1=FLTARR(600)
PPXDATA2=FLTARR(600)
PPXDATA3=FLTARR(600)
PPYDATA1=FLTARR(600)
PPYDATA2=FLTARR(600)
PPYDATA3=FLTARR(600)
PPZDATA1=FLTARR(600)
PPZDATA2=FLTARR(600)
PPZDATA3=FLTARR(600)
OPXDATA=FLTARR(600)
OPYDATA=FLTARR(600)
OPZDATA=FLTARR(600)
OPXDATA1=FLTARR(600)
OPXDATA2=FLTARR(600)
OPXDATA3=FLTARR(600)
OPYDATA1=FLTARR(600)
OPYDATA2=FLTARR(600)
OPYDATA3=FLTARR(600)
OPZDATA1=FLTARR(600)
OPZDATA2=FLTARR(600)
OPZDATA3=FLTARR(600)
```

```
VMDATA=FLTARR(600)
VMDATA2=FLTARR(600)
BDATA=BYTARR(100)
NUMBER=INTARR(100)
pid=strarr(60)
pidd=strarr(1)
pidd1=strarr(250)
pidd2=strarr(1)
pidd3=strarr(1)
pidd4=strarr(1)
pidd5=strarr(1)
pidd6=strarr(1)
pidd7=strarr(1)
pidd8=strarr(15)
pidd9=strarr(1)
pidd0=strarr(1)
pidda=strarr(1)
piddb=strarr(1)
piddc=strarr(1)
piddd=strarr(1)
pidd='R&S Inc.: AIR Labs HFECG Analysis'
pidd0='Patient ID: '
piddd='-----------------------------------------------------------------
xxxx=STRARR(1)
xxx1=STRARR(1)
nnn='X'
S1=STRARR(31)
PATNAME=STRARR(100)
PAT=STRARR(1)
FILEX=STRARR(1)
FILEY=STRARR(1)
FILEZ=STRARR(1)
ssex=STRARR(1)
SRACE=STRARR(6)
SRACE(0)='Caucasian'
SRACE(1)='Black'
SRACE(2)='Oriental'
SRACE(3)='Hispanic'
SRACE(5)='Indian'
SRACE(4)='Unknown'
S3='.'
S4='1'
SX='x'
SY='y'
SZ='z'
S1(0)='0'
S1(1)='1'
S1(2)='2'
S1(3)='3'
S1(4)='4'
S1(5)='5'
S1(6)='6'
S1(7)='7'
S1(8)='8'
S1(9)='9'
S1(10)='10'
filen=2
for i=10,30 do begin
```

```
s1(i)=strtrim(i,2)
endfor
tablab2(0)=' ABS VAL  % TOT    %L00     %DAYREF'
tablab3(0)='X 0-10 Hz    '
tablab3(1)='Y 0-10 Hz    '
tablab3(2)='Z 0-10 Hz    '
tablab3(3)='X 10-60 Hz    '
tablab3(4)='Y 10-60 Hz    '
tablab3(5)='Z 10-60 Hz    '
tablab3(6)='V  10-60 Hz   '
tablab3(7)='X 60-150 Hz '
tablab3(8)='Y 60-150 Hz '
tablab3(9)='Z 60-150 Hz '
tablab3(10)='V  60-150 Hz'
tablab3(11)='X 150-250 Hz'
tablab3(12)='Y 150-250 Hz'
tablab3(13)='Z 150-250 Hz'
tablab3(14)='V 150-250 Hz'
tablab3(15)='X TOTAL     '
tablab3(16)='Y TOTAL     '
tablab3(17)='Z TOTAL     '
tablab4(0)='X 0-10 Hz    '
tablab4(1)='Y 0-10 Hz    '
tablab4(2)='Z 0-10 Hz    '
tablab4(3)='X 10-60 Hz    '
tablab4(4)='Y 10-60 Hz    '
tablab4(5)='Z 10-60 Hz    '
tablab4(6)='X 60-150 Hz '
tablab4(7)='Y 60-150 Hz '
tablab4(8)='Z 60-150 Hz '
tablab4(9)='X 150-250 Hz'
tablab4(10)='Y 150-250 Hz'
tablab4(11)='Z 150-250 Hz'
tablab4(12)='X TOTAL     '
tablab4(13)='Y TOTAL     '
tablab4(14)='Z TOTAL     '
tables(*,2)=100.0
tables(*,3)=100.0
for ii=0,20 do begin
ij=ii*4
tables(27:32,ij+1)=100.0
endfor
ref=strarr(15)
again=0
;****************************************************************
;        INITIALIZE PLOTTING DEVICE
;****************************************************************
for iii=0,255 do begin
rr(iii)=0
gg(iii)=0
bb(iii)=0
endfor
rr(160)=239
gg(160)=24
bb(160)=108
gg(96)=228
bb(206)=255
gg(206)=220
```

```
rr(254)=255
gg(254)=255
bb(28)=205
gg(28)=195
rr(28)=255
bb(10)=255
gg(10)=255
rr(10)=255
modifyct,4,4,rr,gg,bb
;****************************************************************
;  READ IN PATIENTS ID AND NUMBER
;****************************************************************
jumpagain:
PRINT, 'ENTER NUMBER OF PATIENTS TO BE PROCESSED'
READ, PATNUMBER
IF PATNUMBER EQ 0  THEN GOTO, JUMPOUT
FOR K=1,PATNUMBER DO BEGIN
PAT(*)=''
PRINT, 'FOR PATIENT ',strtrim(string(K),2),' ENTER NAME'
READ, PAT
PATNAME(K-1)=PAT
PRINT, PATNAME(K-1)
PRINT, 'FOR ', PATNAME(K-1), ' ENTER NUMBER OF DATASETS TO BE PROCESSED (M
READ, N
NUMBER(k-1)=N
ENDFOR ; /***End reading patient names and numbers ***/
;****************************************************************
;  LOOP FOR EACH PATIENT
;****************************************************************
FOR K=1,PATNUMBER DO BEGIN
nraz=intarr(30,3)
osd=strarr(10,7)
osdk=0
oxs=strarr(number(k-1))
oxx=fltarr(number(k-1),600)
oxy=fltarr(number(k-1),600)
oxz=fltarr(number(k-1),600)
LASTECG=1
numdays=0
for iiq=0,9 do begin
cca(iiq,5,1)=0.0
endfor
corref=0
jk=0
jk2=0
flag00= 0
IF NUMBER(K-1) EQ 0 THEN GOTO, JUMPOUT
if number(k-1) eq 1 then begin
flag00=1; flag for case of one dataset only
flag002=0;
number(k-1)=2
endif
jdiff=0
jumnum=0
flagjump=0
il=1
FOR J=1,NUMBER(K-1) DO BEGIN
if j eq 1 then begin
```

```
if NUMBER(K-1) ge 3 then begin
     print,' you have input PATIENT ',PATNAME(K-1),'  with ',NUMBER(K-1),'
     print,'ENTER a DATASET NUMBER TO BEGIN ANALYSIS OR 0 for default(0)'
     read,cn
     jumnum=fix(strtrim(cn,2))
     if jumnum eq number(k-1) then jumnum=jumnum-1
endif
endif
if j eq 2 then begin
if jumnum ne 0 then begin
j=jumnum
flagjump=1
jdiff=jumnum-j
endif
if flag00 eq 1 then begin
j=j-1
flag002=1
endif
endif
flexx=enter3(k)
if flexx gt 1 then goto, jumpat
print,filen
XXDATA=-1.0*XDATA
YYDATA=-1.0*YDATA
ZZDATA=-1.0*ZDATA
if j gt 1 then begin
if ecg ne lastecg or (j-jk) ge 4 then begin
jkk=jk
jn=j-jk-1
jk2=jk
jk=j-1
if flagjump eq 0 then begin
if jkk ne 0 then begin
device,pseudo=8
WINDOW, 0, colors=128, YPOS=80, XSIZE=1150, ySIZE=820, title=pidd
WINDOW, 5, colors=128, XSIZE=750, ypos=20,ySIZE=50, title=pidd
mmatdly0,jn;
PRINT,'ENTER X TO EXIT OR ANY OTHER CHAR TO CONTINUE'
READ,xxxx
if (xxxx(0) eq nnn) then goto, jump1
mmadly0,jn;
endif $
else begin
mmatdly,jn;
PRINT,'ENTER X TO EXIT OR ANY OTHER CHAR TO CONTINUE'
READ,xxxx
if (xxxx(0) eq nnn) then goto, jump1
mmadly2,jn;
endelse
pidd8(12)=''
PRINT,'ENTER X TO EXIT OR ANY OTHER CHAR TO CONTINUE'
READ,xxxx
if (xxxx(0) eq nnn) then goto, jump1
wdelete,1
;**********************************************************************
;CALCULATE THE SCORES OF THE PATIENT COMPARED TO THE NORMAL POPULATION
;**********************************************************************
scorez,tables,nraz,jkk,jn,csd,osdk
```

```
PRINT,'ENTER X TO EXIT OR ANY OTHER CHAR TO CONTINUE'
READ,xxxx
if (xxxx(0) eq nnn) then goto, jump1
scorefs2,oxs,jkk,jk-1,oxx,oxy,oxz
PRINT,'ENTER X TO EXIT OR ANY OTHER CHAR TO CONTINUE'
READ,xxxx
if (xxxx(0) eq nnn) then goto, jump1
wdelete,0
wdelete,5
endif
endif
endif
flagjump=0
pidd8(12)=pidd8(12)+s55+' '
;*****************************************************************
;     BEGIN PROCESSING AND PLOTTING
;*****************************************************************
piddc=pidd+'    '+pidd0
tablabl(j-1)=s55
if (again eq 0) then begin
numdays=0
corref=0
loadct,4
status='WELCOME TO'
status='Press any character to continue'
wdelete,0
!P.BACKGROUND=1
endif
status='DISPLAY STATUS:  Time Domain Plots for X, Y, Z ECG leads'
!Y.MARGIN=[3,4]
!X.STYLE=1
!X.CRANGE=[0,599]
!P.BACKGROUND=1
!P.MULTI=[0,4,4,0,0]
!Y.TITLE='amplitude (microvolts)'
!Y.TITLE='sample number'
!P.CHARSIZE=1.35
!X.CHARSIZE=1.35
!Y.CHARSIZE=1.35
;****************************************************************
; FILTERING THE PATIENT DATA INTO 4 BANDS
;****************************************************************
mmfilter6
scorefs,xdata,ydata,zdata,xr,oxsd
Oxs(j-1)=oxsd
oxx(j-1,0:599)=xdata(0:599)
oxy(j-1,0:599)=ydata(0:599)
oxz(j-1,0:599)=zdata(0:599)
wdelete,1
status='DISPLAY STATUS:  Power Spectral Density Plots for X, Y, Z ECG lead
;*****************************************************************
;POWER SPECTRUM CALCULATION AND DISPLAY
;*****************************************************************
mmpower6
wdelete,1
tabnam(1,j-1)=pxx
tabnam(2,j-1)=pidd2
for ind=0,14 do begin
```

```
jj=(j-1)*4
tables(ind,jj)=xr(ind)
tables(ind+15,jj)=xr(ind+50)
endfor
for ind=0,2 do begin
jj=(j-1)*4
ij=16+(ind*6)
ind3=ind*4
ind1=ind*4+3
ind2=ind*3+3
tables(ind1,jj+1)=xr(ij)
tables(ind1+1,jj+1)=xr(ij+2)
tables(ind1+2,jj+1)=xr(ij+4)
tables(ind2+15,jj+1)=xr(ij+47)
tables(ind2+16,jj+1)=xr(ij+49)
tables(ind2+17,jj+1)=xr(ij+51)
tables(ind1+3,jj+1)=xr(ij-ind3-1)
endfor
for ind=0,2 do begin
jj=(j-1)*4
tables(27+ind,jj)=xr(40+ind)
tables(30+ind,jj)=xr(90+ind)
tables(ind,jj+1)=xr(43+ind)
tables(ind+15,jj+1)=xr(93+ind)
endfor
if (ecg ne lastecg or (j-jk2) ge 4) then begin
if (j gt 1) then begin
jk2=jk
pidd8(0)=s55
piddl(7)=' first datasets of successive days: '
numdays=numdays+1
;*****************************************************************
; DAY - DAY COMPARISON
;*****************************************************************
il=j
mmtimexs,il,j,nraz
wdelete,1
;*******************************
; correlations
;*******************************
cca(numdays, 6, 0)=correlate(oxdata,xdata)
cca(numdays, 6, 1)=correlate(oydata,ydata)
cca(numdays, 6, 2)=correlate(ozdata,zdata)
cca(numdays, 6, 3)=correlate(oxdata1,xdata1)
cca(numdays, 6, 4)=correlate(oydata1,ydata1)
cca(numdays, 6, 5)=correlate(ozdata1,zdata1)
cca(numdays, 6, 6)=correlate(oxdata2,xdata2)
cca(numdays, 6, 7)=correlate(oydata2,ydata2)
cca(numdays, 6, 8)=correlate(ozdata2,zdata2)
cca(numdays, 6, 9)=correlate(oxdata3,xdata3)
cca(numdays, 6, 10)=correlate(oydata3,ydata3)
cca(numdays, 6, 11)=correlate(ozdata3,zdata3)
cca(numdays, 6, 12)=correlate(opxdata,pxdata)
cca(numdays, 6, 13)=correlate(opydata,pydata)
cca(numdays, 6, 14)=correlate(opzdata,pzdata)
cca(numdays, 6, 15)=correlate(opxdata1,pxdata1)
cca(numdays, 6, 16)=correlate(opydata1,pydata1)
cca(numdays, 6, 17)=correlate(opzdata1,pzdata1)
```

```
cca(numdays, 6, 18)=correlate(opxdata2,pxdata2)
cca(numdays, 6, 19)=correlate(opydata2,pydata2)
cca(numdays, 6, 20)=correlate(opzdata2,pzdata2)
cca(numdays, 6, 21)=correlate(opxdata3,pxdata3)
cca(numdays, 6, 22)=correlate(opydata3,pydata3)
cca(numdays, 6, 23)=correlate(opzdata3,pzdata3)
;***************************************************************
; day - day Differential Power Spectrum Plots
;***************************************************************
mmadiff6
for jj=0,14 do begin
ind=(j-1)*4
tables(jj,ind+3)=xr(234+jj)
tables(jj,ind+2)=xr(334+jj)
endfor
for jj=0,11 do begin
tables(jj+15,ind+3)=xr(162+jj)
tables(jj+15,ind+2)=xr(462+jj)
endfor
for jj=0,2 do begin
tables(27+jj,ind+3)=xr(428+jj)
tables(27+jj,ind+2)=xr(441+jj)
tables(30+jj,ind+3)=xr(478+jj)
tables(30+jj,ind+2)=xr(491+jj)
endfor
endif
jumpx:
;*******************************************************/
;       start intra day plots
;*********************************************************/
ref(corref)=s55
corref=corref+1
pidd9(0)=s55
xr(102)=xr(100)
xr(103)=xr(101)
if (j eq 1) then begin
OX=XDATA
OY=YDATA
OZ=ZDATA
OX1=XDATA1
OY1=YDATA1
OZ1=ZDATA1
OX1A=XDATA1A
OY1A=YDATA1A
OZ1A=ZDATA1A
OX2=XDATA2
OY2=YDATA2
OZ2=ZDATA2
OX3=XDATA3
OY3=YDATA3
OZ3=ZDATA3
OXN=XXDATA
OYN=YYDATA
OZN=ZZDATA
OVM2=VMDATA2
OVM=VMDATA
ok30=k3&ok40=k4&ox30=x3&ox40=x4
for i=0,14 do begin
```

```
xr(300+i)=xr(i)
xr(350+i)=xr(i+50)
endfor
for i=0,5 do begin
xr(438+i)=xr(40+i)
xr(488+i)=xr(90+i)
endfor
OPX=PXDATA
OPY=PYDATA
OPZ=PZDATA
OPX1A=XDATA1A
OPY1A=YDATA1A
OPZ1A=ZDATA1A
OPX1=PXDATA1
OPX2=PXDATA2
OPX3=PXDATA3
OPY1=PYDATA1
OPY2=PYDATA2
OPY3=PYDATA3
OPZ1=PZDATA1
OPZ2=PZDATA2
OPZ3=PZDATA3
endif
;*************************************************************************
;j eq 1: store 100
;*************************************************************************
jj=(j-1)*4+3
tables(*,jj)=100.0
OXDATA=XDATA
OYDATA=YDATA
OZDATA=ZDATA
Xume(10,0,0:599)=xdata1a(0:599)
Xume(11,0,0:599)=ydata1a(0:599)
Xume(12,0,0:599)=zdata1a(0:599)
OXDATA1=XDATA1
OYDATA1=YDATA1
OZDATA1=ZDATA1
OXDATA2=XDATA2
OYDATA2=YDATA2
OZDATA2=ZDATA2
OXDATA3=XDATA3
OYDATA3=YDATA3
OZDATA3=ZDATA3
OXDATAN=XXDATA
OYDATAN=YYDATA
OZDATAN=ZZDATA
OVMDATA2=VMDATA2
OVMDATA=VMDATA
for i=0,14 do begin
xr(200+i)=xr(i)
xr(250+i)=xr(i+50)
endfor
for i=0,5 do begin
xr(425+i)=xr(40+i)
xr(475+i)=xr(90+i)
endfor
OPXDATA=PXDATA
OPYDATA=PYDATA
```

```
OPZDATA=PZDATA
OPXDATA1=PXDATA1
OPXDATA2=PXDATA2
OPXDATA3=PXDATA3
OPYDATA1=PYDATA1
OPYDATA2=PYDATA2
OPYDATA3=PYDATA3
OPZDATA1=PZDATA1
OPZDATA2=PZDATA2
OPZDATA3=PZDATA3
endif
if (j gt 1) then begin
if (ecg eq lastecg) then begin
cca(numdays, 5,1)=cca(numdays,5,1)+1.0
pidd8(0)=s55
piddl(7)=' Pre and Post CP '
;
;*****************************************************************
;          Time plots
;*****************************************************************
mmtimexs,il,j,nraz
wdelete,1
mmadiff6
for jj=0,14 do begin
ind=(j-1)*4
tables(jj,ind+3)=xr(234+jj)
tables(jj,ind+2)=xr(334+jj)
endfor
for jj=0,11 do begin
tables(jj+15,ind+3)=xr(162+jj)
tables(jj+15,ind+2)=xr(462+jj)
endfor
for jj=0,2 do begin
tables(27+jj,ind+3)=xr(428+jj)
tables(27+jj,ind+2)=xr(441+jj)
tables(30+jj,ind+3)=xr(478+jj)
tables(30+jj,ind+2)=xr(491+jj)
endfor
;****************************
; correlations
;******************************
dayset=fix(cca(numdays,5,1))
cca(numdays, dayset, 0)=correlate(oxdata,xdata)
cca(numdays, dayset, 1)=correlate(oydata,ydata)
cca(numdays, dayset, 2)=correlate(ozdata,zdata)
cca(numdays, dayset, 3)=correlate(oxdata1,xdata1)
cca(numdays, dayset, 4)=correlate(oydata1,ydata1)
cca(numdays, dayset, 5)=correlate(ozdata1,zdata1)
cca(numdays, dayset, 6)=correlate(oxdata2,xdata2)
cca(numdays, dayset, 7)=correlate(oydata2,ydata2)
cca(numdays, dayset, 8)=correlate(ozdata2,zdata2)
cca(numdays, dayset, 9)=correlate(oxdata3,xdata3)
cca(numdays, dayset, 10)=correlate(oydata3,ydata3)
cca(numdays, dayset, 11)=correlate(ozdata3,zdata3)
cca(numdays, dayset, 12)=correlate(opxdata,pxdata)
cca(numdays, dayset, 13)=correlate(opydata,pydata)
cca(numdays, dayset, 14)=correlate(opzdata,pzdata)
cca(numdays, dayset, 15)=correlate(opxdata1,pxdata1)
```

```
cca(numdays, dayset, 16)=correlate(opydata1,pydata1)
cca(numdays, dayset, 17)=correlate(opzdata1,pzdata1)
cca(numdays, dayset, 18)=correlate(opxdata2,pxdata2)
cca(numdays, dayset, 19)=correlate(opydata2,pydata2)
cca(numdays, dayset, 20)=correlate(opzdata2,pzdata2)
cca(numdays, dayset, 21)=correlate(opxdata3,pxdata3)
cca(numdays, dayset, 22)=correlate(opydata3,pydata3)
cca(numdays, dayset, 23)=correlate(opzdata3,pzdata3)
js=j-jk-1
XXD(js,0:599)=PXDATA(0:599)
YYD(js,0:599)=PYDATA(0:599)
ZZD(js,0:599)=PZDATA(0:599)
XXD1(js,0:599)=PXDATA1(0:599)
YYD1(js,0:599)=PYDATA1(0:599)
ZZD1(js,0:599)=PZDATA1(0:599)
XXD2(js,0:599)=PXDATA2(0:599)
YYD2(js,0:599)=PYDATA2(0:599)
ZZD2(js,0:599)=PZDATA2(0:599)
XXD3(js,0:599)=PXDATA3(0:599)
YYD3(js,0:599)=PYDATA3(0:599)
ZZD3(js,0:599)=PZDATA3(0:599)
Xume(0,js,0:599)=XDATA1A(0:599)
xume(1,js,0:599)=YDATA1A(0:599)
xume(2,js,0:599)=ZDATA1A(0:599)
XXD4(js,0:599)=XDATA1(0:599)
YYD4(js,0:599)=YDATA1(0:599)
ZZD4(js,0:599)=ZDATA1(0:599)
XXD5(js,0:599)=XDATA2(0:599)
YYD5(js,0:599)=YDATA2(0:599)
ZZD5(js,0:599)=ZDATA2(0:599)
XXD6(js,0:599)=XDATA3(0:599)
YYD6(js,0:599)=YDATA3(0:599)
ZZD6(js,0:599)=ZDATA3(0:599)
xume(20,js,0:599)=xume(20,0,0:599)
xume(21,js,0:599)=xume(21,0,0:599)
xume(22,js,0:599)=xume(22,0,0:599)
xume(23,js,0:599)=xume(23,0,0:599)
xume(24,js,0:599)=xume(24,0,0:599)
xume(25,js,0:599)=xume(25,0,0:599)
xume(26,js,0:599)=xume(26,0,0:599)
xume(27,js,0:599)=xume(27,0,0:599)
xume(28,js,0:599)=xume(28,0,0:599)
xume(29,js,0:599)=xume(29,0,0:599)
xume(30,js,0:599)=xume(30,0,0:599)
xume(31,js,0:599)=xume(31,0,0:599)
xume(32,js,0:599)=xume(32,0,0:599)
xume(33,js,0:599)=xume(33,0,0:599)
xume(34,js,0:599)=xume(34,0,0:599)
xume(35,js,0:599)=xume(35,0,0:599)
xume(36,js,0:599)=xume(36,0,0:599)
xume(37,js,0:599)=xume(37,0,0:599)
xume(38,js,0:599)=xume(38,0,0:599)
xume(39,js,0:599)=xume(39,0,0:599)
xume(40,js,0:599)=xume(40,0,0:599)
xume(41,js,0:599)=xume(41,0,0:599)
xume(42,js,0:599)=xume(42,0,0:599)
xume(43,js,0:599)=xume(43,0,0:599)
endif
```

```
endif
;**********************************************************************
;END OF PROCESSING AND PLOTTING
;**********************************************************************
CLOSE, 1
wdelete,1
LASTECG=ECG
again=1
print,'fllllllllllllaaaaaaaaggggg',number(k-1),j,flag00
ENDFOR
jn=j-jk-1;
pidd8(11)=pidd2
if jk ne 0 then begin
WINDOW, 0, colors=128, YPOS=80, XSIZE=1150, ySIZE=820, title=pidd
WINDOW, 5, colors=128, XSIZE=750, ypos=20,ySIZE=50, title=pidd
mmatdly0,jn;
PRINT, 'ENTER X TO EXIT OR ANY OTHER CHAR TO CONTINUE'
READ, xxxx
if( xxxx(0) eq nnn) then goto, jump1
mmadly0,jn;
endif $
else begin
mmatdly,jn;
PRINT, 'ENTER X TO EXIT OR ANY OTHER CHAR TO CONTINUE'
READ, xxxx
if( xxxx(0) eq nnn) then goto, jump1
mmadly2,jn;
endelse
pidd8(12)=''
PRINT, 'ENTER X TO EXIT OR ANY OTHER CHAR TO CONTINUE'
READ, xxxx
if( xxxx(0) eq nnn) then goto, jump1
if jk eq 0 then begin jkk=0&jk=j-1&endif else begin jkk=jk&jk=j-1&endelse
scorez,tables,nraz,jkk,jn,osd,osdk
PRINT, 'ENTER X TO EXIT OR ANY OTHER CHAR TO CONTINUE'
READ, xxxx
if( xxxx(0) eq nnn) then goto, jump1
scorefs2,oxs,jkk,jk-1,oxx,oxy,oxz
PRINT,'ENTER X TO EXIT OR ANY OTHER CHAR TO CONTINUE'
READ,xxxx
if (xxxx(0) eq nnn) then goto, jump1
wdelete,5
wdelete,0
if(j eq 2) then goto, jump2
for eeq=0,numdays do begin
ddset(eeq)=fix(cca(eeq,5,1))
endfor
numn=number(k-1)-jdiff
mmnorma, tables, numn,ddset,numdays
mmnorm, tables, numn,ddset,numdays
sdat='L'
eed=0
for eeq=0,numdays do begin
dayset=fix(cca(eeq,5,1))
if ( eeq gt 0) then begin
yyy=0.90
xx=-0.07
yyy=yyy-0.05
```

```
yyy=yyy-0.05
yyy=yyy-0.05
for dll=0,23 do begin
if( dll eq 12) then begin
yyy=yyy-0.05
yyy=yyy-0.05
xx=-0.07
endif
xx=xx+0.08
endfor
endif
if ( dayset gt 0) then begin
yyy=0.90
xx=-0.07
yyy=yyy-0.05
endif
if(eeq gt 0) then begin
eed=eed+cca(eeq-1,5,1)+1
endif
for dq=0,dayset-1 do begin
xx=-0.07
eeqqd=dq+1+eed
if eeqqd gt 10 then sd=sdat+sl(dq+1+eed) else sd=sdat+sl(0)+sl(eeqqd)
yyy=yyy-0.05
yyy=yyy-0.05
for dll=0,23 do begin
if( dll eq 12) then begin
yyy=yyy-0.05
XYOUTS, 0.0, yyy, SIZE=0.9, 'Between Power Plots:', /Normal, /noclip,color
yyy=yyy-0.05
xx=-0.07
endif
xx=xx+0.08
XYOUTS, xx, yyy, SIZE=0.7, cca(eeq,dq+1,dll), /Normal, /noclip,color=127
endfor
yyy=yyy-0.20
endfor
again=1
endfor
jump2:
wdelete,0
;************************************************************
; STORE THE PARIENTS DATA IN TABLES
;************************************************************
mmtablesz,k,sdat,numdays,osd
close,1
close,2
wdelete,5
PRINT, 'END OF PROCESSING FOR: '+PAT
jumpat:
ENDFOR
;***************************************************************************
;End loop for patient
;***************************************************************************
jumpout:
again=1
jump1:
close,1
```

| Dec 20 1994 19:31:35 | ecgzx.pro | Page 15 |
|---|---|---|

```
close,2
wdelete,5
wdelete,0
wdelete,1
SPAWN, 'rm *.lis'
END
```

| Dec 20 1994 19:29:09 | scorez.pro | Page 1 |
|---|---|---|

```
;*************************************************************************
; THIS SUBROUTINE CALCULATES THE TEST SCORES FOR THE PATIENT
; COMPARED TO THE NORMAL POPULATION
;*************************************************************************
pro scorez,tables,nraz,jk,jn,osd,osdk
;******************************************************************
; initialize arrays
;******************************************************************
nst=28
rj=4*jk
rjj=rj+1
ss=fltarr(nst)
osd(osdk,0)=''
osd(osdk,1)=''
osd(osdk,2)=''
osd(osdk,3)=''
osd(osdk,4)=''
osd(osdk,5)=''
osd(osdk,6)=''
;******************************************************************
;read the normal population mean and standard deviation
; for each test
;******************************************************************
av=fltarr(nst)
av2=fltarr(nst)
get_lun,vb
openr,vb,'avgl'
non=0
readu,vb,non
readu,vb,av
readu,vb,av2
cc=fltarr(nst,non)
readu,vb,cc
cc2=intarr(nst)
close,vb
;******************************************************************
;calculate the patient's scores in 28 tests by comparing with
; the mean and the standard deviation of the normals for
; each test
;******************************************************************
ss(0)=(tables(0,rjj)-av(0))/av2(0)
if ss(0) ge 1 then begin $
xc=cc(0,*) ge tables(0,rjj)
xct=fix(total(xc)/non*100)
cc2(0)=xct
endif

ss(1)=(av(1)-tables(3,rjj))/av2(1)
if ss(1) ge 1 then begin $
xc=cc(1,*) le tables(3,rjj)
xct=fix(total(xc)/non*100)
cc2(1)=xct
endif

ss(2)=(tables(7,rjj)-av(2))/av2(2)
if ss(2) ge 1 then begin $
xc=cc(2,*) ge tables(7,rjj)
xct=fix(total(xc)/non*100)
```

```
cc2(2)=xct
endif

ss(3)=(tables(11,rjj)-av(3))/av2(3)
if ss(3) ge 1 then begin $
xc=cc(3,*) ge tables(11,rjj)
xct=fix(total(xc)/non*100)
cc2(3)=xct
endif

ss(4)=(tables(1,rjj)-av(4))/av2(4)
gg=ss(4)&ss(4)=abs(ss(4))
if gg ge 1 then begin $
xc=cc(4,*) ge tables(1,rjj)
xct=fix(total(xc)/non*100)
cc2(4)=xct
endif else if gg le -1 then begin $
xc=cc(4,*) le tables(1,rjj)
xct=fix(total(xc)/non*100)
cc2(4)=xct
endif

ss(5)=(tables(4,rjj)-av(5))/av2(5)
gg=ss(5)&ss(5)=abs(ss(5))
if gg ge 1 then begin $
xc=cc(5,*) ge tables(4,rjj)
xct=fix(total(xc)/non*100)
cc2(5)=xct
endif else if gg le -1 then begin $
xc=cc(5,*) le tables(4,rjj)
xct=fix(total(xc)/non*100)
cc2(5)=xct
endif

ss(6)=(tables(8,rjj)-av(6))/av2(6)
if ss(6) ge 1 then begin $
xc=cc(6,*) ge tables(8,rjj)
xct=fix(total(xc)/non*100)
cc2(6)=xct
endif

ss(7)=(tables(12,rjj)-av(7))/av2(7)
if ss(7) ge 1 then begin $
xc=cc(7,*) ge tables(12,rjj)
xct=fix(total(xc)/non*100)
cc2(7)=xct
endif

ss(8)=(tables(2,rjj)-av(8))/av2(8)
if ss(8) ge 1 then begin $
xc=cc(8,*) ge tables(2,rjj)
xct=fix(total(xc)/non*100)
cc2(8)=xct
endif

ss(9)=(av(9)-tables(5,rjj))/av2(9)
if ss(9) ge 1 then begin $
xc=cc(9,*) le tables(5,rjj)
```

```
xct=fix(total(xc)/non*100)
cc2(9)=xct
endif

ss(10)=(av(10)-tables(9,rjj))/av2(10)
if ss(10) ge 1 then begin $
xc=cc(10,*) le tables(9,rjj)
xct=fix(total(xc)/non*100)
cc2(10)=xct
endif

ss(11)=(av(11)-tables(13,rjj))/av2(11)
if ss(11) ge 1 then begin $
xc=cc(11,*) le tables(13,rjj)
xct=fix(total(xc)/non*100)
cc2(11)=xct
endif

ss(12)=(av(12)-tables(0,rj))/av2(12)
if ss(12) ge 1 then begin $
xc=cc(12,*) le tables(0,rj)
xct=fix(total(xc)/non*100)
cc2(12)=xct
endif

ss(13)=(av(13)-tables(3,rj))/av2(13)
if ss(13) ge 1 then begin $
xc=cc(13,*) le tables(3,rj)
xct=fix(total(xc)/non*100)
cc2(13)=xct
endif

ss(14)=(av(14)-tables(7,rj))/av2(14)
if ss(14) ge 1 then begin $
xc=cc(14,*) le tables(7,rj)
xct=fix(total(xc)/non*100)
cc2(14)=xct
endif

ss(15)=(av(15)-tables(27,rj))/av2(15)
if ss(15) ge 1 then begin $
xc=cc(15,*) le tables(27,rj)
xct=fix(total(xc)/non*100)
cc2(15)=xct
endif

xg=tables(27,rj)/tables(28,rj)
ss(16)=(xg-av(16))/av2(16)
if ss(16) ge 1 then begin $
xc=cc(16,*) ge xg
xct=fix(total(xc)/non*100)
cc2(16)=xct
endif

ss(17)=(av(17)-tables(1,rj))/av2(17)
if ss(17) ge 1 then begin $
xc=cc(17,*) le tables(1,rj)
xct=fix(total(xc)/non*100)
```

```
cc2(17)=xct
endif

ss(18)=(av(18)-tables(4,rj))/av2(18)
if ss(18) ge 1 then begin $
xc=cc(18,*) le tables(4,rj)
xct=fix(total(xc)/non*100)
cc2(18)=xct
endif

ss(19)=(av(19)-tables(8,rj))/av2(19)
if ss(19) ge 1 then begin $
xc=cc(19,*) le tables(8,rj)
xct=fix(total(xc)/non*100)
cc2(19)=xct
endif


ss(20)=(av(20)-tables(12,rj))/av2(20)
if ss(20) ge 1 then begin $
xc=cc(20,*) le tables(12,rj)
xct=fix(total(xc)/non*100)
cc2(20)=xct
endif

ss(21)=(av(21)-tables(28,rj))/av2(21)
if ss(21) ge 1 then begin $
xc=cc(21,*) le tables(28,rj)
xct=fix(total(xc)/non*100)
cc2(21)=xct
endif

xg=tables(28,rj)/tables(29,rj)
ss(22)=(av(22)-xg)/av2(22)
if ss(22) ge 1 then begin $
xc=cc(22,*) le xg
xct=fix(total(xc)/non*100)
cc2(22)=xct
endif

ss(23)=(tables(2,rj)-av(23))/av2(23)
if ss(23) ge 1 then begin $
xc=cc(23,*) ge tables(2,rj)
xct=fix(total(xc)/non*100)
cc2(23)=xct
endif

ss(24)=(av(24)-tables(9,rj))/av2(24)
if ss(24) ge 1 then begin $
xc=cc(24,*) le tables(9,rj)
xct=fix(total(xc)/non*100)
cc2(24)=xct
endif

ss(25)=(av(25)-tables(13,rj))/av2(25)
if ss(25) ge 1 then begin $
xc=cc(25,*) le tables(13,rj)
xct=fix(total(xc)/non*100)
```

```
cc2(25)=xct
endif

ss(26)=(tables(29,rj)-av(26))/av2(26)
if ss(26) ge 1 then begin $
xc=cc(26,*) ge tables(29,rj)
xct=fix(total(xc)/non*100)
cc2(26)=xct
endif

xg=tables(27,rj)/tables(29,rj)
ss(27)=(av(27)-xg)/av2(27)
if ss(27) ge 1 then begin $
xc=cc(27,*) le xg
xct=fix(total(xc)/non*100)
cc2(27)=xct
endif
;*****************************************************************
;store the test scores for the patient
;*****************************************************************
xc2=strarr(nst)
p=strarr(nst)
ss1=ss gt 1
ss9=fix(ss*ss1)
trt=0
for j=0,27 do begin
if ss9(j) lt 1 then begin $
xc2(j)='    '
endif else  begin $
xc2(j)=strtrim(string(cc2(j)),1)+'   '
if cc2(j) lt 5 then trt=trt+ss9(j)
endelse
endfor

ms=intarr(4)&md=intarr(2)
vs=strarr(nst)
for i=0,27 do vs(i)=strtrim(string(ss9(i)),2)
osd(osdk,0)=vs(12)+'  '+vs(13)+'  '+vs(14)+'     '+vs(15)+'   '+vs(16)+$
'      '+vs(17)+'  '+vs(18)+'  '+vs(19)+'  '+vs(20)+'  '+vs(21)+'  '+vs(22)
'      '+vs(23)+'    '+vs(24)+'  '+vs(25)+'  '+vs(26)+'  '+vs(27)+$
'       '+string(trt)

osd(osdk,5)=vs(0)+'  '+vs(1)+'  '+vs(2)+'  '+vs(3)+'         '+$
vs(4)+'  '+vs(5)+'  '+vs(6)+'  '+vs(7)+'         '+$
vs(8)+'  '+vs(9)+'  '+vs(10)+'  '+vs(11)

for i=0,27 do p(i)=strmid(xc2(i),0,3)
osd(osdk,4)=p(12)+p(13)+p(14)+'  '+p(15)+p(16)+$
'    '+p(17)+p(18)+p(19)+p(20)+p(21)+p(22)+$
'    '+p(23)+'  '+p(24)+p(25)+p(26)+p(27)
osd(osdk,6)=p(0)+p(1)+p(2)+p(3)+$
'          '+p(4)+p(5)+p(6)+p(7)+$
'          '+p(8)+p(9)+p(10)+p(11)
return
end
```

```
;***********************************************************************
;THIS SUBROUTINE FILTERS THE X,Y,Z SIGNALS INTO 4 BANDS : 0-10 Hz,
; 10-60 Hz, 60-150 Hz, and 150-250 Hz
;***********************************************************************
PRO MMFILTER6
;*******************************************************
; INITIALIZE ARRAYS
;*******************************************************
COMMON SP1, K3, K4, X3, X4, K3D, K4D
COMMON SP2, PXDATA, PYDATA, PZDATA, PXDATA1, PXDATA2, PXDATA3, PYDATA1
COMMON SP3, PYDATA2, PZDATA2, PZDATA1, PYDATA3, PZDATA3, XDATA, YDATA, ZDA
COMMON SP4, pidd0
COMMON SP5, XDATA1, YDATA1, ZDATA1, XDATA2, YDATA2, ZDATA2, XDATA3,YDATA3,
COMMON SP6, VMDATA2, pid,pidd1,pidd2,pidd3,pidd4,pidd5,pidd6,pidd7,pidd8,p
COMMON SP7, VMDATA
COMMON SP8, XR
COMMON SP9, OXDATA, OYDATA, OZDATA, OXDATA1, OXDATA2,OXDATA3, OXDATAN,OVMD
COMMON SP10, OVMDATA, OYDATA1,OYDATA2,OYDATA3,OZDATA1,OZDATA2,OZDATA3,OYDA
COMMON SP11, OZDATAN, OPXDATA1,OPXDATA2,OPXDATA3,OPXDATA, OPYDATA,OPYDATA1
COMMON SP12, OPYDATA2,OPYDATA3, OPZDATA,OPZDATA1,OPZDATA2,OPZDATA3
COMMON SP13, PIDDA
COMMON SP16, XDATA1A,YDATA1A,ZDATA1A,XXD,YYD,ZZD,XXD1,YYD1,ZZD1,XXD2,YYD2,
OUARR=FLTARR(4,600)
k3=FLTARR(600)
k4=FLTARR(600)
X3=FLTARR(600)
x4=FLTARR(600)
XINDEX=INTARR(600)
XINDEX2=INTARR(150)
XINDEX3=INTARR(256)
XINDEX4=INTARR(100)
IDATA=INTARR(2096)
xxxx=STRARR(1)
piddc=pidd+'   '+pidd0
id1=fix(xr(100))
id2=fix(xr(101))
scale1=25.0
scale2=2.0
scale3=0.5
for i=0,599 do begin
xindex(i)=i
endfor
smooth,xdata
smooth,ydata
smooth,zdata
xr(40)=rms(xdata,id1,id2)
xr(41)=rms(ydata,id1,id2)
xr(42)=rms(zdata,id1,id2)
xr(85)=rms(xdata,1,id1)
xr(86)=rms(ydata,1,id1)
xr(87)=rms(zdata,1,id1)
;*******************************************************
;FILTER INTO THE 0-10 Hz and 10-60 Hz  bands
;*******************************************************
nn=70
vnbc=digital_filter(0.02,0.12,70,nn)
xdata1=padcon(xdata,vnbc,nn)
xr(3)=rms(xdata1,id1,id2)
```

```
vnbc1=digital_filter(0.0,0.02,70,nn)
xdata1a=padcon(xdata,vnbc1,nn)
xr(0)=rms(xdata1a,id1,id2)
ydata1=padcon(ydata,vnbc,nn)
xr(4)=rms(ydata1,id1,id2)
ydata1a=padcon(ydata,vnbc1,nn)
xr(1)=rms(ydata1a,id1,id2)
zdata1=padcon(zdata,vnbc,nn)
xr(5)=rms(zdata1,id1,id2)
zdata1a=padcon(zdata,vnbc1,nn)
xr(2)=rms(zdata1a,id1,id2)
xr(88)=rms(xdata1,1,id1)
xr(89)=rms(ydata1,1,id1)
xr(90)=rms(zdata1,1,id1)
xr(91)=rms(xdata1a,1,id1)
xr(92)=rms(ydata1a,1,id1)
xr(93)=rms(zdata1a,1,id1)
VMDATA=VECT(XDATA1, YDATA1, ZDATA1)
xr(6)=rms(vmdata,id1,id2)
;*****************************************************
;FILTER INTO THE 60-150 Hz band
;*****************************************************
vnbc=digital_filter(0.12,0.3,70,nn)
xdata2=padcon(xdata,vnbc,nn)
xr(7)=rms(xdata2,id1,id2)
ydata2=padcon(ydata,vnbc,nn)
xr(8)=rms(ydata2,id1,id2)
zdata2=padcon(zdata,vnbc,nn)
xr(9)=rms(zdata2,id1,id2)
xr(94)=rms(xdata2,1,id1)
xr(95)=rms(ydata2,1,id1)
xr(96)=rms(zdata2,1,id1)
VMDATA=VECT(XDATA2, YDATA2, ZDATA2)
VMDATA2=VMDATA
xr(10)=rms(vmdata,id1,id2)
;*****************************************************
;FILTER INTO THE 150-250 Hz band
;*****************************************************
vnbc=digital_filter(0.3,0.5,70,nn)
xdata3=padcon(xdata,vnbc,nn)
xr(11)=rms(xdata3,id1,id2)
ydata3=padcon(ydata,vnbc,nn)
xr(12)=rms(ydata3,id1,id2)
zdata3=padcon(zdata,vnbc,nn)
xr(13)=rms(zdata3,id1,id2)
xr(97)=rms(xdata3,1,id1)
xr(98)=rms(ydata3,1,id1)
xr(99)=rms(zdata3,1,id1)
VMDATA=VECT(XDATA3, YDATA3, ZDATA3)
;*****************************************************
;STORE THE FILTERED SIGNALS
;*****************************************************
xr(14)=rms(vmdata,id1,id2)
xtot=xr(3)+xr(7)+xr(11)+xr(0)
ytot=xr(4)+xr(8)+xr(12)+xr(1)
ztot=xr(5)+xr(9)+xr(13)+xr(2)
xr(15)=xr(3)/xr(40)*100.0
xr(16)=xr(3)/xtot*100.0
```

```
xr(17)=xr(4)/xr(41)*100.0
xr(18)=xr(4)/ytot*100.0
xr(19)=xr(5)/xr(42)*100.0
xr(20)=xr(5)/ztot*100.0
xr(43)=xr(0)/xtot*100.0
xr(44)=xr(1)/ytot*100.0
xr(45)=xr(2)/ztot*100.0
vtot=xr(6)+xr(10)+xr(14)
xr(15)=xr(6)/vtot*100.0
xr(17)=xr(10)/vtot*100.0
xr(19)=xr(14)/vtot*100.0
xr(21)=xr(7)/xr(40)*100.0
xr(22)=xr(7)/xtot*100.0
xr(23)=xr(8)/xr(41)*100.0
xr(24)=xr(8)/ytot*100.0
xr(25)=xr(9)/xr(42)*100.0
xr(26)=xr(9)/ztot*100.0
xr(27)=xr(11)/xr(40)*100.0
xr(28)=xr(11)/xtot*100.0
xr(29)=xr(12)/xr(41)*100.0
xr(30)=xr(12)/ytot*100.0
xr(31)=xr(13)/xr(42)*100.0
xr(32)=xr(13)/ztot*100.0
xr(40)=xtot
xr(41)=ytot
xr(42)=ztot
RETURN
END
```

Dec 20 1994 20:31:15 **avgasc**

NORMAL POPULATION MEANS AND STANDARD DEVIATIONS

| | MEAN | STANDARD DEVIATION |
|---|---|---|
| P1 | 571.083 | 144.154 |
| P2 | 246.654 | 74.5161 |
| P3 | 305.662 | 75.3298 |
| P4 | 15.7657 | 7.11228 |
| P5 | 2.98628 | 1.36906 |
| P6 | 624.600 | 183.909 |
| P7 | 280.751 | 99.2970 |
| P8 | 316.841 | 90.4057 |
| P9 | 22.3901 | 9.04580 |
| P10 | 4.67559 | 1.66451 |
| P11 | 369.992 | 132.695 |
| P12 | 150.003 | 68.3454 |
| P13 | 202.886 | 71.2716 |
| P14 | 14.2006 | 5.63649 |
| P15 | 2.91297 | 1.66451 |
| P16 | 42.8821 | 4.66601 |
| P17 | 53.7869 | 4.29935 |
| P18 | 2.79821 | 1.09847 |
| P19 | 0.528566 | 0.198347 |
| P20 | 44.3372 | 5.17700 |
| P21 | 51.0766 | 4.81893 |
| P22 | 3.78138 | 1.53050 |
| P23 | 0.797841 | 0.337331 |
| P24 | 39.5841 | 7.12802 |
| P25 | 55.3434 | 6.17960 |
| P26 | 4.19738 | 1.94699 |
| P27 | 0.870097 | 0.414340 |
| P28 | 0.982148 | 0.347569 |
| P29 | 1.89206 | 0.821214 |
| P30 | 1.74804 | 0.753290 |

(NOTE: THE P1 - P30 DESIGNATIONS CORRESPOND TO THE IDENTIFICATIONS IN THE TEXT OF THE DISCLOSURE)

| NORM | BEMI | BEST | BNOB | BTEST | BGENSIA | BVAS | BTNR | BMAQ | BEXE | SIZES |
|---|---|---|---|---|---|---|---|---|---|---|
| 100 | 100 | 100 | 100 | 100 | 100 | 100 | 100 | 100 | 100 | 0 |
| 53.7931 | 97.222 | 95.96 | 94.059 | 97.674 | 95.455 | 92 | 58.333 | 80.263 | 92.857 | 1 |
| 41.3793 | 95.37 | 91.919 | 93.069 | 94.186 | 90.909 | 84 | 36.111 | 67.105 | 71.429 | 2 |
| 28.2759 | 92.593 | 84.849 | 90.099 | 93.023 | 89.394 | 84 | 25 | 50 | 64.286 | 3 |
| 19.3103 | 91.667 | 83.838 | 84.158 | 90.698 | 89.394 | 84 | 25 | 43.421 | 64.286 | 4 |
| 11.7241 | 87.963 | 81.818 | 78.218 | 87.209 | 87.879 | 84 | 25 | 34.211 | 60.714 | 5 |
| 10.3448 | 84.259 | 76.768 | 71.287 | 84.884 | 86.364 | 80 | 22.222 | 28.947 | 60.714 | 6 |
| 8.27586 | 82.407 | 72.727 | 68.317 | 81.395 | 80.303 | 76 | 22.222 | 23.684 | 53.571 | 7 |
| 6.89655 | 78.704 | 69.697 | 59.406 | 79.07 | 78.788 | 72 | 8.333 | 19.737 | 46.429 | 8 |
| 6.2069 | 75.926 | 66.667 | 56.436 | 74.419 | 75.758 | 72 | 8.333 | 14.474 | 42.857 | 9 |
| 4.82759 | 72.222 | 64.647 | 50.495 | 69.767 | 71.212 | 64 | 2.778 | 11.842 | 42.857 | 10 |
| 3.44828 | 67.593 | 62.626 | 46.535 | 66.279 | 62.121 | 60 | 2.778 | 11.842 | 42.857 | 11 |
| 2.75862 | 64.815 | 58.586 | 45.545 | 62.791 | 59.091 | 52 | 2.778 | 10.526 | 42.857 | 12 |
| 2.06897 | 61.111 | 56.566 | 45.545 | 58.14 | 51.515 | 48 | 2.778 | 7.895 | 32.143 | 13 |
| 2.06897 | 59.259 | 51.515 | 42.574 | 56.977 | 46.97 | 36 | 2.778 | 7.895 | 28.571 | 14 |
| 1.37931 | 57.407 | 49.495 | 38.614 | 54.651 | 46.97 | 32 | 0 | 7.895 | 21.429 | 15 |
| 0.689655 | 51.852 | 45.455 | 35.644 | 50 | 40.909 | 32 | 0 | 7.895 | 17.857 | 16 |
| 0.689655 | 48.148 | 41.414 | 31.683 | 41.861 | 36.364 | 32 | 0 | 6.579 | 17.857 | 17 |
| 0 | 46.296 | 39.394 | 25.743 | 37.209 | 30.303 | 32 | 0 | 5.263 | 14.286 | 18 |
| 0 | 44.444 | 36.364 | 20.792 | 36.047 | 28.788 | 32 | 0 | 5.263 | 14.286 | 19 |
| 0 | 43.519 | 32.323 | 15.842 | 33.721 | 27.273 | 28 | 0 | 5.263 | 14.286 | 20 |
| 0 | 38.889 | 29.293 | 14.852 | 27.907 | 22.727 | 24 | 0 | 3.947 | 14.286 | 21 |
| 0 | 37.037 | 27.273 | 12.871 | 26.744 | 19.697 | 24 | 0 | 2.632 | 14.286 | 22 |
| 0 | 34.259 | 25.253 | 10.891 | 25.581 | 19.697 | 24 | 0 | 2.632 | 10.714 | 23 |
| 0 | 32.407 | 23.232 | 9.901 | 22.093 | 16.667 | 20 | 0 | 1.316 | 10.714 | 24 |
| 0 | 25 | 21.212 | 9.901 | 16.279 | 13.636 | 20 | 0 | 1.316 | 10.714 | 25 |
| 0 | 23.148 | 21.212 | 8.911 | 16.279 | 13.636 | 20 | 0 | 1.316 | 10.714 | 26 |
| 0 | 22.222 | 20.202 | 7.921 | 16.279 | 10.606 | 12 | 0 | 1.316 | 10.714 | 27 |
| 0 | 22.222 | 19.192 | 7.921 | 12.791 | 10.606 | 12 | 0 | 1.316 | 7.143 | 28 |
| 0 | 17.593 | 17.172 | 6.931 | 12.791 | 7.576 | 8 | 0 | 1.316 | 7.143 | 29 |
| 0 | 14.815 | 14.141 | 6.931 | 11.628 | 7.576 | 8 | 0 | 1.316 | 7.143 | 30 |
| 0 | 13.889 | 12.121 | 6.931 | 11.628 | 4.545 | 8 | 0 | 1.316 | 7.143 | 31 |
| 0 | 12.963 | 10.101 | 2.97 | 11.628 | 3.03 | 8 | 0 | 1.316 | 7.143 | 32 |
| 0 | 10.185 | 9.091 | 1.98 | 10.465 | 1.515 | 8 | 0 | 1.316 | 7.143 | 33 |
| 0 | 10.185 | 8.081 | 1.98 | 8.14 | 1.515 | 8 | 0 | 1.316 | 3.571 | 34 |
| 0 | 7.407 | 8.081 | 1.98 | 8.14 | 1.515 | 8 | 0 | 1.316 | 3.571 | 35 |
| 0 | 5.556 | 6.061 | 1.98 | 8.14 | 1.515 | 4 | 0 | 1.316 | 3.571 | 36 |
| 0 | 5.556 | 5.051 | 0.99 | 8.14 | 1.515 | 4 | 0 | 1.316 | 3.571 | 37 |
| 0 | 4.63 | 5.051 | 0.99 | 8.14 | 1.515 | 4 | 0 | 0 | 3.571 | 38 |
| 0 | 3.704 | 4.04 | 0 | 6.977 | 1.515 | 0 | 0 | 0 | 3.571 | 39 |
| 0 | 3.704 | 4.04 | 0 | 6.977 | 1.515 | 0 | 0 | 0 | 3.571 | 40 |

It is also disclosed that Tracking of a subject can be continuous, and can utilize data obtained before and after, for instance: a suitable stress test; intervention (angioplasty etc.); and/or medical therapy. Of interest is the fact that signal magnitude in Frequency Domain Plots, (eg. 7aX1-7aZ5), particulary in 60-150 and 150-250 HZ ranges has routinely been noted to drop by thirty (30%) percent or more upon subjecting patients who are prone to ischemia, to a cold-pressor test.

It is also to be understood that the terminology "Coronary Artery Disease" is used throughout this Disclosure, the present invention serves to identify Coronary Disfunction generally, which can include myocaridal poblems separate from Coronary Artery disease per se.

Finally, it is generally described herein that, for instance, as differences between Normal Subject Population, and Subject Representative Parameters increase, the "Score" of the present invention increases. It would be a simple matter indeed to place a negative sign on the "Score" and declare that it "decreases" when differences between Normal Subject Population and Subject Representative Parameters increase. It would further be a simple matter to utilize slightly different but substantially the same Normal Subject Population, and Subject Data Frequency Bands, or select slightly different but substantially the same Normal Subject Population, and Subject Data (ECG) cycle portions. As to attempt to draft definite Claim language to overcome all such possiblities would be an impossible task in view of the complexity of the present invention subject matter, it is therefore to be understood that the Doctrine of Equivalent applies to, and the Claims are to be interpreted to include all such contrived and substantially indifferent functional equivalents in the practice of the recited method of the present invention, emphasis added.

Having hereby disclosed the subject matter of the present invention, it should be obvious that many modifications, substitutions and variations of the present invention are possible in light of the teachings. It is therefore to be understood that the invention may be practiced other than as specifically described, and should be limited in breadth and scope only by the Claims.

## Claims

1. A noninvasive method of investigating cardiac status of a subject and enabling classification of a subject into normal and abnormal cardiac categories utilizing electrocardiography (ECG) data obtained therefrom, said method comprising, in a functional sequence, performance of the steps of:

   a. obtaining data from (ECG) cycle(s) from each of a multiplicity of members of a population of subjects who have been documented as normal subjects, in that they do not show risk factors for, or demonstrate detectable cardiac abnormality, by monitoring at least one lead(s) of an (ECG) system;

   b. selecting some (ECG) cycle portion, and calculating an average selected (ECG) cycle portion data set for at least one monitored (ECG) system lead(s) by, for a monitored (ECG) system lead, a procedure comprising combining corresponding (ECG) cycle portion data points for said selected (ECG) cycle portion for (ECG) cycle(s) obtained from each of a number of members of said multiplicity of members of a population of subjects who have been documented as normal subjects, each said calculated average selected (ECG) cycle portion data set being a composite data set of said selected (ECG) cycle portion for said population of normal subjects, for a monitored (ECG) system lead;

   c. obtaining data from (ECG) cycle(s) from a subject, by monitoring at least one lead(s) of an (ECG) system, said (ECG) system lead(s) monitored being the same as the monitored (ECG) system lead(s) utilized in step a. to obtain data utilized in step b.;

   d. selecting some (ECG) cycle portion, which is essentially that selected in step b., and calculating an average selected (ECG) cycle portion data set for at least one monitored (ECG) system lead(s) by, for a monitored (ECG) system lead, a procedure comprising combining corresponding (ECG) cycle portion data points for said selected (ECG) cycle portion for (ECG) cycle(s) obtained from said subject, each said calculated average selected (ECG) cycle portion data set being a composite data set of said selected (ECG) cycle portion for said subject, for a monitored (ECG) system lead;

   e. calculating corresponding representative parameter(s) from resulting composite data sets calculated in steps b. and d., for monitored (ECG) system lead(s), for, respectively, said normal subject population and said subject;

   f. comparing subject to corresponding normal subject population representative parameter(s), and combining results thereof to arrive at a "score", the magnitude of which "score" results from difference(s) between magnitude(s) of corresponding normal subject population, and subject representative parameter(s), which "score" magnitude increases when said difference(s) in magnitude(s) between corresponding normal subject population, and subject, representative parameter(s) increase, the magnitude of which "score" provides an indication of the cardiac status of said subject, with a "score" near zero being indicative of a subject properly categorized as a cardiac normal in that the magnitude(s) of subject representative parameter(s) are generally more closely matched to the magnitude(s) of corresponding normal subject population representative parameter(s), and with a progressively higher "score" being indicative of a subject progressively more properly categorized as a cardiac abnormal in that the magnitude(s) of subject representative parameter(s) are generally progressively less closely matched to the magnitude(s) of corresponding normal subject population representative parameter(s).

**2.** A noninvasive method of investigating cardiac status of a subject and enabling classification of a subject into normal and abnormal cardiac categories utilizing electrocardiography (ECG) data obtained therefrom as in Claim 1, which further comprises performance of the steps of calculating, and comparing ratio(s) of subject, to corresponding ratio(s) of normal subject population, representative parameters, and combining results thereof with those from comparing subject to corresponding normal subject population, representative parameter(s), in arriving at said "score", the magnitude of which "score" then further results from difference(s) between magnitude(s) of corresponding normal subject population and subject ratio(s) of representative parameters, which "score" magnitude increases when difference(s) in magnitude(s) between corresponding normal subject population, and subject, ratio(s) of representative parameters increase.

**3.** A noninvasive method of investigating cardiac status of a subject and enabling classification of a subject into normal and abnormal cardiac categories utilizing electrocardiography (ECG) data obtained therefrom as in Claim 2, which further comprises performance of the step of applying a confidence level acceptance test to results of comparing specific ratio(s) of subject, to corresponding specific ratio(s) of normal subject population representative parameters prior to combining results thereof to arrive at a "score", said confidence level test comprising:

a. the step of determining mean and standard deviation acceptance parameters for specific ratio(s) of normal subject population representative parameters based upon the (ECG) data from which said composite data set of said selected (ECG) cycle portion for said population of normal subjects was calculated;

b. the step of determining an acceptance parameter for each corresponding specific ratio of subject representative parameters based upon the (ECG) data from which said composite data set of said selected (ECG) cycle portion for said subject was calculated; and

c. the step of accepting the results of comparing a specific ratio of subject representative parameters, to a corresponding specific ratio of representative parameters for said normal subject population, in arriving at said "score", only if said acceptance parameter for said specific ratio of said subject representative parameters is set off by at least one associated normal subject population acceptance standard deviation from the acceptance mean of said corresponding specific ratio of normal subject population representative parameters.

**4.** A noninvasive method of investigating cardiac status of a subject and enabling classification of a subject into normal and abnormal cardiac categories utilizing electrocardiography (ECG) data obtained therefrom as in Claim 1, which further comprises performance of the step of applying a confidence level acceptance test to results of comparing specific subject, to corresponding specific normal subject population, representative parameter(s) prior to combining results thereof to arrive at a "score", said confidence level test comprising:

a. the step of determining mean and standard deviation acceptance parameters for specific normal subject population representative parameter(s) based upon the (ECG) data from which said composite data set of said selected (ECG) cycle portion for said population of normal subjects was calculated;

b. the step of determining an acceptance parameter for each corresponding specific subject representative parameter based upon the (ECG) data from which said composite data set of said selected (ECG) cycle portion for said subject was calculated; and

c. the step of accepting the results of comparing a specific subject representative parameter, to a corresponding specific normal subject population representative parameter, in arriving at said "score", only if the acceptance parameter for said specific subject representative parameter is set off by at least one associated normal subject population acceptance standard deviation from the acceptance mean of said corresponding specific normal subject population representative parameter.

**5.** A noninvasive method of investigating cardiac status of a subject and enabling classification of a subject into normal and abnormal cardiac categories utilizing electrocardiograpny (ECG) data obtained therefrom as in Claim 1, which further comprises performance of the steps of:

a. providing at least one coordinate system selected from the group consisting of magnitude vs. time, and magnitude vs. frequency;

b. for an (ECG) cycle portion, performing calculations necessary to plot and display and plotting and displaying normal subject population and subject (ECG) data as a function of at least one parameter selected from the group consisting of time and frequency, to respectively provide as desired, visually interpretable plots of (ECG) magnitude and power spectral density data, observation of which provides an indication of the cardiac status of said subject; and

c. observing and interpreting the displayed plot(s).

**6.** A noninvasive method of investigating cardiac status of a subject and enabling classification of a subject into normal and abnormal cardiac categories utilizing electrocardiography (ECG) data obtained therefrom as in Claim 1, in which the step of comparing subject, to normal subject population, representative parameter(s) and combining the results thereof to arrive at a "score", includes performance of the step of selecting parameters from the group consisting of root-mean-square mean and root-mean-square standard deviation as representative parameters.

**7.** A noninvasive method of investigating cardiac status of a subject and enabling classification of a subject into normal and abnormal cardiac categories utilizing electrocardiography (ECG) data obtained therefrom, said method comprising, in a functional sequence, performance of the steps of:

a. obtaining data from (ECG) cycle(s) from each of a multiplicity of members of a population of subjects who have been documented as normal subjects, in that they do not show risk factors for, or demonstrate detectable cardiac abnormality, by monitoring at least one lead(s) of an (ECG) system;

b. selecting some (ECG) cycle portion, and calculating an average selected (ECG) cycle portion data set for at least one monitored (ECG) system lead(s) by, for a monitored (ECG) system lead, a procedure comprising combining corresponding (ECG) cycle portion data points for said selected (ECG) cycle portion for (ECG) cycle(s) obtained from each of a number of members of said multiplicity of members of a population of subjects who have been documented as normal subjects, each said calculated average selected (ECG) cycle portion data set being a composite data set of said selected (ECG) cycle portion for said population of normal subjects, for a monitored (ECG) system lead;

c. obtaining data from (ECG) cycle(s) from a subject, by monitoring at least one lead(s) of an (ECG) system, said (ECG) system lead(s) monitored being the same as the monitored (ECG) system lead(s) utilized in step a. to obtain data utilized in step b.;

d. selecting some (ECG) cycle portion, which is essentially that selected in step b., and calculating an average selected (ECG) cycle portion data set for at least one monitored (ECG) system lead(s) by, for a monitored (ECG) system lead, a procedure comprising combining corresponding (ECG) cycle portion data points for said selected (ECG) cycle portion for (ECG) cycle(s) obtained from said subject, each said calculated average selected (ECG) cycle portion data set being a composite data set of said selected (ECG) cycle portion for said subject, for a monitored (ECG) system lead;

e. calculating corresponding representative parameter(s) and corresponding ratio(s) involving representative parameters from resulting composite data sets calculated in steps b. and d., for monitored (ECG) system lead(s), for, respectively, said normal subject population and said subject;

f. comparing specific ratio(s) of subject to corresponding specific ratio(s) of normal subject population representative parameters, and combining results thereof to arrive at a "score", the magnitude of which "score" results from difference(s) between magnitude(s) of specific corresponding ratio(s) of normal subject population, and ratio(s) of subject representative parameters, which "score" magnitude increases when said difference(s) in magnitude(s) between specific ratio(s) of corresponding normal subject population, and specific ratio(s) of subject representative parameters increase, the magnitude of which "score" provides an indication of the cardiac status of said subject, with a "score" near zero being indicative of a subject properly categorized as a cardiac normal in that the magnitude(s) of ratio(s) of subject representative parameters are generally more closely matched to the magnitude(s) of corresponding ratio(s) of normal subject population representative parameters, and with a progressively higher "score" being indicative of a subject progressively more properly categorized as a cardiac abnormal in that the magnitude(s) of ratio(s) of subject representative parameters are generally progressively less closely matched to the magnitude(s) of ratio(s) of corresponding normal subject population representative parameters.

**8.** A noninvasive method of investigating cardiac status of a subject and enabling classification of a subject into normal and abnormal cardiac categories utilizing electrocardiography (ECG) data obtained therefrom as in Claim 7, which further comprises performance of the step of applying a confidence level acceptance test to results of comparing ratio(s) of subject, to corresponding ratio(s) of normal subject population, representative parameters prior to combining results thereof to arrive at a "score", said confidence level test comprising:

a. the step of determining acceptance mean and standard deviation parameters for specific ratio(s) of normal subject population representative parameters based upon the (ECG) data from which said composite data set of said selected (ECG) cycle portion for said population of normal subjects was calculated;

b. the step of determining an acceptance parameter for each corresponding specific ratio of subject representative parameters based upon the (ECG) data from which said composite data net of said selected (ECG) cycle portion for said subject was calculated; and

c. the step of accepting the results of comparing a specific ratio of subject representative parameters, to a corresponding specific ratio of representative parameters for said normal subject population, in arriving at said "score", only if the acceptance parameter for said specific ratio of said subject representative parameters is set off by at least one associated normal subject population acceptance standard deviation from the acceptance mean of said corresponding specific ratio of normal subject population representative parameters.

**9.** A noninvasive method of investigating cardiac status of a subject and enabling classification of a subject into normal and abnormal cardiac categories utilizing electrocardiography (ECG) data obtained therefrom as in Claim 7, which further comprises performance of the steps of:

a. providing at least one coordinate system selected from the group consisting of magnitude vs. time, and magnitude vs. frequency;

b. for an (ECG) cycle portion, performing calculations necessary to plot and display and plotting and displaying normal subject population and subject (ECG) data, as a function of at least one parameter selected from the group consisting of time and frequency, to respectively provide as desired, visually interpretable plots of (ECG) magnitude and power spectral density data, observation of which provides an indication of the cardiac status of said subject; and

c. observing and interpreting displayed plot(s).

**10.** A noninvasive method of investigating cardiac status of a subject and enabling classification of a subject into normal and abnormal cardiac categories utilizing electrocardiography (ECG data obtained therefrom as in Claim 7, in which the step of comparing specific ratio(s) of subject, to corresponding specific ratio(s) of normal subject population, representative parameter(s) and combining the results thereof to arrive at a "score", which "score" provides an indication of the presence or absence of cardiac abnormality in said subject, includes performance of the step of selecting parameters from the group consisting of root-mean-square mean and root-mean-square standard deviation as representative parameters.

**11.** A noninvasive method of investigating cardiac status of a subject and enabling classification of a subject into normal and abnormal cardiac categories utilizing electrocardiography (ECG) data obtained therefrom, said method comprising, in a functional sequence, performance of the steps of:

a. obtaining data from (ECG) cycle(s) from each of a multiplicity of members of a population of subjects who have been documented as normal subjects, in that they do not show risk factors for, or demonstrate detectable cardiac abnormality, by monitoring at least one lead(s) of an (ECG) system;

b. selecting some (ECG) cycle portion and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for (ECG) cycle(s) obtained from each of a number of said multiplicity of members of a population of subjects who have been documented as normal subjects, and selecting a plurality of frequency bands and applying filtering techniques, to provide a plurality of data sets for each said at least one (ECG) system lead(s) monitored, each said data set being a composite data set of said selected (ECG) cycle portion for said population of normal subjects in a monitored lead and selected frequency band range;

c. obtaining data from (ECG) cycle(s) from a subject, by monitoring at least one lead(s) of an (ECG) system, said (ECG) system lead(s) monitored being the same as the monitored (ECG) system lead(s) utilized in step a. to obtain data utilized in step b.;

d. selecting some (ECG) cycle portion, said (ECG) cycle portion being essentially that selected in step b. for said normal subject population, and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for subject (ECG) cycle(s), and selecting a plurality of frequency bands, said selected frequency bands being essentially those selected in step b. for said normal subject population, and applying filtering techniques which are essentially those applied in step b. for said normal subject population, to provide a plurality of data sets for each said at least one monitored (ECG) system lead(s), each said data set being a composite data set of said selected (ECG) cycle portion for said subject in a monitored lead and selected frequency band range;

e. calculating corresponding representative parameter(s) from resulting composite data sets calculated in steps b. and d., in said selected frequency band ranges for monitored (ECG) system lead(s), for respectively, said normal subject population and said subject;

f. comparing specific subject to specific normal subject population corresponding representative parameter(s), and combining results thereof to arrive at a "score", the magnitude of which "score" results from difference(s) between magnitude(s) of corresponding normal subject population and subject representative parameter(s), which "score" magnitude increases when said difference(s) in magnitude(s) between corresponding normal subject population and subject representative parameter(s) increase, the magnitude of which "score" provides an indication of the cardiac status of said subject, with a "score" near zero being indicative of a subject properly categorized as a cardiac normal in that the magnitude(s) of subject representative parameter(s) are generally more closely matched to the magnitude(s) of corresponding normal subject population representative parameter(s), and with a progressively higher "score" being indicative of a subject progressively more properly categorized as a cardiac abnormal in that the magnitude(s) of subject representative parameter(s) are generally progressively less closely matched to the magnitude(s) of corresponding normal subject population representative parameter(s).

**12.** A noninvasive method of investigating cardiac status of a subject and enabling classification of a subject into normal and abnormal cardiac categories utilizing electrocardiography (ECG) data obtained therefrom as in Claim 11, which further comprises performance of the step of applying a confidence level acceptance test to results of comparing specific subject, to corresponding specific normal subject population, representative parameters prior to combining results thereof to arrive at a "score", said confidence level test comprising:

a. the step of determining mean and standard deviation acceptance parameters for specific normal subject population representative parameter(s) based upon the (ECG) data from which said composite data set of said selected (ECG) cycle portion for said population of normal subjects was calculated;

b. the step of determining an acceptance parameter for each corresponding specific subject representative parameter based upon the (ECG) data from which said composite data set of said selected (ECG) cycle portion for said subject was calculated; and

c. the step of accepting the results of comparing a specific subject representative parameter to a corresponding specific normal subject population representative parameter, in arriving at said "score", only if the acceptance parameter for said specific subject representative parameter is set off by at least one associated normal population acceptance standard deviation from the acceptance mean of said corresponding specific normal subject population representative parameter.

**13.** A noninvasive method of investigating cardiac status of a subject and enabling classification of a subject into normal and abnormal cardiac categories utilizing electrocardiography (ECG) data obtained therefrom as in Claim 11, which further comprises performance of the steps of:

a. providing at least one coordinate system selected from the group consisting of magnitude vs. time, and magnitude vs. frequency;

b. for an (ECG) cycle portion, performing calculations necessary to plot and display and plotting and displaying normal subject population and subject (ECG) data in at least one frequency band range, as a function of at least one parameter selected from the group consisting of time and frequency, to respectively provide as desired, visually interpretable plots of (ECG) magnitude and power spectral density data, observation of which provides an indication of the cardiac status of said subject; and

c. observing and interpreting displayed plot(s).

**14.** A noninvasive method of investigating cardiac status of a subject and enabling classification of a subject into normal and abnormal cardiac categories utilizing electrocardiography (ECG) data obtained therefrom as in Claim 11, in which the step of comparing corresponding subject, to normal subject population, representative parameter(s) and combining the results thereof to arrive at a "score", includes performance of the step of selecting parameters from the group consisting of root-mean-square mean and root-mean-square standard deviation.

**15.** A noninvasive method of investigating cardiac status of a subject and enabling classification of a subject into normal and abnormal cardiac categories utilizing electrocardiography (ECG) data obtained therefrom, said method comprising, in a functional sequence, performance of the steps of:

a. obtaining data from (ECG) cycle(s) from each of a multiplicity of members of a population of subjects who have been documented as normal subjects, in that they do not show risk factors for, or demonstrate detectable cardiac abnormality, by monitoring at least one lead(s) of an (ECG) system;

b. selecting some (ECG) cycle portion and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for (ECG) cycle(s) obtained from each of a number of said multiplicity of members of a population of subjects who have been documented as normal subjects, and selecting a plurality of frequency bands and applying filtering techniques, to provide a plurality of data sets for said at least one (ECG) system lead(s) monitored, each said data set being a composite data set of said selected (ECG) cycle portion for said population of normal subjects in a monitored lead and selected frequency band range;

c. obtaining data from (ECG) cycle(s) from a subject, by monitoring at least one lead(s) of an (ECG) system, said (ECG) system lead(s) monitored being the same as the monitored (ECG) system lead(s) utilized in step a. to obtain data utilized in step b.;

d. selecting some (ECG) cycle portion, said (ECG) cycle portion being essentially that selected in step b. for said normal subject population, and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for subject (ECG) cycle(s), and selecting a plurality of frequency bands, said selected frequency bands being essentially those selected in step b. for said normal subject population, and applying filtering techniques which are essentially those applied in step b. for said normal subject population, to provide a plurality of data sets for said at least one monitored (ECG) system lead(s), each said data set being a composite data set of said selected (ECG) cycle portion for said subject in a monitored lead and selected frequency band range;

e. calculating corresponding representative parameter(s) and corresponding ratio(s) involving representative parameters from resulting composite data sets calculated in step b. and d., in said selected frequency band ranges for monitored (ECG) system lead(s), for respectively, said normal subject population and said subject;

f. comparing specific ratio(s) of subject to corresponding specific ratio(s) of normal subject population representative parameters, and combining results thereof to arrive at a "score", the magnitude of which "score" results from difference(s) between magnitude(s) of corresponding specific ratio(s) of normal subject population and specific ratio(s) of subject representative parameters, which "score" magnitude increases when said difference(s) in magnitude(s) between specific ratio(s) of corresponding normal subject population and subject representative parameters increase, the magnitude of which "score" provides an indication of the cardiac status of said subject, with a "score" near zero being indicative of a subject properly categorized as a cardiac normal in that the magnitude(s) of ratio(s) of subject representative parameters are generally more closely matched to the magnitude(s) of corresponding ratio(s) of normal subject population representative parameters, and with a progressively higher "score" being indicative of a subject progressively more properly categorized as a cardiac abnormal in that the magnitude(s) of ratio(s) of subject representative parameters are generally progressively less closely matched to the magnitude(s) of ratio(s) of corresponding normal subject population representative parameters.

**16.** A noninvasive method of investigating cardiac status of a subject and enabling classification of a subject into normal and abnormal cardiac categories utilizing electrocardiography (ECG) data obtained therefrom as in Claim 15, which further comprises performance of the step of applying a confidence level acceptance test to results of comparing specific ratio(s) of subject, to corresponding specific ratio(s) of normal subject population, representative parameters prior to combining results thereof to arrive at a "score", said confidence level test comprising:

a. the step of determining mean and standard deviation acceptance parameters for specific ratio(s) of normal subject population representative parameters based upon the (ECG) data from which said composite data set of said selected (ECG) cycle portion for said population of normal subjects was calculated;

b. the step of determining an acceptance parameter for each corresponding specific ratio of subject representative parameters based upon the (ECG) data from which said composite data set of said selected (ECG) cycle portion for said subject was calculated; and

c. the step of accepting the results of comparing a specific ratio of subject representative parameters, to a corresponding specific ratio of representative parameters for said normal subject population, in arriving at said "score", only if the acceptance parameter for said specific ratio of said subject representative parameters is set off by at least one associated normal subject population acceptance standard deviation from the acceptance mean of said specific ratio of normal subject population representative parameters.

**17.** A noninvasive method of investigating cardiac status of a subject and enabling classification of a subject into normal and abnormal cardiac categories utilizing electrocardiography (ECG) data obtained therefrom as in Claim 15, which further comprises performance of the steps of:

a. providing at least one coordinate system selected from the group consisting of magnitude vs. time, and magnitude vs. frequency;

b. for an (ECG) cycle portion, performing calculations necessary to plot and display and plotting and displaying normal subject population and subject (ECG) data for at least one frequency band range, as a function of at least one parameter selected from the group consisting of time and frequency, to respectively provide as desired, visually interpretable plots of (ECG) magnitude and power spectral density data, observation of which provides an indication of the cardiac status of said subject; and

c observing and interpreting displayed plot(s).

**18.** A noninvasive method of investigating cardiac status of a subject and enabling classification of a subject into normal and abnormal cardiac categories utilizing electrocardiography (ECG) data obtained therefrom as in Claim 15, in which the step of comparing ratio(s) of subject, to corresponding ratio(s) of normal subject population, representative parameters and combining the results thereof to arrive at a "score", includes performance of the step of selecting ratios of parameter(s) from the group consisting of a ratio of a root-mean-square mean to at least one other root-mean-square mean, and a ratio of at least one root-mean-square mean to a root-mean-square standard deviation.

**19.** A noninvasive method of investigating cardiac status of a subject and enabling classification of a subject into normal and abnormal cardiac categories utilizing electrocardiography (ECG) data obtained therefrom, said method comprising, in a functional sequence, performance of the steps of:

a. obtaining data from (ECG) cycle(s) from each of a multiplicity of members of a population of subjects who have been documented as normal subjects, in that they do not show risk factors for, or demonstrate detectable cardiac abnormality, by monitoring at least one lead(s) of an (ECG) system;

b. selecting some (ECG) cycle portion and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for (ECG) cycle(s) obtained from each of a number of said multiplicity of members of a population of subjects who have been documented as normal subjects, and selecting a plurality of frequency bands and applying filtering techniques, to provide a plurality of data sets for said at least one (ECG) system lead(s) monitored, each said data set being a composite data set of said selected (ECG) cycle portion for said population of normal subjects in a monitored lead and selected frequency band range;

c. obtaining data from (ECG) cycle(s) from a subject, by monitoring at least one lead(s) of an (ECG) system, said (ECG) system lead(s) monitored being the same as the monitored (ECG) system lead(s) utilized in step a. to obtain data utilized in step b.;

d. selecting some (ECG) cycle portion, said (ECG) cycle portion being essentially that selected in step b. for said normal subject population, and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for subject (ECG) cycle(s), and selecting a plurality of frequency bands, said selected frequency bands being essentially those selected in step b. for said normal subject population, and applying filtering techniques which are essentially those applied in step b. for said normal subject population, to provide a plurality of data sets for said at least one monitored (ECG) system lead(s), each said data set being a composite data set of said selected (ECG) cycle portion for said subject in a monitored lead and selected frequency band range;

e. calculating corresponding representative parameter(s) and corresponding ratio(s) involving representative parameters from resulting composite data sets calculated in steps b. and d., in said selected frequency band ranges for monitored (ECG) system lead(s), for respectively, said normal subject population and said subject;

f. comparing specific subject and corresponding specific normal subject population representative parameter(s), and combining results thereof with the results of comparing specific ratio(s) of subject to corresponding specific ratio(s) of normal subject population representative parameters, to arrive at a "score", the magnitude of which "score" results from difference(s) in magnitude(s) between corresponding subject and normal subject population representative parameter(s) and difference(s) between magnitude(s) of corresponding ratio(s) of normal subject population, and ratio(s) of subject representative parameters, which "score" magnitude increases when difference(s) in magnitude(s) between corresponding subject and normal subject population representative parameter(s) increase and difference(s) in magnitude(s) between ratio(s) of corresponding normal subject population, and ratio(s) of subject representative parameters increase, the magnitude of which "score" provides an indication of the cardiac status of said subject, with a "score" near zero being indicative of a subject properly categorized as a cardiac normal in that magnitude(s) of subject representative parameter(s) are generally more closely matched to the magnitude(s) of corresponding normal subject population representative parameter(s) and magnitude(s) of ratio(s) of subject representative parameters are generally more closely matched to the magnitude(s) of corresponding ratio(s) of normal subject population representative parameters, and with a progressively higher "score" being indicative of a subject progressively more properly categorized as a cardiac abnormal in that magnitude(s) of subject representative parameter(s) are generally progressively less closely matched to the magnitude(s) of corresponding normal subject population representative parameter(s) and magnitude(s) of ratio(s) of subject representative parameter(s) are generally progressively less closely matched to the magnitude(s) of ratio(s) of corresponding normal subject population representative parameters.

**20.** A noninvasive method of investigating cardiac status of a subject and enabling classification of a subject into normal and abnormal cardiac categories utilizing electrocardiography (ECG) data obtained therefrom as in Claim 19, which further comprises performance of the step of applying a confidence level acceptance test to results of comparing subject, to corresponding normal subject population representative parameter(s), and the results of comparing ratios of subject representative parameters to corresponding ratios of normal subject population representative parameters, prior to combining results thereof to arrive at a "score", said confidence level test comprising:

a. the step of determining mean and standard deviation acceptance parameters for specific normal subject population representative parameter(s) and specific ratio(s) of normal subject population representative parameters based upon the (ECG) data from which said composite data set of said selected (ECG) cycle portion for said population of normal subjects was calculated;

b. the step of determining an acceptance parameter for each corresponding specific subject representative parameter and each corresponding specific ratio of subject representative parameters based upon the (ECG) data from which said composite data set of said selected (ECG) cycle portion for said subject was calculated; and

c. the step of accepting the results of comparing a specific subject representative parameter to a corresponding specific normal subject population representative parameter in arriving at said "score", only if the acceptance parameter for said specific subject representative parameter is set off by at least one associated normal subject population acceptance standard deviation from the acceptance mean of the corresponding specific normal subject population representative parameter; and accepting the results of comparing a specific ratio of subject representative parameters to a corresponding specific ratio of representative parameters for said normal subject population, in arriving at said "score", only if the acceptance parameter of said specific ratio of said subject representative parameters is set off by at least one associated normal subject population acceptance standard deviation from the acceptance mean of said corresponding specific ratio of normal subject population representative parameters.

**21.** A noninvasive method of investigating cardiac status of a subject and enabling classification of a subject into normal and abnormal cardiac categories utilizing electrocardiography (ECG) data obtained therefrom as in Claim 19, which further comprises performance of the steps of:

a. providing at least one coordinate system selected from the group consisting of magnitude vs. time, and magnitude vs. frequency;

b. for an (ECG) cycle portion, performing calculations necessary to plot and display and plotting and displaying normal subject population and subject (ECG) data for at least one frequency band range, as a function of at least one parameter selected from the group consisting of time and frequency, to respectively provide as desired, visually interpretable plots of (ECG) magnitude and power spectral density data, observation of which provides an indication of the cardiac status of said subject; and

c. observing and interpreting displayed plot(s).

**22.** A noninvasive method of investigating cardiac status of a subject and enabling classification of a subject into normal and abnormal cardiac categories utilizing electrocardiography (ECG) data obtained therefrom as in Claim 19, in which the step of comparing corresponding subject, to normal subject population, representative parameter(s) and ratio(s) thereof, and combining the results thereof to arrive at a "score", which "score" provides an indication of the presence or absence of cardiac abnormality in said subject, includes performance of the step of selecting representative parameter(s) and ratio(s) thereof formed between representative parameters from the group consisting of root-mean-square mean and root-mean-square standard deviation.

**23.** A noninvasive method of investigating cardiac status of a subject and enabling classification of a subject into normal and abnormal cardiac categories utilizing electrocardiography (ECG) data obtained therefrom, said method comprising, in a functional sequence, performance of the steps of:

a. obtaining (ECG) data from (ECG) cycle(s) at each lead of a three lead Frank X-Y-Z (ECG) system, from each of a multiplicity of subjects who have been documented as normal subjects, in that they do not show risk factors for, or demonstrate detectable cardiac abnormality;

b. selecting the QRS portion of said obtained (ECG) cycle(s) obtained from each Frank X-Y-Z (ECG) system lead and calculating an average selected QRS cycle portion data set for each monitored Frank (X-Y-Z) (ECG) system lead, by, for each Frank (X-Y-Z) (ECG) system lead, a procedure comprising combining corresponding QRS cycle portion data points for said selected QRS cycle portion for (ECG) cycle(s) obtained from each of a number of members of said multiplicity of members of a population of subjects who have been documented as normal subjects, and selecting a plurality of frequency bands and applying filtering techniques, to provide a plurality of data sets for each Frank X-Y-Z (ECG) system lead, each said data set being a composite QRS data set for said population of normal subjects, for a Frank (X-Y-Z) (ECG) system lead, in a selected frequency band range;

c. obtaining (ECG) data from (ECG) cycle(s) from each Frank X-Y-Z (ECG) system lead for a subject;

d. selecting the QRS complex of said obtained (ECG) cycle(s) obtained at each Frank X-Y-Z (ECG) system lead, said (ECG) QRS cycle portion being the same as that selected in step b. for said normal subject population, and calculating an average selected QRS cycle portion data set for each monitored Frank (X-Y-Z) (ECG) system lead, by, for each Frank (X-Y-Z) (ECG) system lead, a procedure comprising combining corresponding QRS cycle portion data points for said selected QRS cycle portion for subject (ECG) cycle(s), and selecting a plurality of frequency bands, said frequency bands being the same as those selected in step b. for said normal subject population and applying filtering techniques which are the same as those applied in step b. for said normal subject population, to provide a plurality of data sets for each Frank X-Y-Z (ECG) system lead, each said data set being a composite QRS data set for said subject, for a Frank (X-Y-Z) (ECG) system lead, in a selected frequency band range;

e. calculating corresponding root-mean-square mean and root-mean-square standard deviation parameter(s) as well as corresponding ratio(s) involving root-mean-square and root-mean-square standard deviation parameters from resulting composite QRS data sets calculated in step b. and d., in each selected frequency band range for each Frank X-Y-Z (ECG) system lead, for, respectively, said normal subject population and subject;

f. comparing specific subject and corresponding specific normal subject population root-mean-square parameter(s), and combining results thereof with the results of comparing specific ratio(s) of subject to corresponding specific ratio(s) of normal subject population root-mean-square parameters, to arrive at a "score", the magnitude of which "score" results from difference(s) in magnitude(s) between corresponding subject and normal subject population root-mean-square parameter(s) and difference(s) between magnitude(s) of corresponding ratio(s) of normal subject population, and ratio(s) of subject root-mean-square parameters, which "score" magnitude increases when difference(s) in magnitude(s) between corresponding subject and normal subject population root-mean-square parameter(s) increase and difference(s) in magnitude(s) between ratio(s) of corresponding normal subject population, and ratio(s) of subject root-mean-square parameters increase, the magnitude of which "score" provides an indication of the cardiac status of said subject, with a "score" near zero being indicative of a subject properly categorized as a cardiac normal in that magnitude(s) of subject root-mean-square parameter(s) are generally more closely matched to the magnitude(s) of corresponding normal subject population root-mean-square parameter(s) and magnitude(s) of ratio(s) of subject root-mean-square parameters are generally more closely matched to the magnitude(s) of corresponding ratio(s) of normal subject population root-mean-square parameters, and with a progressively higher "score" being indicative of a subject progressively more properly categorized as a cardiac abnormal in that magnitude(s) of subject root-mean-square parameter(s) are generally progressively less closely matched to the magnitude(s) of corresponding normal subject population root-mean-square parameter(s) and magnitude(s) of ratio(s) of subject root-mean-square parameter(s) are generally progressively less closely matched to the magnitude(s) of ratio(s) of corresponding normal subject population root-mean-square parameters.

**24.** A noninvasive method of investigating cardiac status of a subject and enabling classification of a subject into normal and abnormal cardiac categories utilizing electrocardiography (ECG) data obtained therefrom as in Claim 23, which further comprises performance of the step of applying a confidence level acceptance test to results of comparing corresponding specific normal subject population to specific subject root-mean-square parameters, and corresponding specific ratios of said root-mean-square parameters of subject, to specific ratios of normal subject population, root-mean-square parameters prior to combining results thereof to arrive at a "score", said confidence level test comprising:

a. the step of determining mean and standard deviation acceptance parameters for specific normal subject population root-mean-square parameter(s) and specific ratio(s) of normal subject population root-mean-square parameter(s) based upon the (ECG) data from which said composite data set of said selected (ECG) cycle portion for said population of normal subjects was calculated;

b. the step of determining an acceptance parameter for each specific corresponding subject root-mean-square parameter and each corresponding specific ratio of subject root-mean-square parameters based upon the (ECG) data from which said composite data set of said selected (ECG) cycle portion for said subject was calculated; and

c. the step of accepting the results of comparing a specific subject root-mean-square parameter to a corresponding specific normal subject population root-mean-square parameter in arriving at said "score", only if the acceptance parameter for said specific subject root-mean-square parameter is set off by at least one associated normal subject population acceptance standard deviation from the acceptance mean of the corresponding specific normal subject population root-mean-square parameter; and accepting the results of comparing a specific ratio of subject root-mean-square parameters to a corresponding specific ratio of root-mean-square parameters for said normal subject population, in arriving at said "score", only if the acceptance parameter of said specific ratio of said subject root-mean-square parameters is set off by at least one associated normal population acceptance standard deviation from the acceptance mean of said corresponding specific ratio of normal subject population root-mean-square parameters.

**25.** A noninvasive method of investigating cardiac status of a subject and enabling classification of a subject into normal and abnormal cardiac categories utilizing electrocardiography (ECG) data obtained therefrom as in Claim 24 in which the step applying a confidence level test for accepting differences between corresponding normal subject population to subject root-mean-square parameters, and corresponding ratios of said root-mean-square parameters, for each Frank X-Y-Z (ECG) system lead and using said accepted differences to arrive at a "score", includes performing the step of determining if an acceptance parameter of the subject falls outside a region of distribution of corresponding normal subject population data, centered around an acceptance mean for said corresponding normal subject population, which region of distribution of normal subject population data includes ninety-five (95%) percent of said corresponding normal subject population distribution data.

**26.** A noninvasive method of investigating cardiac status of a subject and enabling classification of a subject into normal and abnormal cardiac categories utilizing electrocardiography (ECG) data obtained therefrom as in Claim 23, which includes in performance of the step of applying filtering techniques in steps b. and d. thereof, the specific formation of fifteen (15) data sets for each of the normal subject population and the subject, said data sets being five (5) per each Frank X-Y-Z (ECG) system lead, said five (5) data sets per each Frank X-Y-Z (ECG) system lead covering:

a. all frequencies present;

b. the frequency range of zero (0) to ten (10) Hertz;

c. the frequency range of ten (10) to sixty (60) Hertz;

d. the frequency range of sixty (60) to one-hundred-fifty (150) Hertz; and

e. the frequency range of one-hundred-fifty (150) to two-hundred-fifty (250) Hertz.

**27.** A noninvasive method of investigating cardiac status of a subject and enabling classification of a subject into normal and abnormal cardiac categories utilizing electrocardiography (ECG) data obtained therefrom as in Claim 23, which further comprises performance of the steps of:

a. providing at least one coordinate system selected from the group consisting of magnitude vs. time, and magnitude vs. frequency;

b. for an (ECG) cycle portion, performing calculations necessary to plot and display and plotting and displaying normal subject population and subject (ECG) data for at least one frequency band range, as a function of at least one parameter selected from the group consisting of time and frequency, to respectively provide as desired, visually interpretable plots of (ECG) magnitude and power spectral density data, observation of which provides an indication of the cardiac status of said subject; and

c. observing and interpreting displayed plot(s).

**28.** A noninvasive method of investigating cardiac status of a subject and enabling classification of a subject into normal and abnormal cardiac categories utilizing electrocardiography (ECG) data obtained therefrom as in Claim 23, in which corresponding ratios of said root-mean-square parameters are compared, said method including performance of the steps of:

a. selecting said ratios to be calculated as, for each (ECG) lead and frequency band range utilized, a root-mean-square mean for said frequency band range divided by a sum of root-mean-square means of all frequency band ranges present; said ratio for a normal subject population being subtracted from a corresponding ratio for said subject, each said subtracted difference being divided by a root-mean-square standard deviation for said normal subject population in a corresponding frequency band range; and

b. performing the calculations described in step a.

**29.** A noninvasive method of investigating cardiac status of a subject and enabling classification of a subject into normal and abnormal cardiac categories utilizing electrocardiography (ECG) data obtained therefrom as in Claim 23, in which corresponding ratios of said root-mean-square parameters are compared, includes performance of the steps of:

a. selecting said ratios to be calculated as, for each (ECG) lead and frequency band range utilized, a root-mean-square mean for one (ECG) lead divided by a root-mean-square mean for another (ECG) lead; said ratio for a normal subject population being subtracted from a corresponding ratio for said subject, each said subtracted difference being divided by a root-mean-square standard deviation for said normal subject population ratio in a corresponding frequency band range; and

b. performing the calculations described in step a.

**30.** A noninvasive method of tracking cardiac status of a subject utilizing electrocardiography (ECG) data obtained therefrom, said method comprising, in a functional sequence, performance of the steps of:

a. obtaining initial data from (ECG) cycles from a subject;

b. selecting some (ECG) cycle portion and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for (ECG) cycle(s) obtained from said subject, and selecting a plurality of frequency bands and applying filtering techniques, to provide a plurality of data sets for said at least one (ECG) system lead(s) monitored, each said data set being an initial composite data set of said selected (ECG) cycle portion for said subjects in a monitored lead and selected frequency band range;

c. obtaining follow-on data from (ECG) cycle(s) from a subject at a later time, by monitoring at least one lead(s) of an (ECG) system, said (ECG) system lead(s) monitored being the same as the monitored (ECG) system lead(s) utilized in step a. to obtain data utilized in step b.;

d. selecting some (ECG) cycle portion, said (ECG) cycle portion being essentially that selected in step b. for said initial subject data, and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for subject (ECG) cycle(s), and selecting a plurality of frequency bands, said selected frequency bands being essentially those selected in step b. for said initial subject data, and applying filtering techniques which are essentially those applied in step b. for said initial subject data, to provide a plurality of data sets for said at least one monitored (ECG) system lead(s), each said data set being a follow-on composite data set of said selected (ECG) cycle portion for said subject in a monitored lead and selected frequency band range;

e. calculating corresponding representative parameter(s) from resulting composite data sets calculated in steps b. and d., in said selected frequency band ranges for monitored (ECG) system lead(s), for respectively, said initial subject data and said follow-on subject data;

f. comparing at least one member of the group consisting of: (initial subject to corresponding follow-on subject representative parameter(s), and calculated specific ratio(s) of initial subject to corresponding specific ratio(s) of follow-on subject representative parameters), and combining results thereof to arrive at a "score"; the magnitude of which "score" results from difference(s) between magnitude(s) of corresponding initial subject, and follow-on subject representative parameter(s) and/or ratio(s) of initial subject representative parameters, and follow-on subject representative parameters; which "score" magnitude increases when said difference(s) in magnitude(s) between corresponding initial subject, and follow-on subject, representative parameter(s) and/or ratio(s) of initial subject representative parameters, and follow-on subject representative parameters increase, the magnitude of which "score" provides an indication of a change in cardiac status of said subject, with a "score" near zero being indicative of a subject properly categorized as having undergone no cardiac change, and with a progressively higher "score" being indicative of a subject progressively more properly categorized as a subject who has undergone cardiac change.

**31.** A noninvasive method of tracking the cardiac status of a subject utilizing electrocardiography (ECG) data obtained therefrom as in Claim 30, which further comprises performance of the step of applying a confidence level acceptance test to results of comparing corresponding specific initial subject to specific follow-on subject representative parameters, and corresponding specific ratios of said representative parameters of follow-on subject, to specific ratios of initial normal subject, representative parameters prior to combining results thereof to arrive at a "score", said confidence level test comprising:

a. the step of determining mean and standard deviation acceptance parameters for specific initial subject representative parameter(s) and specific ratio(s) of initial subject representative parameter(s) based upon the (ECG) data from which said composite data set of said selected (ECG) cycle portion for said initial subject datawas calculated;

b. the step of determining an acceptance parameter for each specific corresponding follow-on subject representative parameter and each corresponding specific ratio of follow-on subject rrepresentative parameters based upon the (ECG) data from which said composite data set of said selected (ECG) cycle portion for said follow-on subject data was calculated; and

c. the step of accepting the results of comparing a specific follow-on subject representative parameter to a corresponding specific initial subject representative parameter in arriving at said "score", only if the acceptance parameter for said specific follow-on subject representative parameter is set off by at least one associated initial subject acceptance standard deviation from the acceptance mean of the corresponding specific initial subject representative parameter; and accepting the results of comparing a specific ratio of follow-on subject representative parameters to a corresponding specific ratio of representative parameters for said initial subject population, in arriving at said "score", only if the acceptance parameter of said specific ratio of said follow-on subject representative parameters is set off by at least one associated initial subject acceptance standard deviation from the acceptance mean of said corresponding specific ratio of initial subject representative parameters.

**32.** A noninvasive method of investigating cardiac status of a subject and enabling classification of a subject into normal and abnormal cardiac categories utilizing electrocardiography (ECG) data obtained therefrom, said method comprising, in a functional sequence, performance of the steps of:

a. obtaining data from (ECG) cycle(s) from each of a multiplicity of members of a population of subjects who have been documented as normal subjects, in that they do not show risk factors for, or demonstrate detectable cardiac abnormality, by monitoring at least one lead(s) of an (ECG) system;

b. selecting some (ECG) cycle portion and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for (ECG) cycle(s) obtained from each of a number of said multiplicity of members of a population of subjects who have been documented as normal subjects, and selecting a plurality of frequency bands and applying filtering techniques, to provide a plurality of data sets for said at least one (ECG) system lead(s) monitored, each said data set being a composite data set of said selected (ECG) cycle portion for said population of normal subjects in a monitored lead and selected frequency band range;

c. obtaining data from (ECG) cycle(s) from a subject, by monitoring at least one lead(s) of an (ECG) system, said (ECG) system lead(s) monitored being the same as the monitored (ECG) system lead(s) utilized in step a. to obtain data utilized in step b.;

d. selecting some (ECG) cycle portion, said (ECG) cycle portion being essentially that selected in step b. for said normal subject population, and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for subject (ECG) cycle(s), and selecting a plurality of frequency bands, said selected frequency bands being essentially those selected in step b. for said normal subject population, and applying filtering techniques which are essentially those applied in step b. for said normal subject population, to provide a plurality of data sets for said at least one monitored (ECG) system lead(s), each said data set being a composite data set of said selected (ECG) cycle portion for said subject in a monitored lead and selected frequency band range;

e. performing at least one of the following steps f. and g.;

f. calculating mean and standard deviation representative parameters from at least one resulting composite data set calculated in step b., said mean and standard deviation parameters being from a selected frequency band range for a monitored (ECG) system lead, and providing a coordinate system consisting of magnitude vs. time on ordinate and abscissa respectively and plotting and displaying on said coordinate system loci consisting of:

1. normal subject population standard deviation bounds located above and below said normal subject population mean, and

2. a corresponding subject data set,

then observing differences between normal subject population and subject data plots, with a subject data set falling within the normal subject standard deviation bounds being indicative of a subject properly classified as a cardiac normal, and with a subject data set falling progressively further outside said normal subject standard deviation bounds being indicative of a subject progressively more properly classified as a cardiac abnormal;

g. calculating power spectral density data for at least one resulting composite data set calculated in step b. and for a corresponding resulting composite data set calculated in step d. for a selected frequency band range for a monitored (ECG) system lead, and providing a coordinate system consisting of magnitude vs. frequency on ordinate and abscissa respectively and plotting and displaying on said coordinate system power spectral density data loci, then observing differences between normal subject population and subject data loci, with closely matched corresponding subject and normal subject population data set power spectral density loci being indicative of a subject properly classified as a cardiac normal and with progressively more mismatched subject and normal subject population data set power spectral density loci being indicative of a subject progressively more properly classified as a cardiac abnormal.

**33.** A noninvasive method of tracking cardiac status change in a subject utilizing electrocardiography (ECG) data obtained therefrom, said method comprising, in a functional sequence, performance of the steps of:

a. obtaining initial data from (ECG) cycles from a subject;

b. selecting some (ECG) cycle portion and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for (ECG) cycle(s) obtained from said subject, and selecting a plurality of frequency bands and applying filtering techniques, to provide a plurality of data sets for said at least one (ECG) system lead(s) monitored, each said data set being an initial composite data set of said selected (ECG) cycle portion for said subjects in a monitored lead and selected frequency band range;

c. obtaining follow-on data from (ECG) cycle(s) from a subject at a later time, by monitoring at least one lead(s) of an (ECG) system, said (ECG) system lead(s) monitored being the same as the monitored (ECG) system lead(s) utilized in step a. to obtain data utilized in step b.;

d. selecting some (ECG) cycle portion, said (ECG) cycle portion being essentially that selected in step b. for said initial subject data, and calculating an average selected (ECG) cycle portion data set for at least one monitored (ECG) system lead(s), by, for a monitored (ECG) system lead, a procedure comprising combining corresponding (ECG) cycle portion data points for said selected (ECG) cycle portion for subject (ECG) cycle(s), and selecting a plurality of frequency bands, said selected frequency bands being essentially those selected in step b. for said initial subject data, and applying filtering techniques which are essentially those applied in step b. for said initial subject data, to provide a plurality of data sets for said at least one monitored (ECG) system lead(s), each said data set being a follow-on composite data set of said selected (ECG) cycle portion for said subject in a monitored lead and selected frequency bind range;

e. performing at least one of the following steps f. and g.;

f. calculating mean and standard deviation representative parameters from at least one resulting composite data set calculated in step b., said mean and standard deviation parameters being from a selected frequency band range for a monitored (ECG) system lead, and providing a coordinate system consisting of magnitude vs. time on ordinate and abscissa respectively and plotting and displaying on said coordinate system loci consisting of:

1. initial subject standard deviation bounds located above and below said initial subject data set mean, and

2. a corresponding follow-on subject data set,

then observing differences between initial subject and follow-on subject data plots, with a follow-on subject data set falling within the initial subject data set standard deviation bounds being indicative of a subject properly classified as having not undergone cardiac change, and with a follow-on subject data set falling progressively further outside said initial subject data set standard deviation bounds being indicative of a subject progressively more properly classified as having undergone cardiac change;

g. calculating power spectral density data for at least one resulting composite data set calculated in step b. and for a corresponding resulting composite data set calculated in step d. for a selected frequency band range for a monitored (ECG) system lead, and providing a coordinate system consisting of magnitude vs. frequency on ordinate and abscissa respectively and plotting and displaying on said coordinate system power spectral density data loci, then observing differences between initial subject and follow-on subject data loci, with closely matched corresponding initial subject and follow-on subject data set power spectral density loci being indicative of a subject who has not undergone cardiac change and with progressively more mismatched initial subject and follow-on subject data set power spectral density loci being indicative of a subject progressively more properly classified having undergone cardiac change.

**34.** A noninvasive method of performing a procedure selected from the group consisting of: (investigating and tracking), cardiac status of a subject as in Claims 1 - 33 & 38 which further comprises the step of dividing the "score" for a subject by the subject's cardiac ejection fraction, and if the result of said division is greater than one (1.0), considering said subject as at high risk for sudden death.

**35.** A noninvasive method of performing a procedure selected from the group consisting of: (investigating and tracking), cardiac status of a subject and enabling classification of a subject into normal and abnormal cardiac categories utilizing electrocardiography (ECG) data obtained therefrom as in Claims 5, 9, 13, 17, 21, 27, 32 and 33, which method further comprises the steps selecting the portion of the (ECG) cycle to include at least a portion of the S-T segement following the QRS complex, and of identifying the presence of:

"Rhomboids"

following said QRS complex, and if present, considering said subject as at high risk for sudden death.

**36.** A noninvasive method of performing a procedure selected from the group consisting of: (investigating and tracking), cardiac status of a subject and enabling classification of a subject into normal and abnormal cardiac categories utilizing electrocardiography (ECG) data obtained therefrom as in Claims 1-35 & 38 further comprising determination of pattern(s) amongst at least one selection from the group consisting of: (subject represenatative parameter values and ratios of subject representative parameter values and a "score"/ejection fraction), and utilizing said pattern(s) as a basis for diagnosing specific cardiac abnormality.

**37.** A system utilized in practicing the method recited in Claims 1 through 36 & 38, said system comprising electrodes ((RA) (LA) (LL) (X) (Y) (Z) (v1) (v2) (v3) (v4) (v5) (v6)) for accessing (ECG) signals from the body of a subject, an (ECG) monitor, a computing means and at least one selection from the group consisting of: (a PRINTER /PLOTTER and a VISUAL DISPLAY).

**38.** A selection from the group consisting of: (a method and a system for practicing said method), said method being a selection from the group consisting of: (a noninvasive method of tracking, and of investigating cardiac status of a subject), said method comprising, in a functional sequence, performance of the steps of:

a. providing a representative (ECG) cycle for a selection from the group consisting of: (a subject baseline and a presumed population of normal subjects for a lead of an (ECG) system);

b. selecting some (ECG) cycle portion for said (ECG) system lead;

c. obtaining data from (ECG) cycle(s) from a subject, by monitoring a lead of an (ECG) system, said (ECG) system lead monitored being the same as the (ECG) system lead for which representative (ECG) cycle was provided in step a.;

d. selecting some (ECG) cycle portion, which is essentially that selected in step b., and arriving at a composite selected (ECG) cycle portion data set for said monitored (ECG) system lead;

e. calculating corresponding representative parameter(s) from results obtained in steps b. and d.;

f. comparing said corresponding representative parameter(s) to arrive at a "score", the magnitude of which "score" provides an indication of the cardiac status of said subject.

**39.** A noninvasive method of tracking cardiac status change in a subject utilizing electrocardiography (ECG) data obtained therefrom as in Claim 30, in which said follow-on data is obtained at a time after acquisition of said initial data selected from: (immediately thereafter as in a continuous monitoring scenario, and after application of a suitable stress test, and after intervention, and after medical therapy), the benefit being identification of a subject who has undergone cardiac change indicative of the presence of easily induced ischemia causing abnormality.

**40.** A method of analyzing stored electrocardiography (ECG) data of a specific subject, comprising the steps of

a.) obtaining data from ECG cycle(s) of said subject, selecting frequency band(s), and applying necessary filtering techniques to said data to separate said data into at least one frequency band(s),

b.) selecting some ECG cycle portion and arriving at representative parameter(s) for each selected frequency band,

c.) comparing said representative parameter(s) with corresponding representative parameter(s) obtained by applying essentially similar techniques to data from ECG cycle(s) of subject(s) identified as normal, and

d.) combining selected differences between corresponding subject and normal representative parameter(s) to arrive at a score, said score being the result of differences in magnitudes of corresponding subject and normal representative parameter(s).

**41.** A method of analyzing stored electrocardiography (ECG) data of a specific subject, comprising the steps of

a.) obtaining data from ECG cycles of said subject

b.) selecting some ECG cycle portion and calculating an average selected ECG cycle portion data set for at least one monitored ECG system leads, by selecting a plurality of frequency bands and applying filtering techniques to provide a plurality of data sets for each of said monitored system leads

c.) calculating corresponding representative parameters from resulting data sets in said selected frequency bands

d.) comparing these representative parameters with representative values calculated from data form ECG cycles from a plurality of subjects of normal cardiac categories and combining results thereof to arrive at a score resulting from the differences between magnitudes of representative values of said normal subjects and said specific subject.

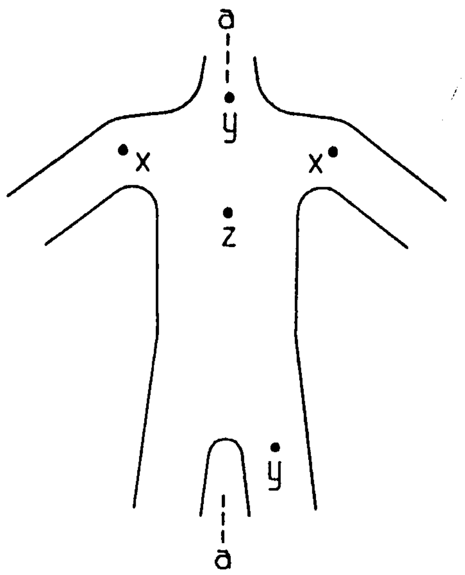


FIG. 1a

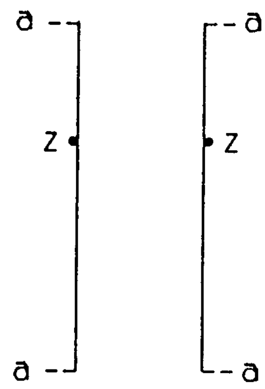


FIG. 1b

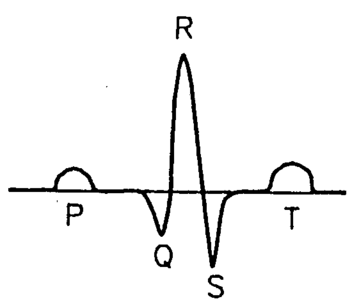


FIG. 2

| FREQUENCIES PRESENT | FRANK (ECG) LEAD X | Y | Z |
|---|---|---|---|
| ALL | | | |
| 0-10 Hz | | | |
| 10-60 Hz | | | |
| 60-150 Hz | | | |
| 150-250 Hz | | | |

FIG. 3

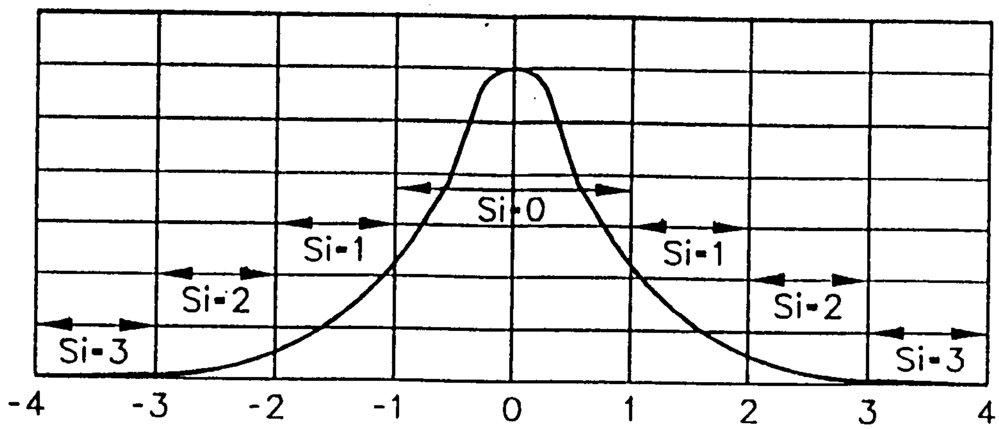
Si=0
Si=1
Si=1
Si=2
Si=2
Si=3
Si=3
-4
-3
-2
-1
0
1
2
3
4

FIG. 4

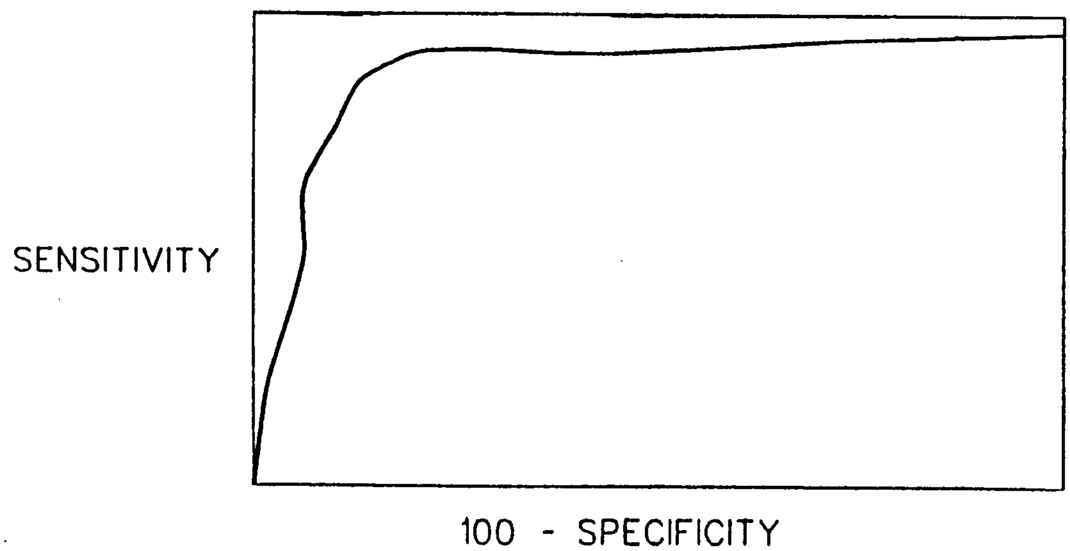
SENSITIVITY
100 - SPECIFICITY

FIG. 5

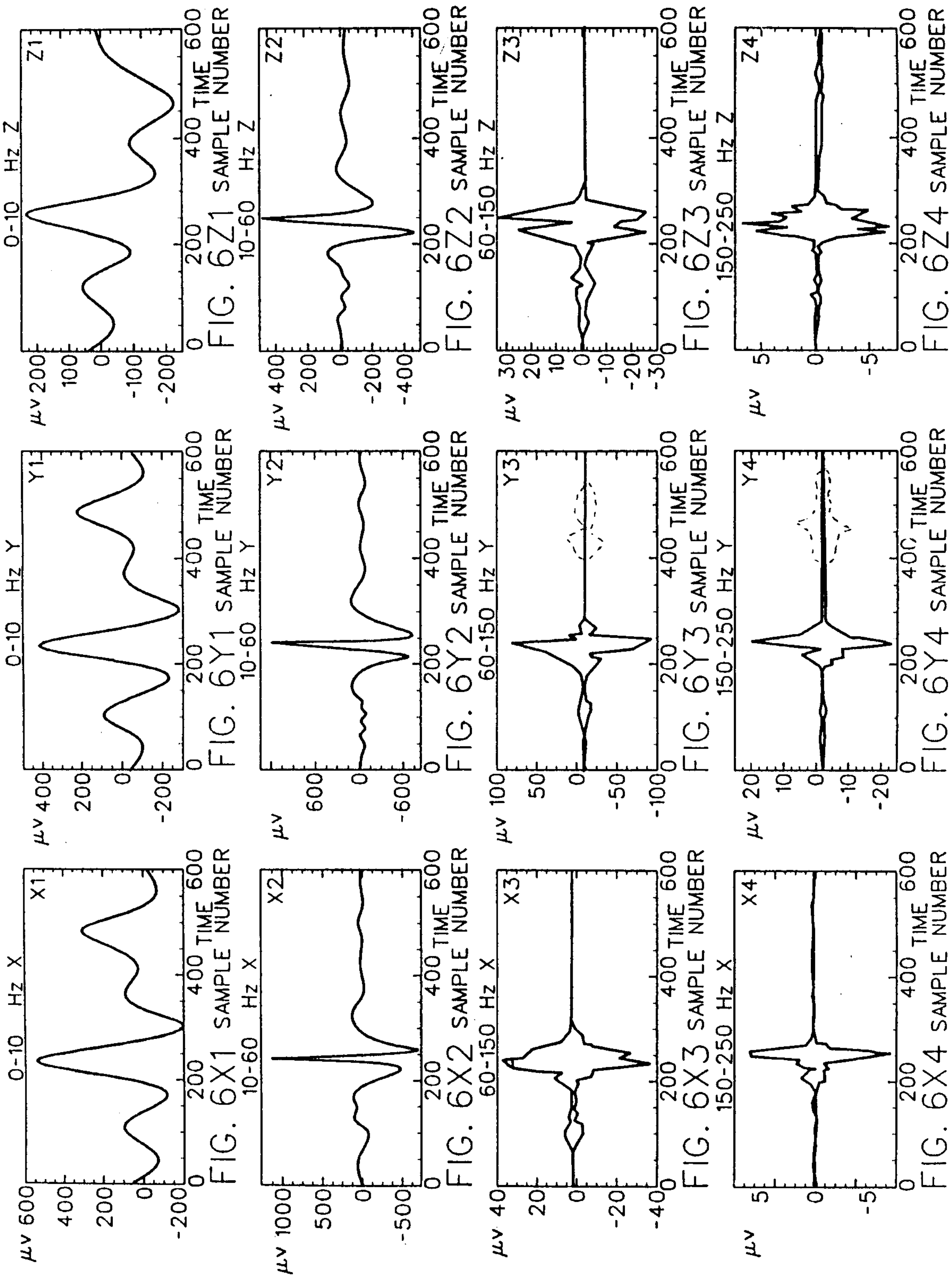

FIG. 6X1 0-10 Hz X

FIG. 6X2 10-60 Hz X

FIG. 6X3 60-150 Hz X

FIG. 6X4 150-250 Hz X

FIG. 6Y1 0-10 Hz Y

FIG. 6Y2 10-60 Hz Y

FIG. 6Y3 60-150 Hz Y

FIG. 6Y4 150-250 Hz Y

FIG. 6Z1 0-10 Hz Z

FIG. 6Z2 10-60 Hz Z

FIG. 6Z3 60-150 Hz Z

FIG. 6Z4 150-250 Hz Z

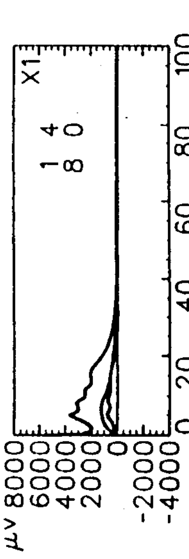

FIG. 7aX1

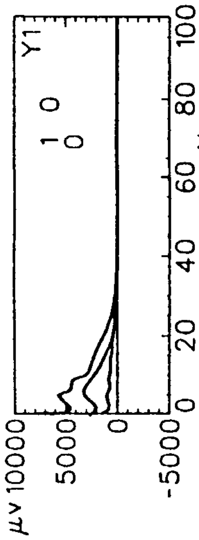

FIG. 7aY1

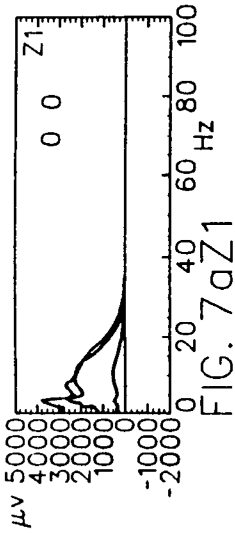

FIG. 7aZ1

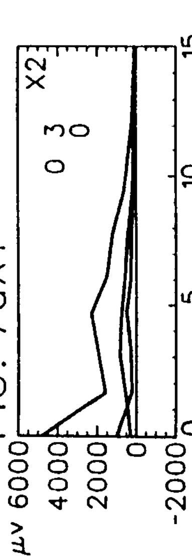

FIG. 7aX2

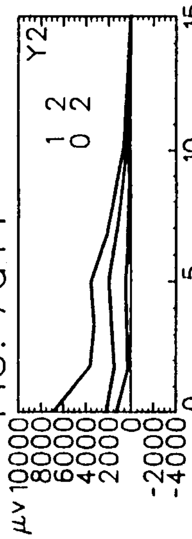

FIG. 7aY2

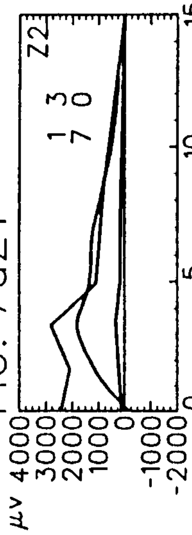

FIG. 7aZ2

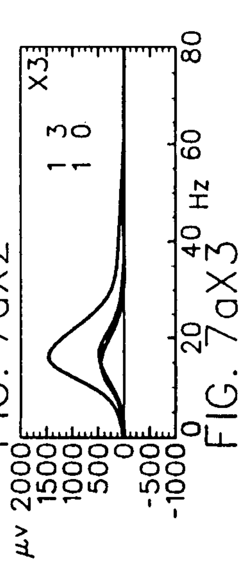

FIG. 7aX3

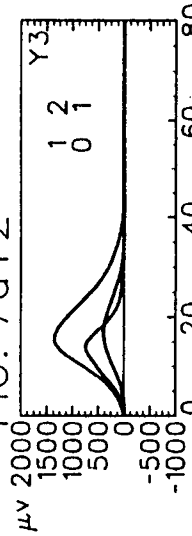

FIG. 7aY3

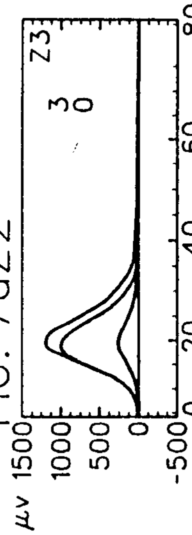

FIG. 7aZ3

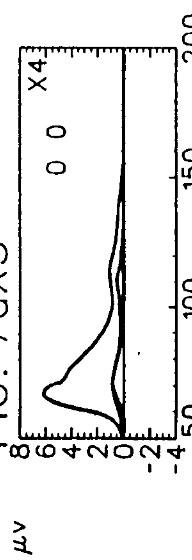

FIG. 7aX4

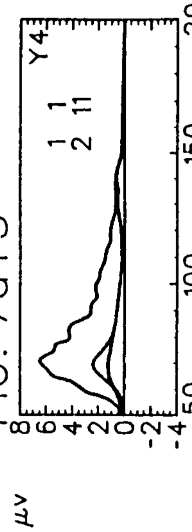

FIG. 7aY4

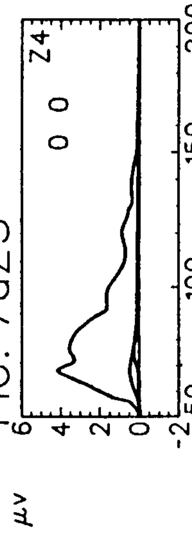

FIG. 7aZ4

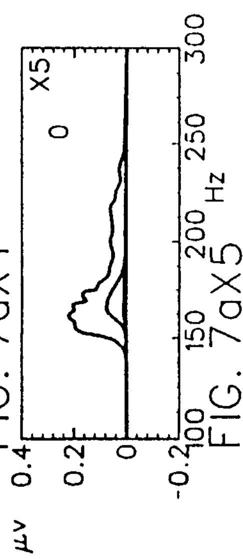

FIG. 7aX5

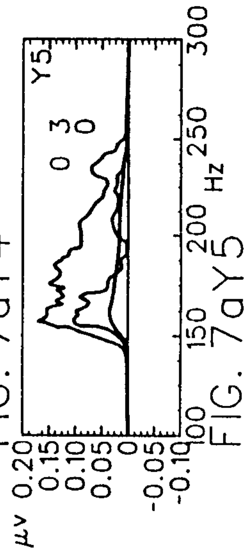

FIG. 7aY5

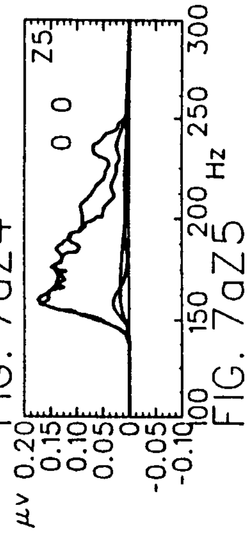

FIG. 7aZ5

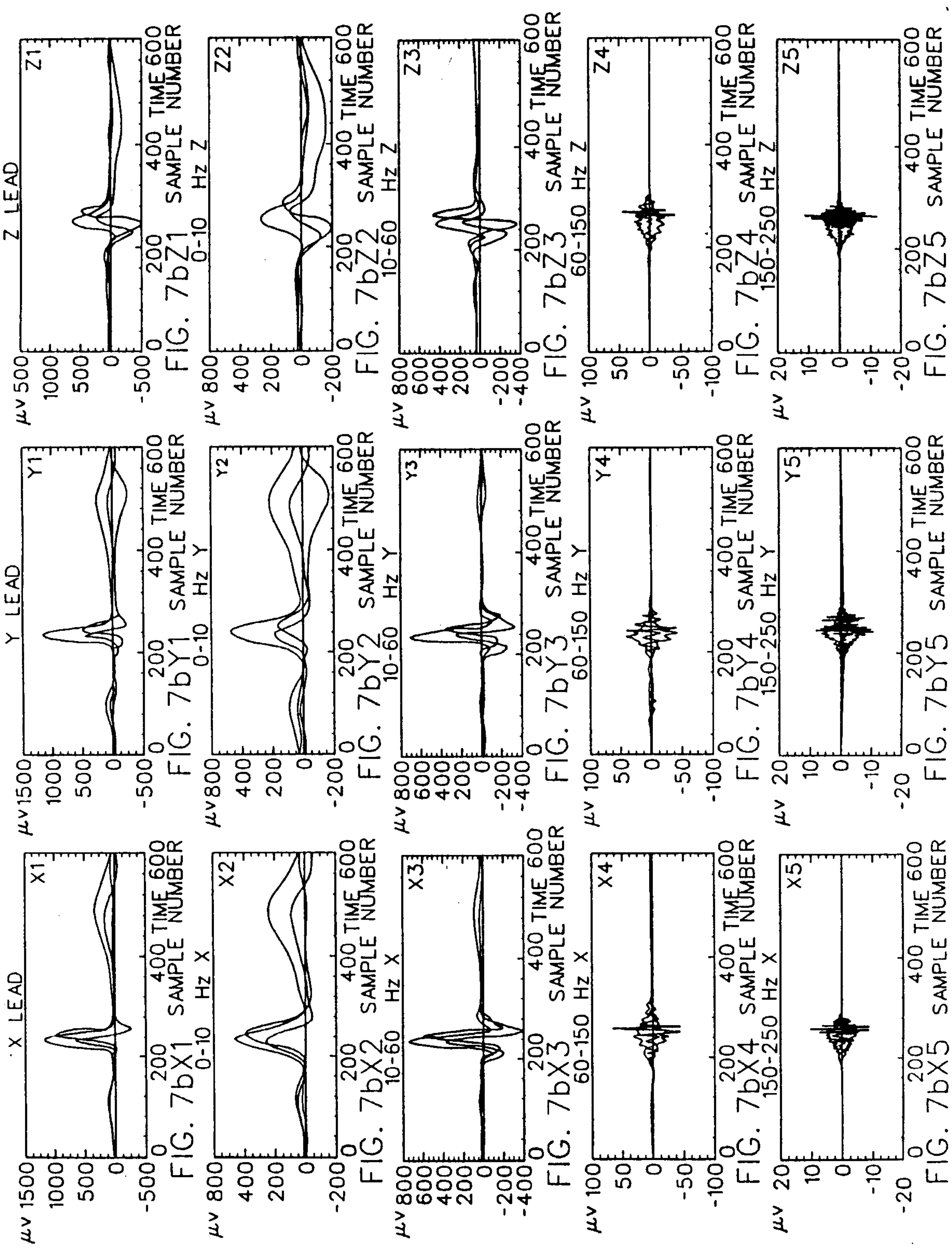


FIG. 7bX1 0-10 Hz X

FIG. 7bX2 10-60 Hz X

FIG. 7bX3 60-150 Hz X

FIG. 7bX4 150-250 Hz X

FIG. 7bX5

FIG. 7bY1 0-10 Hz Y

FIG. 7bY2 10-60 Hz Y

FIG. 7bY3 60-150 Hz Y

FIG. 7bY4 150-250 Hz Y

FIG. 7bY5

FIG. 7bZ1 0-10 Hz Z

FIG. 7bZ2 10-60 Hz Z

FIG. 7bZ3 60-150 Hz Z

FIG. 7bZ4 150-250 Hz Z

FIG. 7bZ5

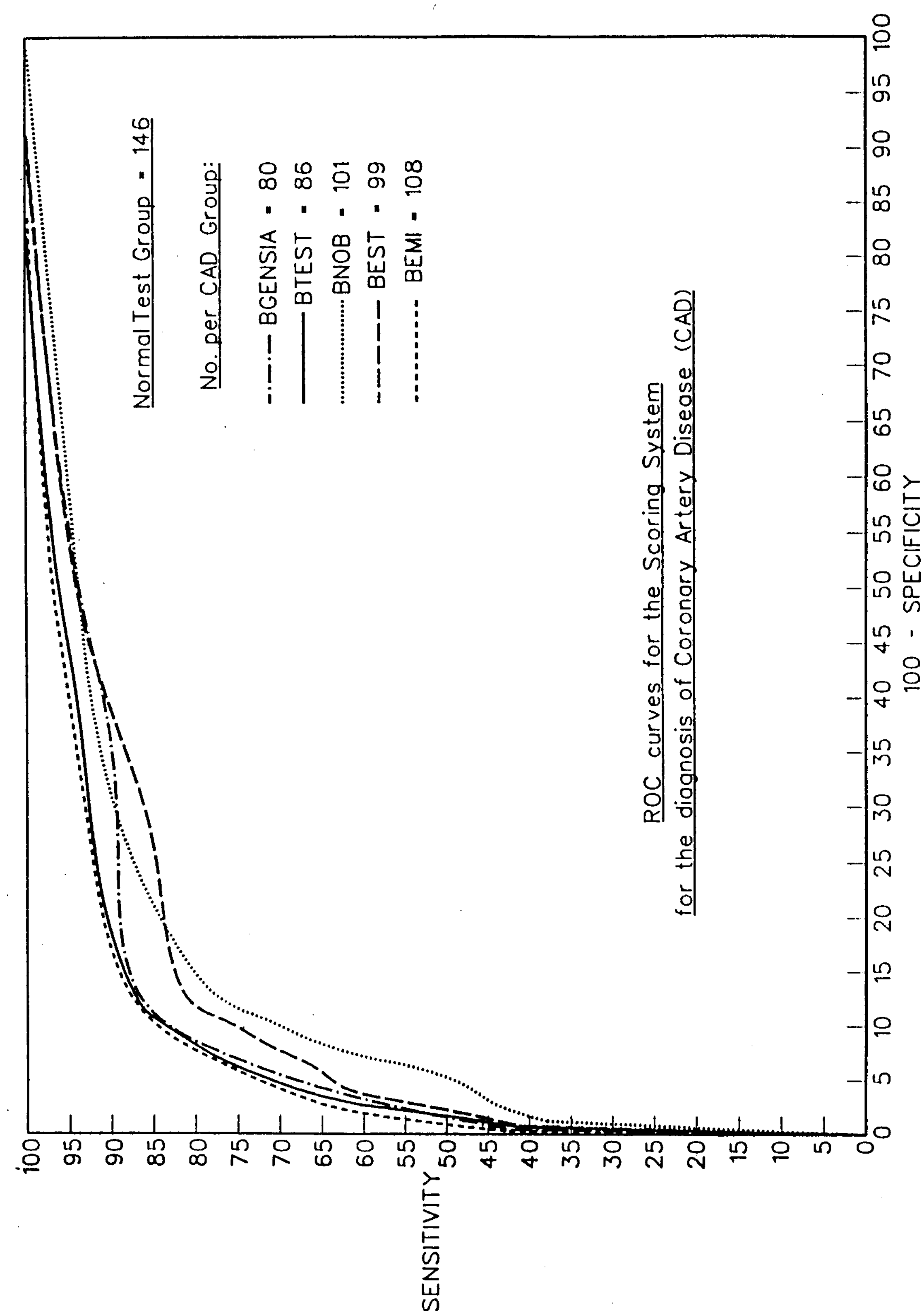
Normal Test Group = 146
No. per CAD Group:
BGENSIA = 80
BTEST = 86
BNOB = 101
BEST = 99
BEMI = 108
ROC curves for the Scoring System
for the diagnosis of Coronary Artery Disease (CAD)
SENSITIVITY
100 - SPECIFICITY

FIG. 8

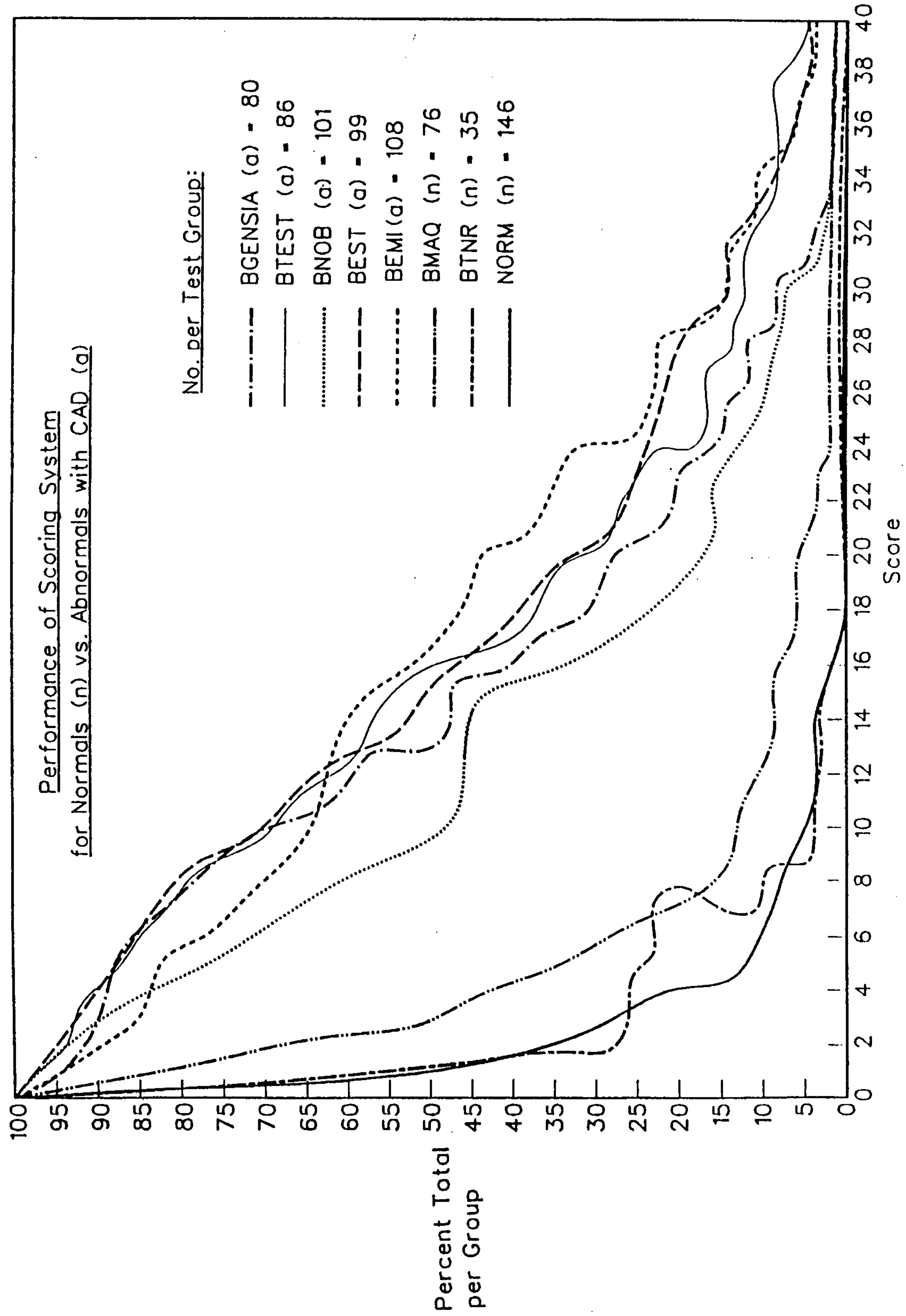
Performance of Scoring System
for Normals (n) vs. Abnormals with CAD (a)
No. per Test Group:
BGENSIA (a) = 80
BTEST (a) = 86
BNOB (a) = 101
BEST (a) = 99
BEMI (a) = 108
BMAQ (n) = 76
BTNR (n) = 35
NORM (n) = 146
Percent Total per Group
Score
100
95
90
85
80
75
70
65
60
55
50
45
40
35
30
25
20
15
10
5
0
2
4
6
8
10
12
14
16
18
20
22
24
26
28
30
32
34
36
38
40

FIG. 9

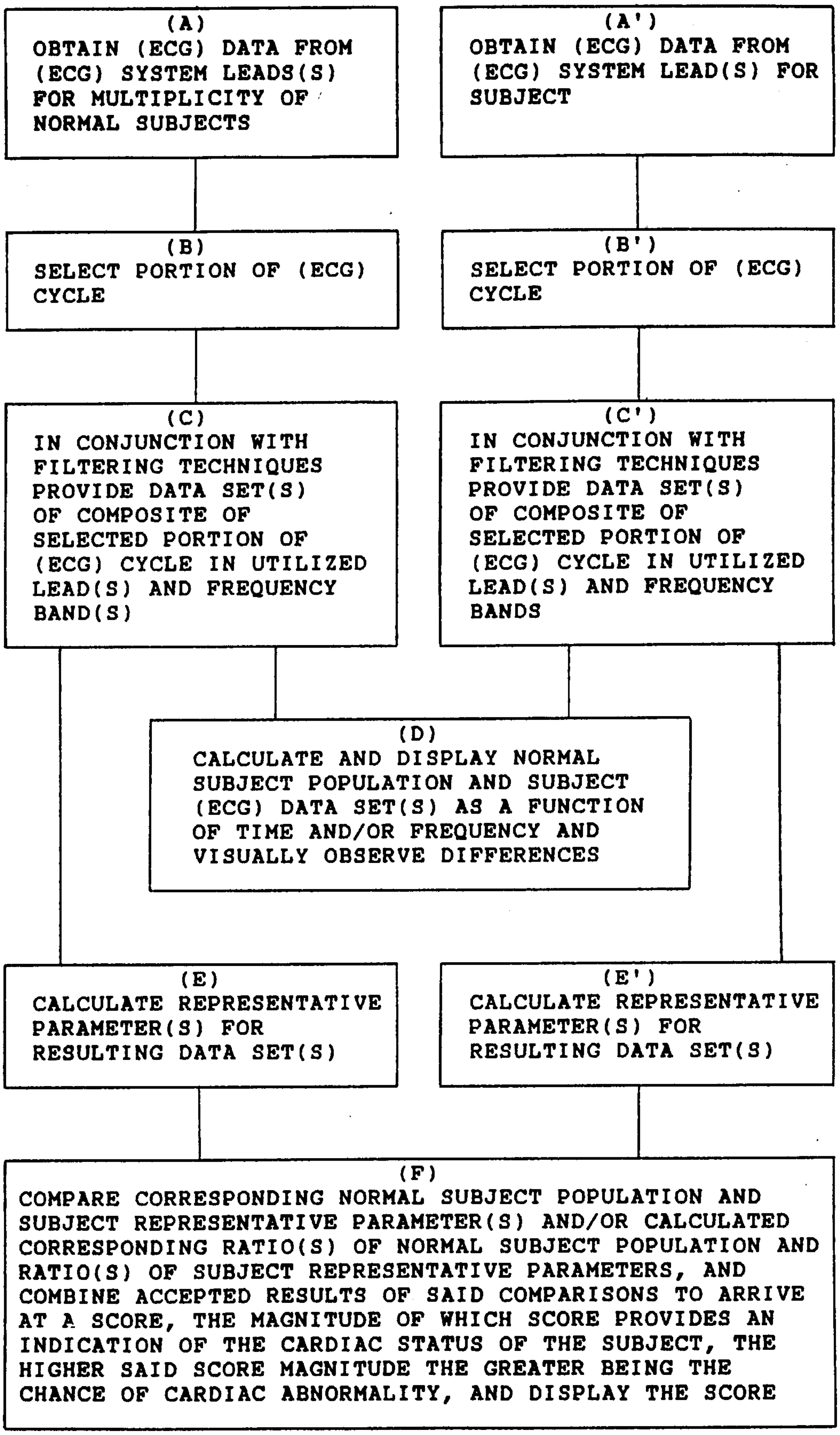
(A)
OBTAIN (ECG) DATA FROM (ECG) SYSTEM LEADS(S) FOR MULTIPLICITY OF NORMAL SUBJECTS
(A')
OBTAIN (ECG) DATA FROM (ECG) SYSTEM LEAD(S) FOR SUBJECT
(B)
SELECT PORTION OF (ECG) CYCLE
(B')
SELECT PORTION OF (ECG) CYCLE
(C)
IN CONJUNCTION WITH FILTERING TECHNIQUES PROVIDE DATA SET(S) OF COMPOSITE OF SELECTED PORTION OF (ECG) CYCLE IN UTILIZED LEAD(S) AND FREQUENCY BAND(S)
(C')
IN CONJUNCTION WITH FILTERING TECHNIQUES PROVIDE DATA SET(S) OF COMPOSITE OF SELECTED PORTION OF (ECG) CYCLE IN UTILIZED LEAD(S) AND FREQUENCY BANDS
(D)
CALCULATE AND DISPLAY NORMAL SUBJECT POPULATION AND SUBJECT (ECG) DATA SET(S) AS A FUNCTION OF TIME AND/OR FREQUENCY AND VISUALLY OBSERVE DIFFERENCES
(E)
CALCULATE REPRESENTATIVE PARAMETER(S) FOR RESULTING DATA SET(S)
(E')
CALCULATE REPRESENTATIVE PARAMETER(S) FOR RESULTING DATA SET(S)
(F)
COMPARE CORRESPONDING NORMAL SUBJECT POPULATION AND SUBJECT REPRESENTATIVE PARAMETER(S) AND/OR CALCULATED CORRESPONDING RATIO(S) OF NORMAL SUBJECT POPULATION AND RATIO(S) OF SUBJECT REPRESENTATIVE PARAMETERS, AND COMBINE ACCEPTED RESULTS OF SAID COMPARISONS TO ARRIVE AT A SCORE, THE MAGNITUDE OF WHICH SCORE PROVIDES AN INDICATION OF THE CARDIAC STATUS OF THE SUBJECT, THE HIGHER SAID SCORE MAGNITUDE THE GREATER BEING THE CHANCE OF CARDIAC ABNORMALITY, AND DISPLAY THE SCORE

FIG. 10

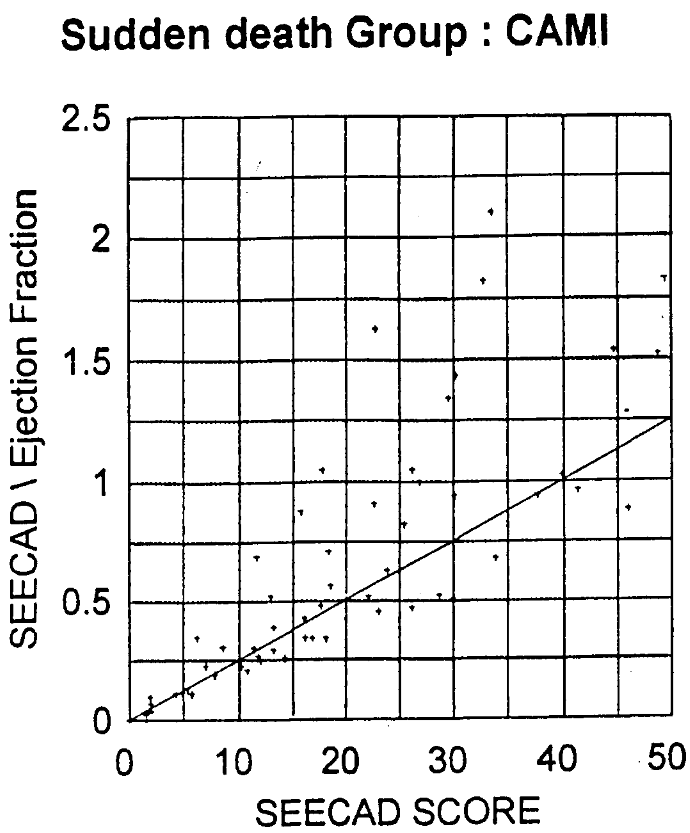


FIG. 11a

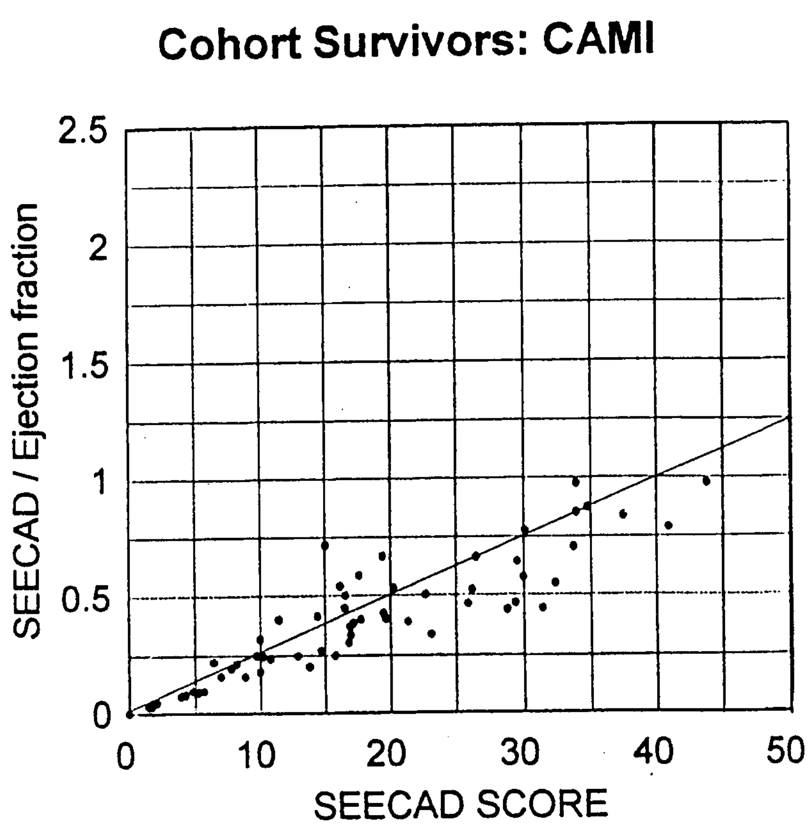


FIG. 11b

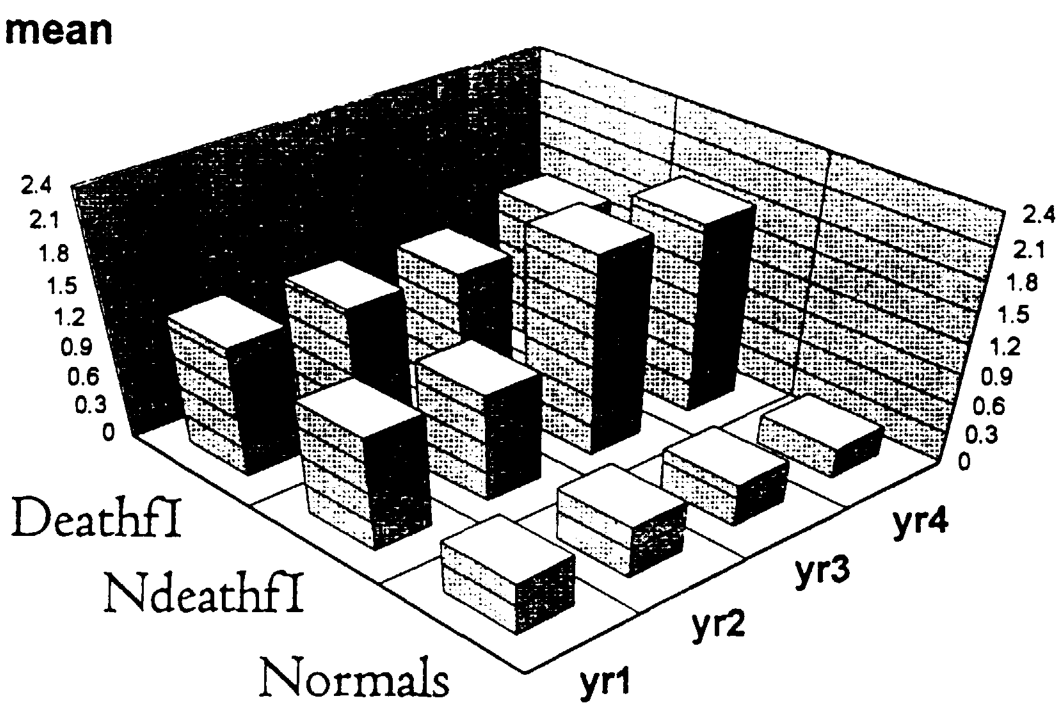
mean
2.4
2.1
1.8
1.5
1.2
0.9
0.6
0.3
0
DeathfI
NdeathfI
Normals
yr1
yr2
yr3
yr4

FIG. 12

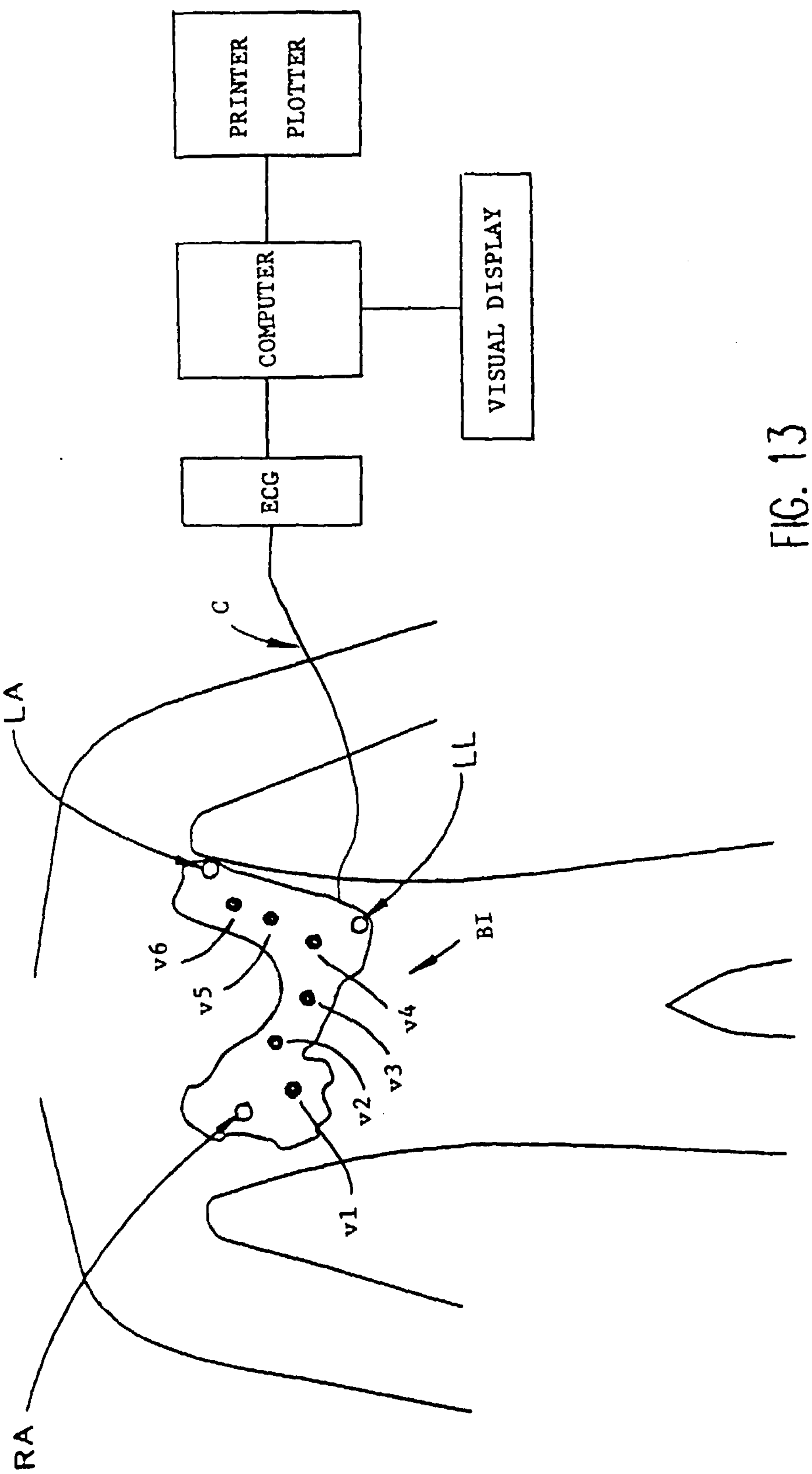
PRINTER
PLOTTER
COMPUTER
VISUAL DISPLAY
ECG
C
LA
LL
BI
v6
v5
v4
v3
v2
v1
RA

FIG. 13

European Patent Office

# EUROPEAN SEARCH REPORT

Application Number
EP 97 81 0562

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| E | US 5 655 540 A (SEEGOBIN RONALD D) 12 August 1997<br>* column 73, line 5 - column 90, line 17 *<br>--- | 1-29,32 | G06F17/00 |
| X | US 4 974 598 A (JOHN ERWIN R) 4 December 1990 | 1,7,11, 15,19, 23,32, 38,40,41 | |
| | * abstract *<br>* column 2, line 50 - column 4, line 24 *<br>* column 11, line 50 - column 12, line 5 *<br>--- | | |
| X | EP 0 448 196 A (DEL MAR AVIONICS) 25 September 1991 | 1,7,11, 15,19, 23,32, 38,40,41 | |
| Y | * column 6, line 43 - column 12, line 56 *<br>* column 15, line 47 - column 16, line 20; figure 1 *<br>--- | 30,33 | |
| Y | US 5 271 411 A (RIPLEY ET AL) 21 December 1993<br>* abstract *<br>* column 2, line 20 - column 3, line 36 *<br>--- | 30,33 | TECHNICAL FIELDS SEARCHED (Int.Cl.6)<br>G06F |
| A | US 5 299 119 A (KRAF ET AL) 29 March 1994<br>* abstract *<br>* column 7, line 30 - line 31 *<br>----- | 1 | |

The present search report has been drawn up for all claims

| Place of search | Date of completion of the search | Examiner |
|---|---|---|
| THE HAGUE | 3 June 1998 | Schenkels, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)